(19) Europäisches Patentamt
European Patent Office
Office européen des brevets

(11) EP 4 681 740 A1

(12) **EUROPEAN PATENT APPLICATION**
published in accordance with Art. 153(4) EPC

(43) Date of publication:
**21.01.2026 Bulletin 2026/04**

(21) Application number: **24770920.7**

(22) Date of filing: **13.03.2024**

(51) International Patent Classification (IPC):
***A61K 47/68*** *(2017.01)* ***A61K 31/4745*** *(2006.01)*
***A61K 39/395*** *(2006.01)* ***A61K 45/00*** *(2006.01)*
***A61P 13/12*** *(2006.01)* ***A61P 15/00*** *(2006.01)*
***A61P 35/00*** *(2006.01)* ***A61P 43/00*** *(2006.01)*
***C07K 7/02*** *(2006.01)* ***C07K 16/30*** *(2006.01)*
***C12N 15/13*** *(2006.01)*

(52) Cooperative Patent Classification (CPC):
**A61K 31/4745; A61K 39/395; A61K 45/00; A61K 47/68; A61P 13/12; A61P 15/00; A61P 35/00; A61P 43/00; C07K 7/02; C07K 16/00; C07K 16/30**

(86) International application number:
**PCT/JP2024/009716**

(87) International publication number:
**WO 2024/190815 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.03.2023 JP 2023039600**

(71) Applicant: **Daiichi Sankyo Company, Limited**
**Tokyo 103-8426 (JP)**

(72) Inventor: **NAGASE Shotaro**
**Tokyo 103-8426 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

(54) **COMBINATION OF ANTI-CDH6 ANTIBODY-DRUG CONJUGATE WITH VEGF INHIBITOR**

(57) The present invention provides a pharmaceutical composition and a method of treatment, wherein a specific anti-CDH6 antibody-drug conjugate and a VEGF inhibitor are administered in combination. The present invention provides a pharmaceutical composition and a method of treatment, wherein an antibody-drug conjugate and a VEGF inhibitor are administered in combination, the antibody-drug conjugate being an antibody-drug conjugate in which a drug linker represented by the following formula (wherein A represents a connecting position to an anti-CDH6 antibody or a functional fragment of the antibody) and an anti-CDH6 antibody or a functional fragment of the antibody are conjugated via a thioether bond.

EP 4 681 740 A1

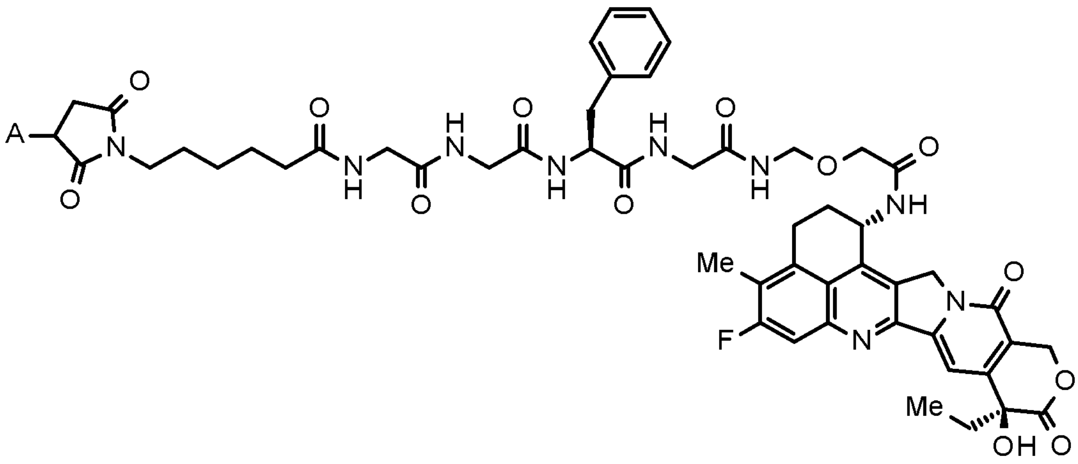

## Description

[Technical Field]

The present invention relates to a pharmaceutical composition wherein a specific anti-CDH6 antibody-drug conjugate and a VEGF inhibitor are administered in combination, and/or a method of treatment comprising administering a specific anti-CDH6 antibody-drug conjugate and a VEGF inhibitor in combination to a subject.

[Background Art]

Angiogenesis which contributes to the supply of nutrients or oxygen and the excretion of metabolic wastes is important for solid tumor growth. Vascular endothelial growth factor (VEGF), particularly, VEGF-A, has been identified as an important factor for inducing tumor angiogenesis. VEGF can activate the VEGF signal transduction of endothelial cells through its interaction with a VEGF receptor, thereby stimulating the growth and survival of the endothelial cells, increasing vascular permeability, and supporting metabolic needs of a growing tumor. VEGF inhibitors are known as drugs that inhibit angiogenesis by inhibiting the interaction between VEGF and a VEGF receptor, and exhibit an antitumor effect. Examples of such VEGF inhibitors can include anti-VEGF antibodies, anti-VEGF receptor antibodies, fusion proteins comprising a VEGF receptor extracellular domain, and bispecific antibodies comprising an anti-VEGF binding domain (Non Patent Literature 1 to 4).

An antibody-drug conjugate (ADC), in which an antibody that binds to an antigen expressed on the surface of cancer cells and can internalize the antigen into the cell is conjugated to a drug having cytotoxic activity, can selectively deliver the drug to cancer cells, and can thereby kill the cancer cells by accumulating the drug in the cancer cells. An antibody-drug conjugate comprising an anti-CDH6 antibody and a topoisomerase I inhibitor exatecan derivative as components is a known antibody-drug conjugate (Patent Literature 1).

However, neither test results exhibiting an excellent combinatorial effect, nor scientific rationale suggesting such test results, when the anti-CDH6 antibody-drug conjugate described above and a VEGF inhibitor are used in combination, has been published.

[Citation List]

[Patent Literature]

[Patent Literature 1] International Publication No. WO 2018/212136

[Non Patent Literature]

[Non Patent Literature 1] Garcia et al., Cancer Treat. Rev. (2020) 86: 102017
[Non Patent Literature 2] Geindreau et al. Int. J. Mol. Sci. (2021) 22 (9): 4871.
[Non Patent Literature 3] Esfandiari et al., Nat. Rev. Drug Discov. (2022) 21 (6): 411-412.
[Non Patent Literature 4] Xu et al., Cancer Lett. (2022) 538: 215699.

[Summary of Invention]

[Technical Problem]

The anti-CDH6 antibody-drug conjugate (anti-CDH6 antibody-drug conjugate comprising an exatecan derivative as a component) used in the present invention has been confirmed to exhibit an excellent antitumor effect even when used alone. However, there has been a need to obtain a method of treatment which can suppress growth of cancer cells in multiple ways and exert an even more superior antitumor effect by using the antibody-drug conjugate in combination with another anticancer agent having a different mechanism of action.

An object of the present invention is to provide a pharmaceutical composition wherein a specific anti-CDH6 antibody-drug conjugate and a VEGF inhibitor are administered in combination, and/or a method of treatment comprising administering a specific anti-CDH6 antibody-drug conjugate and a VEGF inhibitor in combination to a subject.

[Solution to Problem]

The present inventors have conducted intensive studies directed towards achieving the above-described object, and consequently completed the present invention by finding that a specific anti-CDH6 antibody-drug conjugate and a VEGF inhibitor administered in combination exhibit an excellent combinatorial effect (a highly safe and marked antitumor effect).

Thus, the present invention provides the following [1] to [309].

[1] A pharmaceutical composition comprising an anti-CDH6 antibody-drug conjugate, wherein the anti-CDH6 antibody-drug conjugate and a VEGF inhibitor are administered in combination, and
the anti-CDH6 antibody-drug conjugate is an antibody-drug conjugate in which a drug-linker represented by the formula:

[Formula 1]

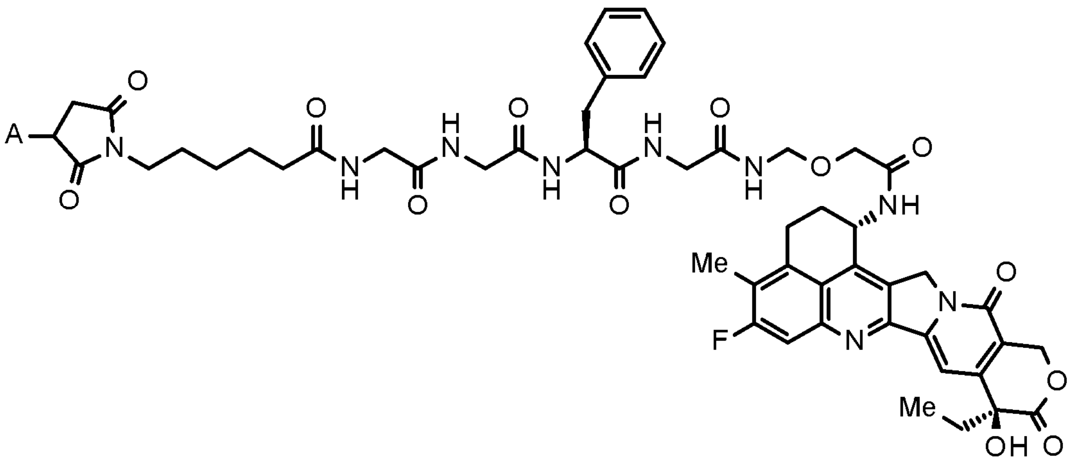

wherein A represents a connecting position to an anti-CDH6 antibody or a functional fragment of the antibody, is conjugated to the anti-CDH6 antibody or the functional fragment of the antibody via a thioether bond.

[2] The pharmaceutical composition according to [1], wherein the anti-CDH6 antibody or the functional fragment of the antibody is an antibody which specifically binds to the amino acid sequence shown in SEQ ID NO: 1 and has an internalization ability that permits cellular uptake, or a functional fragment of the antibody.

[3] The pharmaceutical composition according to [1] or [2], wherein the anti-CDH6 antibody or the functional fragment of the antibody is an antibody comprising CDRH1, CDRH2 and CDRH3 in any one combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID NO: 4,
(2) CDRH1 consisting of the amino acid sequence shown in SEQ ID NO: 2, CDRH2 consisting of the amino acid sequence shown in SEQ ID NO: 8, and CDRH3 consisting of the amino acid sequence shown in SEQ ID NO: 4,
(3) CDRH1 consisting of the amino acid sequence shown in SEQ ID NO: 9, CDRH2 consisting of the amino acid sequence shown in SEQ ID NO: 10, and CDRH3 consisting of the amino acid sequence shown in SEQ ID NO: 11,
(4) CDRH1 consisting of the amino acid sequence shown in SEQ ID NO: 15, CDRH2 consisting of the amino acid sequence shown in SEQ ID NO: 16, and CDRH3 consisting of the amino acid sequence shown in SEQ ID NO: 17, and
(5) CDRH1 consisting of the amino acid sequence shown in SEQ ID NO: 21, CDRH2 consisting of the amino acid sequence shown in SEQ ID NO: 22, and CDRH3 consisting of the amino acid sequence shown in SEQ ID NO: 23,
and CDRL1, CDRL2 and CDRL3 in any one combination selected from the group consisting of the following combinations (6) to (9):
(6) CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(7) CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(8) CDRL1 consisting of the amino acid sequence shown in SEQ ID NO: 18, CDRL2 consisting of the amino acid sequence shown in SEQ ID NO: 19, …

sequence shown in SEQ ID NO: 19, and CDRL3 consisting of the amino acid sequence shown in SEQ ID NO: 20, and
(9) CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,
or a functional fragment of the antibody.

[4] The pharmaceutical composition according to any one of [1] to [3], wherein the anti-CDH6 antibody or the functional fragment of the antibody is an antibody comprising CDRH1, CDRH2 and CDRH3, and CDRL1, CDRL2 and CDRL3 in any combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(2) CDRH1 consisting of the amino acid sequence shown in SEQ ID NO: 2, CDRH2 consisting of the amino acid sequence shown in SEQ ID NO: 8, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(3) CDRH1 consisting of the amino acid sequence shown in SEQ ID NO: 9, CDRH2 consisting of the amino acid sequence shown in SEQ ID NO: 10, CDRH3 consisting of the amino acid sequence shown in SEQ ID NO: 11, CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(4) CDRH1 consisting of the amino acid sequence shown in SEQ ID NO: 15, CDRH2 consisting of the amino acid sequence shown in SEQ ID NO: 16, CDRH3 consisting of the amino acid sequence shown in SEQ ID NO: 17, CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(5) CDRH1 consisting of the amino acid sequence shown in SEQ ID NO: 21, CDRH2 consisting of the amino acid sequence shown in SEQ ID NO: 22, CDRH3 consisting of the amino acid sequence shown in SEQ ID NO: 23, CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,
or a functional fragment of the antibody.

[5] The pharmaceutical composition according to any one of [1] to [4], wherein the anti-CDH6 antibody or the functional fragment of the antibody is an antibody comprising CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, or a functional fragment of the antibody.
[6] The pharmaceutical composition according to any one of [1] to [5], wherein the anti-CDH6 antibody or the functional fragment of the antibody is a humanized antibody or functional fragment of the antibody.
[7] The pharmaceutical composition according to any one of [1] to [6], wherein the anti-CDH6 antibody or the functional fragment of the antibody is an antibody comprising any one heavy chain variable region selected from the group consisting of the following variable regions (1) to (5):

(1) a heavy chain variable region consisting of the amino acid sequence shown in SEQ ID NO: 28,
(2) a heavy chain variable region consisting of the amino acid sequence shown in SEQ ID NO: 31,
(3) a heavy chain variable region consisting of the amino acid sequence shown in SEQ ID NO: 34,
(4) an amino acid sequence having a sequence identity of at least 95% or more to the sequence of the framework regions other than at each CDR sequence in the amino acid sequences of any one of (1) to (3), and
(5) an amino acid sequence comprising a deletion, substitution or addition of one or several amino acids in the sequence of the framework regions other than at each CDR sequence in the amino acid sequences of any one of (1) to (4), and
any one light chain variable region selected from the group consisting of the following variable regions (6) to (9):
(6) a light chain variable region consisting of the amino acid sequence shown in SEQ ID NO: 37,
(7) a light chain variable region consisting of the amino acid sequence shown in SEQ ID NO: 40,
(8) an amino acid sequence having a sequence identity of at least 95% or more to the sequence of the framework regions other than at each CDR sequence in the amino acid sequences of any one of (6) and (7), and

(9) an amino acid sequence comprising a deletion, substitution or addition of one or several amino acids in the sequence of the framework regions other than at each CDR sequence in the amino acid sequences of any one of (6) to (8),

or a functional fragment of the antibody.
[8] The pharmaceutical composition according to any one of [1] to [7], wherein the anti-CDH6 antibody or the functional fragment of the antibody is an antibody comprising a heavy chain variable region and a light chain variable region in any of the following combinations (1) to (4):

(1) a heavy chain variable region consisting of the amino acid sequence shown in SEQ ID NO: 28 and a light chain variable region consisting of the amino acid sequence shown in SEQ ID NO: 37,
(2) a heavy chain variable region consisting of the amino acid sequence shown in SEQ ID NO: 31 and a light chain variable region consisting of the amino acid sequence shown in SEQ ID NO: 37,
(3) a heavy chain variable region consisting of the amino acid sequence shown in SEQ ID NO: 31 and a light chain variable region consisting of the amino acid sequence shown in SEQ ID NO: 40, and
(4) a heavy chain variable region consisting of the amino acid sequence shown in SEQ ID NO: 34 and a light chain variable region consisting of the amino acid sequence shown in SEQ ID NO: 37,

or a functional fragment of the antibody.
[9] The pharmaceutical composition according to any one of [1] to [8], wherein the anti-CDH6 antibody or the functional fragment of the antibody is an antibody comprising a heavy chain variable region consisting of the amino acid sequence shown in SEQ ID NO: 28 and a light chain variable region consisting of the amino acid sequence shown in SEQ ID NO: 37, or a functional fragment of the antibody.
[10] The pharmaceutical composition according to any one of [1] to [9], wherein the anti-CDH6 antibody or the functional fragment of the antibody is an antibody comprising any of the following combinations (1) to (4):

(1) a heavy chain consisting of the amino acid sequence shown in SEQ ID NO: 29 and a light chain consisting of the amino acid sequence shown in SEQ ID NO: 38,
(2) a heavy chain consisting of the amino acid sequence shown in SEQ ID NO: 32 and a light chain consisting of the amino acid sequence shown in SEQ ID NO: 38,
(3) a heavy chain consisting of the amino acid sequence shown in SEQ ID NO: 32 and a light chain consisting of the amino acid sequence shown in SEQ ID NO: 41, and
(4) a heavy chain consisting of the amino acid sequence shown in SEQ ID NO: 35 and a light chain consisting of the amino acid sequence shown in SEQ ID NO: 38,

or a functional fragment of the antibody.
[11] The pharmaceutical composition according to any one of [1] to [10], wherein the anti-CDH6 antibody or the functional fragment of the antibody is an antibody comprising a heavy chain consisting of the amino acid sequence shown in SEQ ID NO: 29 and a light chain consisting of the amino acid sequence shown in SEQ ID NO: 38, or a functional fragment of the antibody.
[12] The pharmaceutical composition according to any one of [1] to [11], wherein the anti-CDH6 antibody or the functional fragment of the antibody is an antibody in which

the heavy chain or the light chain has undergone one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, conversion of N-terminal glutamine or N-terminal glutamic acid to pyroglutamic acid, and a deletion of one or two amino acids from the carboxyl terminus,
or a functional fragment of the antibody.

[13] The pharmaceutical composition according to [12], wherein the anti-CDH6 antibody or the functional fragment of the antibody is an antibody in which one or two amino acids are deleted from the carboxyl terminus of a heavy chain thereof, or a functional fragment of the antibody.
[14] The pharmaceutical composition according to [13], wherein the anti-CDH6 antibody or the functional fragment of the antibody is an antibody in which one amino acid is deleted from the carboxyl terminus of each of the two heavy chains thereof, or a functional fragment of the antibody.
[15] The pharmaceutical composition according to any one of [12] to [14], wherein the deleted amino acid is lysine.
[16] The pharmaceutical composition according to any one of [12] to [15], wherein the anti-CDH6 antibody or the

functional fragment of the antibody is an antibody in which a proline residue at the carboxyl terminus of a heavy chain thereof is further amidated, or a functional fragment of the antibody.

[17] The pharmaceutical composition according to any one of [1] to [16], wherein the average number of units of the drug-linker conjugated per antibody in the anti-CDH6 antibody-drug conjugate is in the range of from 1 to 10.

[18] The pharmaceutical composition according to any one of [1] to [16], wherein the average number of units of the drug-linker conjugated per antibody in the anti-CDH6 antibody-drug conjugate is in the range of from 2 to 8.

[19] The pharmaceutical composition according to any one of [1] to [16], wherein the average number of units of the drug-linker conjugated per antibody in the anti-CDH6 antibody-drug conjugate is in the range of from 5 to 8.

[20] The pharmaceutical composition according to any one of [1] to [16], wherein the average number of units of the drug-linker conjugated per antibody in the anti-CDH6 antibody-drug conjugate is in the range of from 7 to 8.

[21] The pharmaceutical composition according to any one of [1] to [16], wherein the average number of units of the drug-linker conjugated per antibody in the anti-CDH6 antibody-drug conjugate is in the range of from 7.5 to 8.

[22] The pharmaceutical composition according to any one of [1] to [16], wherein the average number of units of the drug-linker conjugated per antibody in the anti-CDH6 antibody-drug conjugate is approximately 8.

[23] The pharmaceutical composition according to any one of [1] to [22], wherein the VEGF inhibitor is an anti-VEGF antibody, an anti-VEGF receptor antibody, a fusion protein comprising a VEGF receptor extracellular domain, or a multispecific antibody comprising an anti-VEGF binding domain.

[24] The pharmaceutical composition according to [23], wherein the VEGF inhibitor is an anti-VEGF antibody.

[25] The pharmaceutical composition according to [24], wherein the anti-VEGF antibody is bevacizumab.

[26] The pharmaceutical composition according to [23], wherein the VEGF inhibitor is an anti-VEGF receptor antibody.

[27] The pharmaceutical composition according to [26], wherein the anti-VEGF receptor antibody is ramucirumab.

[28] The pharmaceutical composition according to [23], wherein the VEGF inhibitor is a fusion protein comprising a VEGF receptor extracellular domain.

[29] The pharmaceutical composition according to [28], wherein the fusion protein comprising a VEGF receptor extracellular domain is aflibercept.

[30] The pharmaceutical composition according to [23], wherein the VEGF inhibitor is a multispecific antibody comprising an anti-VEGF binding domain.

[31] The pharmaceutical composition according to [30], wherein the multispecific antibody is a bispecific antibody.

[32] The pharmaceutical composition according to any one of [1] to [31], wherein the anti-CDH6 antibody-drug conjugate and the VEGF inhibitor are separately contained (as active components) in different formulations, and are administered at the same time (at approximately the same time) or at different times.

[33] The pharmaceutical composition according to any one of [1] to [31], wherein the anti-CDH6 antibody-drug conjugate and the VEGF inhibitor are contained (as active components) in a single formulation for administration.

[34] The pharmaceutical composition according to any one of [1] to [33], wherein the pharmaceutical composition is for use in treating cancer.

[35] The pharmaceutical composition according to any one of [1] to [33], wherein the pharmaceutical composition is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, and nasopharyngeal cancer.

[36] The pharmaceutical composition according to any one of [1] to [33], wherein the pharmaceutical composition is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

[37] The pharmaceutical composition according to any one of [1] to [33], wherein the pharmaceutical composition is for use in treating at least one selected from the group consisting of renal cell carcinoma, renal clear cell carcinoma, and papillary renal cell carcinoma.

[38] The pharmaceutical composition according to any one of [1] to [33], wherein the pharmaceutical composition is for use in treating at least one selected from the group consisting of ovarian cancer, ovarian serous adenocarcinoma, ovarian clear cell carcinoma, endometrioid ovarian cancer, and ovarian mucinous tumor.

[39] The pharmaceutical composition according to any one of [1] to [33], wherein the pharmaceutical composition is for use in treating ovarian cancer.

[40] The pharmaceutical composition according to [39], wherein the ovarian cancer is epithelial ovarian cancer, fallopian tube cancer, or primary peritoneal cancer.

[41] The pharmaceutical composition according to [39] or [40], wherein the ovarian cancer is metastatic.

[42] A pharmaceutical composition comprising an anti-CDH6 antibody-drug conjugate, wherein the anti-CDH6 antibody-drug conjugate and a VEGF inhibitor are administered in combination, and

the anti-CDH6 antibody-drug conjugate is an antibody-drug conjugate represented by the formula:

[Formula 2]

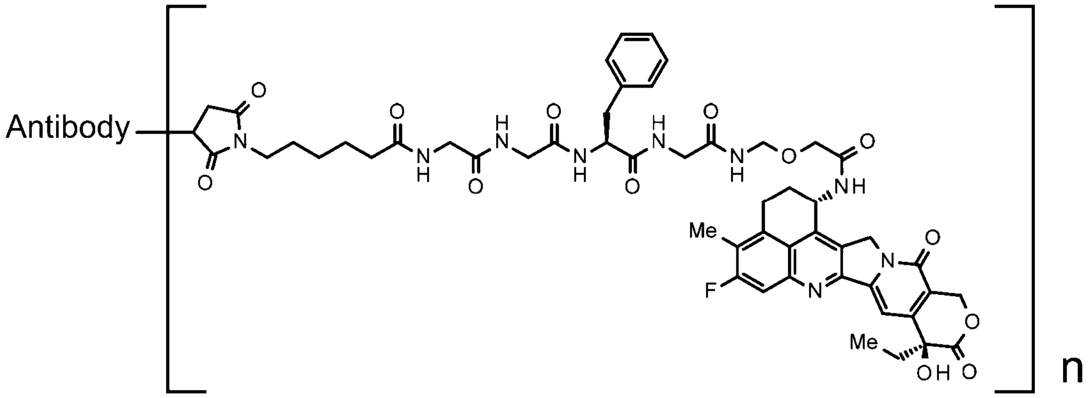

wherein Antibody is an anti-CDH6 antibody, a drug-linker is conjugated to the antibody via a thioether bond, and n represents an average number of units of the drug-linker conjugated per antibody.

[43] The pharmaceutical composition according to [42], wherein the anti-CDH6 antibody is an antibody comprising a heavy chain consisting of the amino acid sequence shown in SEQ ID NO: 29 and a light chain consisting of the amino acid sequence shown in SEQ ID NO: 38.

[44] The pharmaceutical composition according to [42] or [43], wherein a lysine residue is deleted from the carboxyl terminus of a heavy chain of the anti-CDH6 antibody.

[45] The pharmaceutical composition according to any one of [42] to [44], wherein the average number of units of the drug-linker conjugated per antibody in the anti-CDH6 antibody-drug conjugate is in the range of from 1 to 10.

[46] The pharmaceutical composition according to any one of [42] to [44], wherein the average number of units of the drug-linker conjugated per antibody in the anti-CDH6 antibody-drug conjugate is in the range of from 2 to 8.

[47] The pharmaceutical composition according to any one of [42] to [44], wherein the average number of units of the drug-linker conjugated per antibody in the anti-CDH6 antibody-drug conjugate is in the range of from 5 to 8.

[48] The pharmaceutical composition according to any one of [42] to [44], wherein the average number of units of the drug-linker conjugated per antibody in the anti-CDH6 antibody-drug conjugate is in the range of from 7 to 8.

[49] The pharmaceutical composition according to any one of [42] to [44], wherein the average number of units of the drug-linker conjugated per antibody in the anti-CDH6 antibody-drug conjugate is in the range of from 7.5 to 8.

[50] The pharmaceutical composition according to any one of [42] to [44], wherein the average number of units of the drug-linker conjugated per antibody in the anti-CDH6 antibody-drug conjugate is approximately 8.

[51] The pharmaceutical composition according to any one of [42] to [50], wherein the VEGF inhibitor is an anti-VEGF antibody, an anti-VEGF receptor antibody, a fusion protein comprising a VEGF receptor extracellular domain, or a multispecific antibody comprising an anti-VEGF binding domain.

[52] The pharmaceutical composition according to [51], wherein the VEGF inhibitor is an anti-VEGF antibody.

[53] The pharmaceutical composition according to [52], wherein the anti-VEGF antibody is bevacizumab.

[54] The pharmaceutical composition according to [51], wherein the VEGF inhibitor is an anti-VEGF receptor antibody.

[55] The pharmaceutical composition according to [54], wherein the anti-VEGF receptor antibody is ramucirumab.

[56] The pharmaceutical composition according to [51], wherein the VEGF inhibitor is a fusion protein comprising a VEGF receptor extracellular domain.

[57] The pharmaceutical composition according to [56], wherein the fusion protein comprising a VEGF receptor extracellular domain is aflibercept.

[58] The pharmaceutical composition according to [51], wherein the VEGF inhibitor is a multispecific antibody comprising an anti-VEGF binding domain.

[59] The pharmaceutical composition according to [58], wherein the multispecific antibody is a bispecific antibody.

[60] The pharmaceutical composition according to any one of [42] to [59], wherein the anti-CDH6 antibody-drug conjugate and the VEGF inhibitor are separately contained (as active components) in different formulations, and are administered at the same time (at approximately the same time) or at different times.

[61] The pharmaceutical composition according to any one of [42] to [59], wherein the anti-CDH6 antibody-drug conjugate and the VEGF inhibitor are contained (as active components) in a single formulation for administration.
[62] The pharmaceutical composition according to any one of [42] to [61], wherein the pharmaceutical composition is for use in treating cancer.
[63] The pharmaceutical composition according to any one of [42] to [61], wherein the pharmaceutical composition is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, and nasopharyngeal cancer.
[64] The pharmaceutical composition according to any one of [42] to [61], wherein the pharmaceutical composition is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.
[65] The pharmaceutical composition according to any one of [42] to [61], wherein the pharmaceutical composition is for use in treating at least one selected from the group consisting of renal cell carcinoma, renal clear cell carcinoma, and papillary renal cell carcinoma.
[66] The pharmaceutical composition according to any one of [42] to [61], wherein the pharmaceutical composition is for use in treating at least one selected from the group consisting of ovarian cancer, ovarian serous adenocarcinoma, ovarian clear cell carcinoma, endometrioid ovarian cancer, and ovarian mucinous tumor.
[67] The pharmaceutical composition according to any one of [42] to [61], wherein the pharmaceutical composition is for use in treating ovarian cancer.
[68] The pharmaceutical composition according to [67], wherein the ovarian cancer is epithelial ovarian cancer, fallopian tube cancer, or primary peritoneal cancer.
[69] The pharmaceutical composition according to [67] or [68], wherein the ovarian cancer is metastatic.
[70] The pharmaceutical composition according to any one of [1] to [69], wherein the anti-CDH6 antibody-drug conjugate is raludotatug deruxtecan (DS-6000a).
[71] A method of treatment, comprising administering an anti-CDH6 antibody-drug conjugate and a VEGF inhibitor in combination to a subject in need of the treatment, wherein the antibody-drug conjugate is an antibody-drug conjugate in which a drug-linker represented by the formula:

[Formula 3]

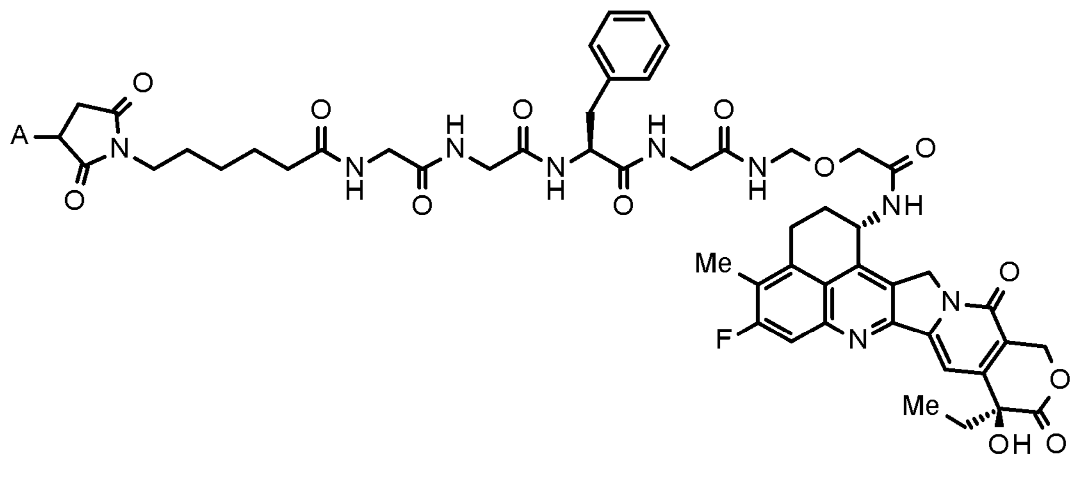

wherein A represents a connecting position to an anti-CDH6 antibody or a functional fragment of the antibody, is conjugated to the anti-CDH6 antibody or the functional fragment of the antibody via a thioether bond.
[72] The method of treatment according to [71], wherein the anti-CDH6 antibody or the functional fragment of the antibody is an antibody which specifically binds to the amino acid sequence shown in SEQ ID NO: 1 and has an internalization ability that permits cellular uptake, or a functional fragment of the antibody.
[73] The method of treatment according to [71] or [72], wherein the anti-CDH6 antibody or the functional fragment of

the antibody is an antibody comprising

CDRH1, CDRH2 and CDRH3 in any one combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID NO: 4,
(2) CDRH1 consisting of the amino acid sequence shown in SEQ ID NO: 2, CDRH2 consisting of the amino acid sequence shown in SEQ ID NO: 8, and CDRH3 consisting of the amino acid sequence shown in SEQ ID NO: 4,
(3) CDRH1 consisting of the amino acid sequence shown in SEQ ID NO: 9, CDRH2 consisting of the amino acid sequence shown in SEQ ID NO: 10, and CDRH3 consisting of the amino acid sequence shown in SEQ ID NO: 11,
(4) CDRH1 consisting of the amino acid sequence shown in SEQ ID NO: 15, CDRH2 consisting of the amino acid sequence shown in SEQ ID NO: 16, and CDRH3 consisting of the amino acid sequence shown in SEQ ID NO: 17, and
(5) CDRH1 consisting of the amino acid sequence shown in SEQ ID NO: 21, CDRH2 consisting of the amino acid sequence shown in SEQ ID NO: 22, and CDRH3 consisting of the amino acid sequence shown in SEQ ID NO: 23, and CDRL1, CDRL2 and CDRL3 in any one combination selected from the group consisting of the following combinations (6) to (9):
(6) CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(7) CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(8) CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(9) CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,

or a functional fragment of the antibody.

[74] The method of treatment according to any one of [71] to [73], wherein the anti-CDH6 antibody or the functional fragment of the antibody is an antibody comprising CDRH1, CDRH2 and CDRH3, and CDRL1, CDRL2 and CDRL3 in any combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(2) CDRH1 consisting of the amino acid sequence shown in SEQ ID NO: 2, CDRH2 consisting of the amino acid sequence shown in SEQ ID NO: 8, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(3) CDRH1 consisting of the amino acid sequence shown in SEQ ID NO: 9, CDRH2 consisting of the amino acid sequence shown in SEQ ID NO: 10, CDRH3 consisting of the amino acid sequence shown in SEQ ID NO: 11, CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(4) CDRH1 consisting of the amino acid sequence shown in SEQ ID NO: 15, CDRH2 consisting of the amino acid sequence shown in SEQ ID NO: 16, CDRH3 consisting of the amino acid sequence shown in SEQ ID NO: 17, CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(5) CDRH1 consisting of the amino acid sequence shown in SEQ ID NO: 21, CDRH2 consisting of the amino acid

sequence shown in SEQ ID NO: 22, CDRH3 consisting of the amino acid sequence shown in SEQ ID NO: 23, CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,

or a functional fragment of the antibody.

[75] The method of treatment according to any one of [71] to [74], wherein the anti-CDH6 antibody or the functional fragment of the antibody is an antibody comprising CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, or a functional fragment of the antibody.

[76] The method of treatment according to any one of [71] to [75], wherein the anti-CDH6 antibody or the functional fragment of the antibody is a humanized antibody or functional fragment of the antibody.

[77] The method of treatment according to any one of [71] to [76], wherein the anti-CDH6 antibody or the functional fragment of the antibody is an antibody comprising any one heavy chain variable region selected from the group consisting of the following variable regions (1) to (5):

(1) a heavy chain variable region consisting of the amino acid sequence shown in SEQ ID NO: 28,
(2) a heavy chain variable region consisting of the amino acid sequence shown in SEQ ID NO: 31,
(3) a heavy chain variable region consisting of the amino acid sequence shown in SEQ ID NO: 34,
(4) an amino acid sequence having a sequence identity of at least 95% or more to the sequence of the framework regions other than at each CDR sequence in the amino acid sequences of any one of (1) to (3), and
(5) an amino acid sequence comprising a deletion, substitution or addition of one or several amino acids in the sequence of the framework regions other than at each CDR sequence in the amino acid sequences of any one of (1) to (4), and

any one light chain variable region selected from the group consisting of the following variable regions (6) to (9):

(6) a light chain variable region consisting of the amino acid sequence shown in SEQ ID NO: 37,
(7) a light chain variable region consisting of the amino acid sequence shown in SEQ ID NO: 40,
(8) an amino acid sequence having a sequence identity of at least 95% or more to the sequence of the framework regions other than at each CDR sequence in the amino acid sequences of any one of (6) and (7), and
(9) an amino acid sequence comprising a deletion, substitution or addition of one or several amino acids in the sequence of the framework regions other than at each CDR sequence in the amino acid sequences of any one of (6) to (8),

or a functional fragment of the antibody.

[78] The method of treatment according to any one of [71] to [77], wherein the anti-CDH6 antibody or the functional fragment of the antibody is an antibody comprising a heavy chain variable region and a light chain variable region in any of the following combinations (1) to (4):

(1) a heavy chain variable region consisting of the amino acid sequence shown in SEQ ID NO: 28 and a light chain variable region consisting of the amino acid sequence shown in SEQ ID NO: 37,
(2) a heavy chain variable region consisting of the amino acid sequence shown in SEQ ID NO: 31 and a light chain variable region consisting of the amino acid sequence shown in SEQ ID NO: 37,
(3) a heavy chain variable region consisting of the amino acid sequence shown in SEQ ID NO: 31 and a light chain variable region consisting of the amino acid sequence shown in SEQ ID NO: 40, and
(4) a heavy chain variable region consisting of the amino acid sequence shown in SEQ ID NO: 34 and a light chain variable region consisting of the amino acid sequence shown in SEQ ID NO: 37,

or a functional fragment of the antibody.

[79] The method of treatment according to any one of [71] to [78], wherein the anti-CDH6 antibody or the functional fragment of the antibody is an antibody comprising a heavy chain variable region consisting of the amino acid sequence shown in SEQ ID NO: 28 and a light chain variable region consisting of the amino acid sequence shown in SEQ ID NO: 37, or a functional fragment of the antibody.

[80] The method of treatment according to any one of [71] to [79], wherein the anti-CDH6 antibody or the functional

fragment of the antibody is an antibody comprising any of the following combinations (1) to (4):

(1) a heavy chain consisting of the amino acid sequence shown in SEQ ID NO: 29 and a light chain consisting of the amino acid sequence shown in SEQ ID NO: 38,
(2) a heavy chain consisting of the amino acid sequence shown in SEQ ID NO: 32 and a light chain consisting of the amino acid sequence shown in SEQ ID NO: 38,
(3) a heavy chain consisting of the amino acid sequence shown in SEQ ID NO: 32 and a light chain consisting of the amino acid sequence shown in SEQ ID NO: 41, and
(4) a heavy chain consisting of the amino acid sequence shown in SEQ ID NO: 35 and a light chain consisting of the amino acid sequence shown in SEQ ID NO: 38, or a functional fragment of the antibody.

[81] The method of treatment according to any one of [71] to [80], wherein the anti-CDH6 antibody or the functional fragment of the antibody is an antibody comprising a heavy chain consisting of the amino acid sequence shown in SEQ ID NO: 29 and a light chain consisting of the amino acid sequence shown in SEQ ID NO: 38, or a functional fragment of the antibody.
[82] The method of treatment according to any one of [71] to [81], wherein the anti-CDH6 antibody or the functional fragment of the antibody is an antibody in which the heavy chain or the light chain has undergone one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, conversion of N-terminal glutamine or N-terminal glutamic acid to pyroglutamic acid, and a deletion of one or two amino acids from the carboxyl terminus, or a functional fragment of the antibody.
[83] The method of treatment according to [82], wherein the anti-CDH6 antibody or the functional fragment of the antibody is an antibody in which one or two amino acids are deleted from the carboxyl terminus of a heavy chain thereof, or a functional fragment of the antibody.
[84] The method of treatment according to [83], wherein the anti-CDH6 antibody or the functional fragment of the antibody is an antibody in which one amino acid is deleted from the carboxyl terminus of each of the two heavy chains thereof, or a functional fragment of the antibody.
[85] The method of treatment according to any one of [82] to [84], wherein the deleted amino acid is lysine.
[86] The method of treatment according to any one of [82] to [85], wherein the anti-CDH6 antibody or the functional fragment of the antibody is an antibody in which a proline residue at the carboxyl terminus of a heavy chain thereof is further amidated, or a functional fragment of the antibody.
[87] The method of treatment according to any one of [71] to [86], wherein the average number of units of the drug-linker conjugated per antibody in the anti-CDH6 antibody-drug conjugate is in the range of from 1 to 10.
[88] The method of treatment according to any one of [71] to [86], wherein the average number of units of the drug-linker conjugated per antibody in the anti-CDH6 antibody-drug conjugate is in the range of from 2 to 8.
[89] The method of treatment according to any one of [71] to [86], wherein the average number of units of the drug-linker conjugated per antibody in the anti-CDH6 antibody-drug conjugate is in the range of from 5 to 8.
[90] The method of treatment according to any one of [71] to [86], wherein the average number of units of the drug-linker conjugated per antibody in the anti-CDH6 antibody-drug conjugate is in the range of from 7 to 8.
[91] The method of treatment according to any one of [71] to [86], wherein the average number of units of the drug-linker conjugated per antibody in the anti-CDH6 antibody-drug conjugate is in the range of from 7.5 to 8.
[92] The method of treatment according to any one of [71] to [86], wherein the average number of units of the drug-linker conjugated per antibody in the anti-CDH6 antibody-drug conjugate is approximately 8.
[93] The method of treatment according to any one of [71] to [92], wherein the VEGF inhibitor is an anti-VEGF antibody, an anti-VEGF receptor antibody, a fusion protein comprising a VEGF receptor extracellular domain, or a multispecific antibody comprising an anti-VEGF binding domain.
[94] The method of treatment according to [93], wherein the VEGF inhibitor is an anti-VEGF antibody.
[95] The method of treatment according to [94], wherein the anti-VEGF antibody is bevacizumab.
[96] The method of treatment according to [93], wherein the VEGF inhibitor is an anti-VEGF receptor antibody.
[97] The method of treatment according to [96], wherein the anti-VEGF receptor antibody is ramucirumab.
[98] The method of treatment according to [93], wherein the VEGF inhibitor is a fusion protein comprising a VEGF receptor extracellular domain.
[99] The method of treatment according to [98], wherein the fusion protein comprising a VEGF receptor extracellular domain is aflibercept.
[100] The method of treatment according to [93], wherein the VEGF inhibitor is a multispecific antibody comprising an anti-VEGF binding domain.
[101] The method of treatment according to [100], wherein the multispecific antibody is a bispecific antibody.

[102] The method of treatment according to any one of [71] to [101], wherein the anti-CDH6 antibody-drug conjugate and the VEGF inhibitor are separately contained (as active components) in different formulations, and are administered at the same time (at approximately the same time) or at different times.
[103] The method of treatment according to any one of [71] to [101], wherein the anti-CDH6 antibody-drug conjugate and the VEGF inhibitor are contained (as active components) in a single formulation for administration.
[104] The method of treatment according to any one of [71] to [103], wherein the method of treatment is for treating cancer.
[105] The method of treatment according to any one of [71] to [103], wherein the method of treatment is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, and nasopharyngeal cancer.
[106] The method of treatment according to any one of [71] to [103], wherein the method of treatment is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.
[107] The method of treatment according to any one of [71] to [103], wherein the method of treatment is for treating at least one selected from the group consisting of renal cell carcinoma, renal clear cell carcinoma, and papillary renal cell carcinoma.
[108] The method of treatment according to any one of [71] to [103], wherein the method of treatment is for treating at least one selected from the group consisting of ovarian cancer, ovarian serous adenocarcinoma, ovarian clear cell carcinoma, endometrioid ovarian cancer, and ovarian mucinous tumor.
[109] The method of treatment according to any one of [71] to [103], wherein the method of treatment is for treating ovarian cancer.
[110] The method of treatment according to [109], wherein the ovarian cancer is epithelial ovarian cancer, fallopian tube cancer, or primary peritoneal cancer.
[111] The method of treatment according to [109] or [110], wherein the ovarian cancer is metastatic.
[112] A method of treatment, comprising administering an anti-CDH6 antibody-drug conjugate and a VEGF inhibitor in combination to a subject in need of the treatment, wherein the anti-CDH6 antibody-drug conjugate is an antibody-drug conjugate represented by the formula:

[Formula 4]

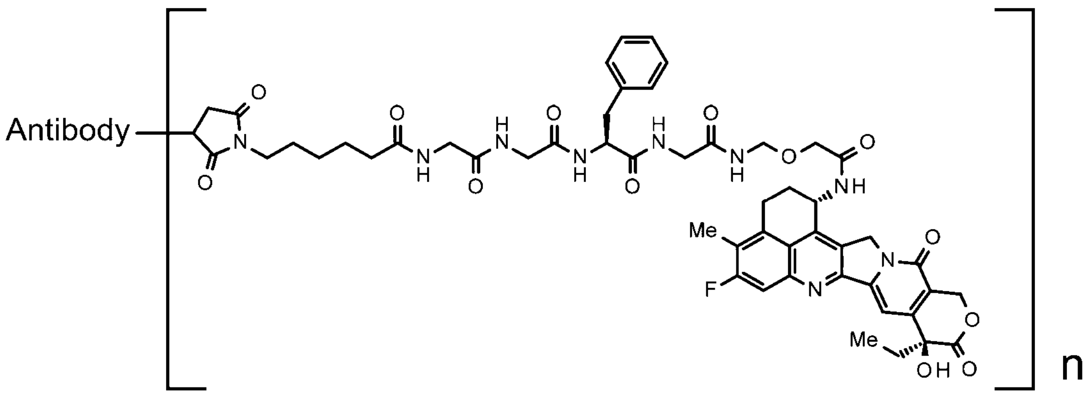

wherein Antibody is an anti-CDH6 antibody, a drug-linker is conjugated to the antibody via a thioether bond, and n represents an average number of units of the drug-linker conjugated per antibody.
[113] The method of treatment according to [112], wherein the anti-CDH6 antibody is an antibody comprising a heavy chain consisting of the amino acid sequence shown in SEQ ID NO: 29 and a light chain consisting of the amino acid sequence shown in SEQ ID NO: 38.
[114] The method of treatment according to [112] or [113], wherein a lysine residue is deleted from the carboxyl terminus of a heavy chain of the anti-CDH6 antibody.

[115] The method of treatment according to any one of [112] to [114], wherein the average number of units of the drug-linker conjugated per antibody in the anti-CDH6 antibody-drug conjugate is in the range of from 1 to 10.
[116] The method of treatment according to any one of [112] to [114], wherein the average number of units of the drug-linker conjugated per antibody in the anti-CDH6 antibody-drug conjugate is in the range of from 2 to 8.
[117] The method of treatment according to any one of [112] to [114], wherein the average number of units of the drug-linker conjugated per antibody in the anti-CDH6 antibody-drug conjugate is in the range of from 5 to 8.
[118] The method of treatment according to any one of [112] to [114], wherein the average number of units of the drug-linker conjugated per antibody in the anti-CDH6 antibody-drug conjugate is in the range of from 7 to 8.
[119] The method of treatment according to any one of [112] to [114], wherein the average number of units of the drug-linker conjugated per antibody in the anti-CDH6 antibody-drug conjugate is in the range of from 7.5 to 8.
[120] The method of treatment according to any one of [112] to [114], wherein the average number of units of the drug-linker conjugated per antibody in the anti-CDH6 antibody-drug conjugate is approximately 8.
[121] The method of treatment according to any one of [112] to [120], wherein the VEGF inhibitor is an anti-VEGF antibody, an anti-VEGF receptor antibody, a fusion protein comprising a VEGF receptor extracellular domain, or a multispecific antibody comprising an anti-VEGF binding domain.
[122] The method of treatment according to [121], wherein the VEGF inhibitor is an anti-VEGF antibody.
[123] The method of treatment according to [122], wherein the anti-VEGF antibody is bevacizumab.
[124] The method of treatment according to [121], wherein the VEGF inhibitor is an anti-VEGF receptor antibody.
[125] The method of treatment according to [124], wherein the anti-VEGF receptor antibody is ramucirumab.
[126] The method of treatment according to [121], wherein the VEGF inhibitor is a fusion protein comprising a VEGF receptor extracellular domain.
[127] The method of treatment according to [126], wherein the fusion protein comprising a VEGF receptor extracellular domain is aflibercept.
[128] The method of treatment according to [121], wherein the VEGF inhibitor is a multispecific antibody comprising an anti-VEGF binding domain.
[129] The method of treatment according to [128], wherein the multispecific antibody is a bispecific antibody.
[130] The method of treatment according to any one of [112] to [129], wherein the anti-CDH6 antibody-drug conjugate and the VEGF inhibitor are separately contained (as active components) in different formulations, and are administered at the same time (at approximately the same time) or at different times.
[131] The method of treatment according to any one of [112] to [129], wherein the anti-CDH6 antibody-drug conjugate and the VEGF inhibitor are contained (as active components) in a single formulation for administration.
[132] The method of treatment according to any one of [112] to [131], wherein the method of treatment is for treating cancer.
[133] The method of treatment according to any one of [112] to [131], wherein the method of treatment is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, and nasopharyngeal cancer.
[134] The method of treatment according to any one of [112] to [131], wherein the method of treatment is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.
[135] The method of treatment according to any one of [112] to [131], wherein the method of treatment is for treating at least one selected from the group consisting of renal cell carcinoma, renal clear cell carcinoma, and papillary renal cell carcinoma.
[136] The method of treatment according to any one of [112] to [131], wherein the method of treatment is for treating at least one selected from the group consisting of ovarian cancer, ovarian serous adenocarcinoma, ovarian clear cell carcinoma, endometrioid ovarian cancer, and ovarian mucinous tumor.
[137] The method of treatment according to any one of [112] to [131], wherein the method of treatment is for treating ovarian cancer.
[138] The method of treatment according to [137], wherein the ovarian cancer is epithelial ovarian cancer, fallopian tube cancer, or primary peritoneal cancer.
[139] The method of treatment according to [137] or [138], wherein the ovarian cancer is metastatic.
[140] The method of treatment according to any one of [71] to [139], wherein the anti-CDH6 antibody-drug conjugate is

raludotatug deruxtecan (DS-6000a).

[141] An anti-CDH6 antibody-drug conjugate for use in treating a disease by administering the anti-CDH6 antibody-drug conjugate and a VEGF inhibitor in combination, wherein in the anti-CDH6 antibody-drug conjugate, a drug-linker represented by the formula:

[Formula 5]

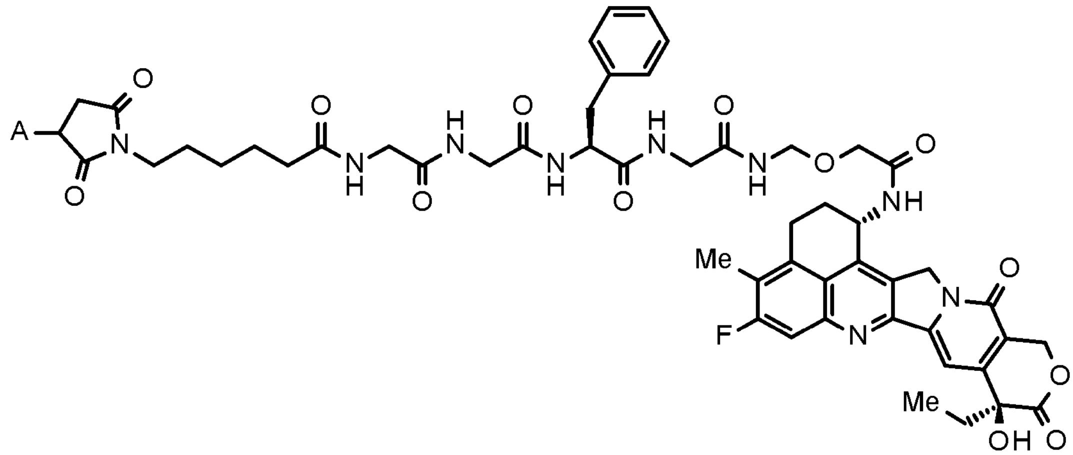

wherein A represents a connecting position to an anti-CDH6 antibody or a functional fragment of the antibody, is conjugated to the anti-CDH6 antibody or the functional fragment of the antibody via a thioether bond.

[142] The anti-CDH6 antibody-drug conjugate according to [141], wherein the anti-CDH6 antibody or the functional fragment of the antibody is an antibody which specifically binds to the amino acid sequence shown in SEQ ID NO: 1 and has an internalization ability that permits cellular uptake, or a functional fragment of the antibody.

[143] The anti-CDH6 antibody-drug conjugate according to [141] or [142], wherein the anti-CDH6 antibody or the functional fragment of the antibody is an antibody comprising

CDRH1, CDRH2 and CDRH3 in any one combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID NO: 4,
(2) CDRH1 consisting of the amino acid sequence shown in SEQ ID NO: 2, CDRH2 consisting of the amino acid sequence shown in SEQ ID NO: 8, and CDRH3 consisting of the amino acid sequence shown in SEQ ID NO: 4,
(3) CDRH1 consisting of the amino acid sequence shown in SEQ ID NO: 9, CDRH2 consisting of the amino acid sequence shown in SEQ ID NO: 10, and CDRH3 consisting of the amino acid sequence shown in SEQ ID NO: 11,
(4) CDRH1 consisting of the amino acid sequence shown in SEQ ID NO: 15, CDRH2 consisting of the amino acid sequence shown in SEQ ID NO: 16, and CDRH3 consisting of the amino acid sequence shown in SEQ ID NO: 17, and
(5) CDRH1 consisting of the amino acid sequence shown in SEQ ID NO: 21, CDRH2 consisting of the amino acid sequence shown in SEQ ID NO: 22, and CDRH3 consisting of the amino acid sequence shown in SEQ ID NO: 23, and CDRL1, CDRL2 and CDRL3 in any one combination selected from the group consisting of the following combinations (6) to (9):
(6) CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(7) CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(8) CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID

NO: 20, and

(9) CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,

or a functional fragment of the antibody.

[144] The anti-CDH6 antibody-drug conjugate according to any one of [141] to [143], wherein the anti-CDH6 antibody or the functional fragment of the antibody is an antibody comprising CDRH1, CDRH2 and CDRH3, and CDRL1, CDRL2 and CDRL3 in any combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID NO: 4, and CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(2) CDRH1 consisting of the amino acid sequence shown in SEQ ID NO: 2, CDRH2 consisting of the amino acid sequence shown in SEQ ID NO: 8, and CDRH3 consisting of the amino acid sequence shown in SEQ ID NO: 4, and CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(3) CDRH1 consisting of the amino acid sequence shown in SEQ ID NO: 9, CDRH2 consisting of the amino acid sequence shown in SEQ ID NO: 10, and CDRH3 consisting of the amino acid sequence shown in SEQ ID NO: 11, and CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(4) CDRH1 consisting of the amino acid sequence shown in SEQ ID NO: 15, CDRH2 consisting of the amino acid sequence shown in SEQ ID NO: 16, and CDRH3 consisting of the amino acid sequence shown in SEQ ID NO: 17, and CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(5) CDRH1 consisting of the amino acid sequence shown in SEQ ID NO: 21, CDRH2 consisting of the amino acid sequence shown in SEQ ID NO: 22, and CDRH3 consisting of the amino acid sequence shown in SEQ ID NO: 23, and CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,

or a functional fragment of the antibody.

[145] The anti-CDH6 antibody-drug conjugate according to any one of [141] to [144], wherein the anti-CDH6 antibody or the functional fragment of the antibody is an antibody comprising CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID NO: 4, and CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, or a functional fragment of the antibody.

[146] The anti-CDH6 antibody-drug conjugate according to any one of [141] to [145], wherein the anti-CDH6 antibody or the functional fragment of the antibody is a humanized antibody or functional fragment of the antibody.

[147] The anti-CDH6 antibody-drug conjugate according to any one of [141] to [146], wherein the anti-CDH6 antibody or the functional fragment of the antibody is an antibody comprising any one heavy chain variable region selected from the group consisting of the following variable regions (1) to (5):

(1) a heavy chain variable region consisting of the amino acid sequence shown in SEQ ID NO: 28,
(2) a heavy chain variable region consisting of the amino acid sequence shown in SEQ ID NO: 31,
(3) a heavy chain variable region consisting of the amino acid sequence shown in SEQ ID NO: 34,
(4) an amino acid sequence having a sequence identity of at least 95% or more to the sequence of the framework regions other than at each CDR sequence in the amino acid sequences of any one of (1) to (3), and
(5) an amino acid sequence comprising a deletion, substitution or addition of one or several amino acids in the sequence of the framework regions other than at each CDR sequence in the amino acid sequences of any one of (1) to (4), and

any one light chain variable region selected from the group consisting of the following variable regions (6) to (9):

(6) a light chain variable region consisting of the amino acid sequence shown in SEQ ID NO: 37,

(7) a light chain variable region consisting of the amino acid sequence shown in SEQ ID NO: 40,
(8) an amino acid sequence having a sequence identity of at least 95% or more to the sequence of the framework regions other than at each CDR sequence in the amino acid sequences of any one of (6) and (7), and
(9) an amino acid sequence comprising a deletion, substitution or addition of one or several amino acids in the sequence of the framework regions other than at each CDR sequence in the amino acid sequences of any one of (6) to (8),

or a functional fragment of the antibody.

[148] The anti-CDH6 antibody-drug conjugate according to any one of [141] to [147], wherein the anti-CDH6 antibody or the functional fragment of the antibody is an antibody comprising a heavy chain variable region and a light chain variable region in any of the following combinations (1) to (4):

(1) a heavy chain variable region consisting of the amino acid sequence shown in SEQ ID NO: 28 and a light chain variable region consisting of the amino acid sequence shown in SEQ ID NO: 37,
(2) a heavy chain variable region consisting of the amino acid sequence shown in SEQ ID NO: 31 and a light chain variable region consisting of the amino acid sequence shown in SEQ ID NO: 37,
(3) a heavy chain variable region consisting of the amino acid sequence shown in SEQ ID NO: 31 and a light chain variable region consisting of the amino acid sequence shown in SEQ ID NO: 40, and
(4) a heavy chain variable region consisting of the amino acid sequence shown in SEQ ID NO: 34 and a light chain variable region consisting of the amino acid sequence shown in SEQ ID NO: 37,

or a functional fragment of the antibody.
[149] The anti-CDH6 antibody-drug conjugate according to any one of [141] to [148], wherein the anti-CDH6 antibody or the functional fragment of the antibody is an antibody comprising a heavy chain variable region consisting of the amino acid sequence shown in SEQ ID NO: 28 and a light chain variable region consisting of the amino acid sequence shown in SEQ ID NO: 37, or a functional fragment of the antibody.
[150] The anti-CDH6 antibody-drug conjugate according to any one of [141] to [149], wherein the anti-CDH6 antibody or the functional fragment of the antibody is an antibody comprising any of the following combinations (1) to (4):

(1) a heavy chain consisting of the amino acid sequence shown in SEQ ID NO: 29 and a light chain consisting of the amino acid sequence shown in SEQ ID NO: 38,
(2) a heavy chain consisting of the amino acid sequence shown in SEQ ID NO: 32 and a light chain consisting of the amino acid sequence shown in SEQ ID NO: 38,
(3) a heavy chain consisting of the amino acid sequence shown in SEQ ID NO: 32 and a light chain consisting of the amino acid sequence shown in SEQ ID NO: 41, and
(4) a heavy chain consisting of the amino acid sequence shown in SEQ ID NO: 35 and a light chain consisting of the amino acid sequence shown in SEQ ID NO: 38,

or a functional fragment of the antibody.
[151] The anti-CDH6 antibody-drug conjugate according to any one of [141] to [150], wherein the anti-CDH6 antibody or the functional fragment of the antibody is an antibody comprising a heavy chain consisting of the amino acid sequence shown in SEQ ID NO: 29 and a light chain consisting of the amino acid sequence shown in SEQ ID NO: 38, or a functional fragment of the antibody.
[152] The anti-CDH6 antibody-drug conjugate according to any one of [141] to [151], wherein the anti-CDH6 antibody or the functional fragment of the antibody is an antibody in which the heavy chain or the light chain has undergone one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, conversion of N-terminal glutamine or N-terminal glutamic acid to pyroglutamic acid, and a deletion of one or two amino acids from the carboxyl terminus, or a functional fragment of the antibody.
[153] The anti-CDH6 antibody-drug conjugate according to [152], wherein the anti-CDH6 antibody or the functional fragment of the antibody is an antibody in which one or two amino acids are deleted from the carboxyl terminus of a heavy chain thereof, or a functional fragment of the antibody.
[154] The anti-CDH6 antibody-drug conjugate according to [153], wherein the anti-CDH6 antibody or the functional fragment of the antibody is an antibody in which one amino acid is deleted from the carboxyl terminus of each of the two heavy chains thereof, or a functional fragment of the antibody.

[155] The anti-CDH6 antibody-drug conjugate according to any one of [152] to [154], wherein the deleted amino acid is lysine.
[156] The anti-CDH6 antibody-drug conjugate according to any one of [152] to [155], wherein the anti-CDH6 antibody or the functional fragment of the antibody is an antibody in which a proline residue at the carboxyl terminus of a heavy chain thereof is further amidated, or a functional fragment of the antibody.
[157] The anti-CDH6 antibody-drug conjugate according to any one of [141] to [156], wherein the average number of units of the drug-linker conjugated per antibody in the anti-CDH6 antibody-drug conjugate is in the range of from 1 to 10.
[158] The anti-CDH6 antibody-drug conjugate according to any one of [141] to [156], wherein the average number of units of the drug-linker conjugated per antibody in the anti-CDH6 antibody-drug conjugate is in the range of from 2 to 8.
[159] The anti-CDH6 antibody-drug conjugate according to any one of [141] to [156], wherein the average number of units of the drug-linker conjugated per antibody in the anti-CDH6 antibody-drug conjugate is in the range of from 5 to 8.
[160] The anti-CDH6 antibody-drug conjugate according to any one of [141] to [156], wherein the average number of units of the drug-linker conjugated per antibody in the anti-CDH6 antibody-drug conjugate is in the range of from 7 to 8.
[161] The anti-CDH6 antibody-drug conjugate according to any one of [141] to [156], wherein the average number of units of the drug-linker conjugated per antibody in the anti-CDH6 antibody-drug conjugate is in the range of from 7.5 to 8.
[162] The anti-CDH6 antibody-drug conjugate according to any one of [141] to [156], wherein the average number of units of the drug-linker conjugated per antibody in the anti-CDH6 antibody-drug conjugate is approximately 8.
[163] The anti-CDH6 antibody-drug conjugate according to any one of [141] to [162], wherein the VEGF inhibitor is an anti-VEGF antibody, an anti-VEGF receptor antibody, a fusion protein comprising a VEGF receptor extracellular domain, or a multispecific antibody comprising an anti-VEGF binding domain.
[164] The anti-CDH6 antibody-drug conjugate according to [163], wherein the VEGF inhibitor is an anti-VEGF antibody.
[165] The anti-CDH6 antibody-drug conjugate according to [164], wherein the anti-VEGF antibody is bevacizumab.
[166] The anti-CDH6 antibody-drug conjugate according to [163], wherein the VEGF inhibitor is an anti-VEGF receptor antibody.
[167] The anti-CDH6 antibody-drug conjugate according to [166], wherein the anti-VEGF receptor antibody is ramucirumab.
[168] The anti-CDH6 antibody-drug conjugate according to [163], wherein the VEGF inhibitor is a fusion protein comprising a VEGF receptor extracellular domain.
[169] The anti-CDH6 antibody-drug conjugate according to [168], wherein the fusion protein comprising a VEGF receptor extracellular domain is aflibercept.
[170] The anti-CDH6 antibody-drug conjugate according to [163], wherein the VEGF inhibitor is a multispecific antibody comprising an anti-VEGF binding domain.
[171] The anti-CDH6 antibody-drug conjugate according to [170], wherein the multispecific antibody is a bispecific antibody.
[172] The anti-CDH6 antibody-drug conjugate according to any one of [141] to [171], wherein the anti-CDH6 antibody-drug conjugate and the VEGF inhibitor are separately contained (as active components) in different formulations, and are administered at the same time (at approximately the same time) or at different times.
[173] The anti-CDH6 antibody-drug conjugate according to any one of [141] to [171], wherein the anti-CDH6 antibody-drug conjugate and the VEGF inhibitor are contained (as active components) in a single formulation for administration.
[174] The anti-CDH6 antibody-drug conjugate according to any one of [141] to [173], wherein the anti-CDH6 antibody-drug conjugate is for use in treating cancer.
[175] The anti-CDH6 antibody-drug conjugate according to any one of [141] to [173], wherein the anti-CDH6 antibody-drug conjugate is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, and nasopharyngeal cancer.
[176] The anti-CDH6 antibody-drug conjugate according to any one of [141] to [173], wherein the anti-CDH6 antibody-drug conjugate is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.
[177] The anti-CDH6 antibody-drug conjugate according to any one of [141] to [173], wherein the anti-CDH6 antibody-drug conjugate is for use in treating at least one selected from the group consisting of renal cell carcinoma, renal clear cell carcinoma, and papillary renal cell carcinoma.

[178] The anti-CDH6 antibody-drug conjugate according to any one of [141] to [173], wherein the anti-CDH6 antibody-drug conjugate is for use in treating at least one selected from the group consisting of ovarian cancer, ovarian serous adenocarcinoma, ovarian clear cell carcinoma, endometrioid ovarian cancer, and ovarian mucinous tumor.

[179] The anti-CDH6 antibody-drug conjugate according to any one of [141] to [173], wherein the anti-CDH6 antibody-drug conjugate is for use in treating ovarian cancer.

[180] The anti-CDH6 antibody-drug conjugate according to [179], wherein the ovarian cancer is epithelial ovarian cancer, fallopian tube cancer, or primary peritoneal cancer.

[181] The anti-CDH6 antibody-drug conjugate according to [179] or [180], wherein the ovarian cancer is metastatic.

[182] An anti-CDH6 antibody-drug conjugate for use in treating a disease by administering the anti-CDH6 antibody-drug conjugate and a VEGF inhibitor in combination, wherein the anti-CDH6 antibody-drug conjugate is represented by the formula:

[Formula 6]

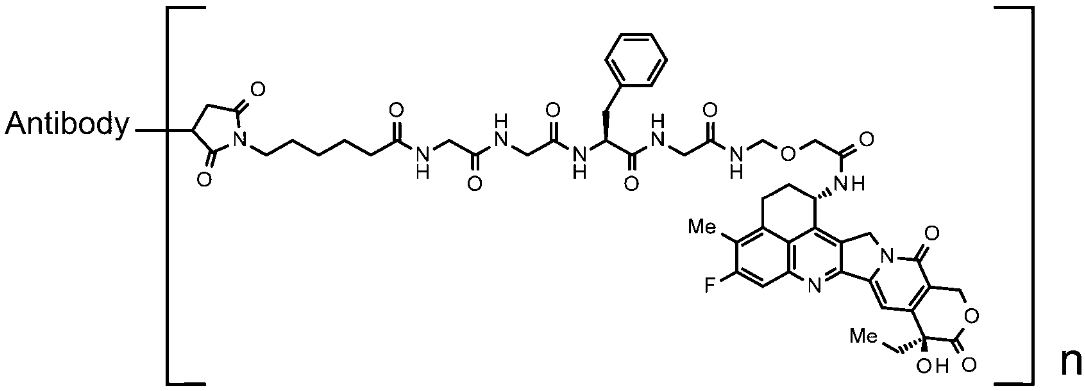

wherein Antibody is an anti-CDH6 antibody, a drug-linker is conjugated to the antibody via a thioether bond, and n represents an average number of units of the drug-linker conjugated per antibody.

[183] The anti-CDH6 antibody-drug conjugate according to [182], wherein the anti-CDH6 antibody is an antibody comprising a heavy chain consisting of the amino acid sequence shown in SEQ ID NO: 29 and a light chain consisting of the amino acid sequence shown in SEQ ID NO: 38.

[184] The anti-CDH6 antibody-drug conjugate according to [182] or [183], wherein a lysine residue is deleted from the carboxyl terminus of a heavy chain of the anti-CDH6 antibody.

[185] The anti-CDH6 antibody-drug conjugate according to any one of [182] to [184], wherein the average number of units of the drug-linker conjugated per antibody in the anti-CDH6 antibody-drug conjugate is in the range of from 1 to 10.

[186] The anti-CDH6 antibody-drug conjugate according to any one of [182] to [184], wherein the average number of units of the drug-linker conjugated per antibody in the anti-CDH6 antibody-drug conjugate is in the range of from 2 to 8.

[187] The anti-CDH6 antibody-drug conjugate according to any one of [182] to [184], wherein the average number of units of the drug-linker conjugated per antibody in the anti-CDH6 antibody-drug conjugate is in the range of from 5 to 8.

[188] The anti-CDH6 antibody-drug conjugate according to any one of [182] to [184], wherein the average number of units of the drug-linker conjugated per antibody in the anti-CDH6 antibody-drug conjugate is in the range of from 7 to 8.

[189] The anti-CDH6 antibody-drug conjugate according to any one of [182] to [184], wherein the average number of units of the drug-linker conjugated per antibody in the anti-CDH6 antibody-drug conjugate is in the range of from 7.5 to 8.

[190] The anti-CDH6 antibody-drug conjugate according to any one of [182] to [184], wherein the average number of units of the drug-linker conjugated per antibody in the anti-CDH6 antibody-drug conjugate is approximately 8.

[191] The anti-CDH6 antibody-drug conjugate according to any one of [182] to [190], wherein the VEGF inhibitor is an anti-VEGF antibody, an anti-VEGF receptor antibody, a fusion protein comprising a VEGF receptor extracellular domain, or a multispecific antibody comprising an anti-VEGF binding domain.

[192] The anti-CDH6 antibody-drug conjugate according to [191], wherein the VEGF inhibitor is an anti-VEGF antibody.

[193] The anti-CDH6 antibody-drug conjugate according to [192], wherein the anti-VEGF antibody is bevacizumab.

[194] The anti-CDH6 antibody-drug conjugate according to [191], wherein the VEGF inhibitor is an anti-VEGF

receptor antibody.

[195] The anti-CDH6 antibody-drug conjugate according to [194], wherein the anti-VEGF receptor antibody is ramucirumab.

[196] The anti-CDH6 antibody-drug conjugate according to [191], wherein the VEGF inhibitor is a fusion protein comprising a VEGF receptor extracellular domain.

[197] The anti-CDH6 antibody-drug conjugate according to [196], wherein the fusion protein comprising a VEGF receptor extracellular domain is aflibercept.

[198] The anti-CDH6 antibody-drug conjugate according to [191], wherein the VEGF inhibitor is a multispecific antibody comprising an anti-VEGF binding domain.

[199] The anti-CDH6 antibody-drug conjugate according to [198], wherein the multispecific antibody is a bispecific antibody.

[200] The anti-CDH6 antibody-drug conjugate according to any one of [182] to [199], wherein the anti-CDH6 antibody-drug conjugate and the VEGF inhibitor are separately contained (as active components) in different formulations, and are administered at the same time (at approximately the same time) or at different times.

[201] The anti-CDH6 antibody-drug conjugate according to any one of [182] to [199], wherein the anti-CDH6 antibody-drug conjugate and the VEGF inhibitor are contained (as active components) in a single formulation for administration.

[202] The anti-CDH6 antibody-drug conjugate according to any one of [182] to [201], wherein the anti-CDH6 antibody-drug conjugate is for use in treating cancer.

[203] The anti-CDH6 antibody-drug conjugate according to any one of [182] to [201], wherein the anti-CDH6 antibody-drug conjugate is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, and nasopharyngeal cancer.

[204] The anti-CDH6 antibody-drug conjugate according to any one of [182] to [201], wherein the anti-CDH6 antibody-drug conjugate is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

[205] The anti-CDH6 antibody-drug conjugate according to any one of [182] to [201], wherein the anti-CDH6 antibody-drug conjugate is for use in treating at least one selected from the group consisting of renal cell carcinoma, renal clear cell carcinoma, and papillary renal cell carcinoma.

[206] The anti-CDH6 antibody-drug conjugate according to any one of [182] to [201], wherein the anti-CDH6 antibody-drug conjugate is for use in treating at least one selected from the group consisting of ovarian cancer, ovarian serous adenocarcinoma, ovarian clear cell carcinoma, endometrioid ovarian cancer, and ovarian mucinous tumor.

[207] The anti-CDH6 antibody-drug conjugate according to any one of [182] to [201], wherein the anti-CDH6 antibody-drug conjugate is for use in treating ovarian cancer.

[208] The anti-CDH6 antibody-drug conjugate according to [207], wherein the ovarian cancer is epithelial ovarian cancer, fallopian tube cancer, or primary peritoneal cancer.

[209] The anti-CDH6 antibody-drug conjugate according to [207] or [208], wherein the ovarian cancer is metastatic.

[210] The anti-CDH6 antibody-drug conjugate according to any one of [141] to [209], wherein the anti-CDH6 antibody-drug conjugate is raludotatug deruxtecan (DS-6000a) .

[211] Use of an anti-CDH6 antibody-drug conjugate for the manufacture of a medicament for treating a disease through being administered in combination with a VEGF inhibitor, wherein in the anti-CDH6 antibody-drug conjugate, a drug-linker represented by the formula:

[Formula 7]

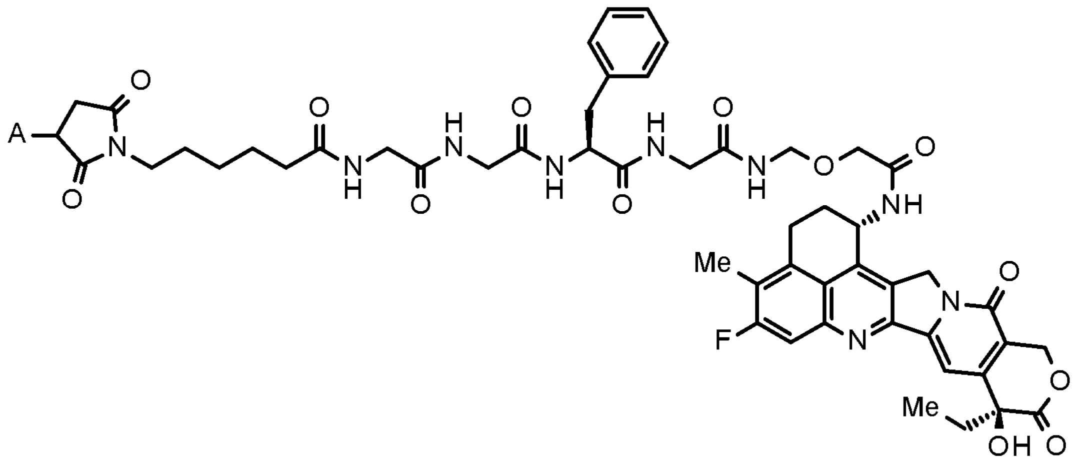

wherein A represents a connecting position to an anti-CDH6 antibody or a functional fragment of the antibody, is conjugated to the anti-CDH6 antibody or the functional fragment of the antibody via a thioether bond.

[212] The use according to [211], wherein the anti-CDH6 antibody or the functional fragment of the antibody is an antibody which specifically binds to the amino acid sequence shown in SEQ ID NO: 1 and has an internalization ability that permits cellular uptake, or a functional fragment of the antibody.

[213] The use according to [211] or [212], wherein the anti-CDH6 antibody or the functional fragment of the antibody is an antibody comprising CDRH1, CDRH2 and CDRH3 in any one combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID NO: 4,
(2) CDRH1 consisting of the amino acid sequence shown in SEQ ID NO: 2, CDRH2 consisting of the amino acid sequence shown in SEQ ID NO: 8, and CDRH3 consisting of the amino acid sequence shown in SEQ ID NO: 4,
(3) CDRH1 consisting of the amino acid sequence shown in SEQ ID NO: 9, CDRH2 consisting of the amino acid sequence shown in SEQ ID NO: 10, and CDRH3 consisting of the amino acid sequence shown in SEQ ID NO: 11,
(4) CDRH1 consisting of the amino acid sequence shown in SEQ ID NO: 15, CDRH2 consisting of the amino acid sequence shown in SEQ ID NO: 16, and CDRH3 consisting of the amino acid sequence shown in SEQ ID NO: 17, and
(5) CDRH1 consisting of the amino acid sequence shown in SEQ ID NO: 21, CDRH2 consisting of the amino acid sequence shown in SEQ ID NO: 22, and CDRH3 consisting of the amino acid sequence shown in SEQ ID NO: 23, and CDRL1, CDRL2 and CDRL3 in any one combination selected from the group consisting of the following combinations (6) to (9):
(6) CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(7) CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(8) CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(9) CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,

or a functional fragment of the antibody.

[214] The use according to any one of [211] to [213], wherein the anti-CDH6 antibody or the functional fragment of the antibody is an antibody comprising CDRH1, CDRH2 and CDRH3, and CDRL1, CDRL2 and CDRL3, in any combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(2) CDRH1 consisting of the amino acid sequence shown in SEQ ID NO: 2, CDRH2 consisting of the amino acid

sequence shown in SEQ ID NO: 8, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(3) CDRH1 consisting of the amino acid sequence shown in SEQ ID NO: 9, CDRH2 consisting of the amino acid sequence shown in SEQ ID NO: 10, CDRH3 consisting of the amino acid sequence shown in SEQ ID NO: 11, CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(4) CDRH1 consisting of the amino acid sequence shown in SEQ ID NO: 15, CDRH2 consisting of the amino acid sequence shown in SEQ ID NO: 16, CDRH3 consisting of the amino acid sequence shown in SEQ ID NO: 17, CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(5) CDRH1 consisting of the amino acid sequence shown in SEQ ID NO: 21, CDRH2 consisting of the amino acid sequence shown in SEQ ID NO: 22, CDRH3 consisting of the amino acid sequence shown in SEQ ID NO: 23, CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,

or a functional fragment of the antibody.
[215] The use according to any one of [211] to [214], wherein the anti-CDH6 antibody or the functional fragment of the antibody is an antibody comprising CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, or a functional fragment of the antibody.
[216] The use according to any one of [211] to [215], wherein the anti-CDH6 antibody or the functional fragment of the antibody is a humanized antibody or functional fragment of the antibody.
[217] The use according to any one of [211] to [216], wherein the anti-CDH6 antibody or the functional fragment of the antibody is an antibody comprising any one heavy chain variable region selected from the group consisting of the following variable regions (1) to (5):

(1) a heavy chain variable region consisting of the amino acid sequence shown in SEQ ID NO: 28,
(2) a heavy chain variable region consisting of the amino acid sequence shown in SEQ ID NO: 31,
(3) a heavy chain variable region consisting of the amino acid sequence shown in SEQ ID NO: 34,
(4) an amino acid sequence having a sequence identity of at least 95% or more to the sequence of the framework regions other than at each CDR sequence in the amino acid sequences of any one of (1) to (3), and
(5) an amino acid sequence comprising a deletion, substitution or addition of one or several amino acids in the sequence of the framework regions other than at each CDR sequence in the amino acid sequences of any one of (1) to (4), and

any one light chain variable region selected from the group consisting of the following variable regions (6) to (9):

(6) a light chain variable region consisting of the amino acid sequence shown in SEQ ID NO: 37,
(7) a light chain variable region consisting of the amino acid sequence shown in SEQ ID NO: 40,
(8) an amino acid sequence having a sequence identity of at least 95% or more to the sequence of the framework regions other than at each CDR sequence in the amino acid sequences of any one of (6) and (7), and
(9) an amino acid sequence comprising a deletion, substitution or addition of one or several amino acids in the sequence of the framework regions other than at each CDR sequence in the amino acid sequences of any one of (6) to (8),

or a functional fragment of the antibody.

[218] The use according to any one of [211] to [217], wherein the anti-CDH6 antibody or the functional fragment of the antibody is an antibody comprising a heavy chain variable region and a light chain variable region in any of the following combinations (1) to (4):

(1) a heavy chain variable region consisting of the amino acid sequence shown in SEQ ID NO: 28 and a light chain variable region consisting of the amino acid sequence shown in SEQ ID NO: 37,

(2) a heavy chain variable region consisting of the amino acid sequence shown in SEQ ID NO: 31 and a light chain variable region consisting of the amino acid sequence shown in SEQ ID NO: 37,
(3) a heavy chain variable region consisting of the amino acid sequence shown in SEQ ID NO: 31 and a light chain variable region consisting of the amino acid sequence shown in SEQ ID NO: 40, and
(4) a heavy chain variable region consisting of the amino acid sequence shown in SEQ ID NO: 34 and a light chain variable region consisting of the amino acid sequence shown in SEQ ID NO: 37,

or a functional fragment of the antibody.
[219] The use according to any one of [211] to [218], wherein the anti-CDH6 antibody or the functional fragment of the antibody is an antibody comprising a heavy chain variable region consisting of the amino acid sequence shown in SEQ ID NO: 28 and a light chain variable region consisting of the amino acid sequence shown in SEQ ID NO: 37, or a functional fragment of the antibody.
[220] The use according to any one of [211] to [219], wherein the anti-CDH6 antibody or the functional fragment of the antibody is an antibody comprising any of the following combinations (1) to (4):

(1) a heavy chain consisting of the amino acid sequence shown in SEQ ID NO: 29 and a light chain consisting of the amino acid sequence shown in SEQ ID NO: 38,
(2) a heavy chain consisting of the amino acid sequence shown in SEQ ID NO: 32 and a light chain consisting of the amino acid sequence shown in SEQ ID NO: 38,
(3) a heavy chain consisting of the amino acid sequence shown in SEQ ID NO: 32 and a light chain consisting of the amino acid sequence shown in SEQ ID NO: 41, and
(4) a heavy chain consisting of the amino acid sequence shown in SEQ ID NO: 35 and a light chain consisting of the amino acid sequence shown in SEQ ID NO: 38,

or a functional fragment of the antibody.
[221] The use according to any one of [211] to [220], wherein the anti-CDH6 antibody or the functional fragment of the antibody is an antibody comprising a heavy chain consisting of the amino acid sequence shown in SEQ ID NO: 29 and a light chain consisting of the amino acid sequence shown in SEQ ID NO: 38, or a functional fragment of the antibody.
[222] The use according to any one of [211] to [221], wherein the anti-CDH6 antibody or the functional fragment of the antibody is an antibody in which the heavy chain or the light chain has undergone one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, conversion of N-terminal glutamine or N-terminal glutamic acid to pyroglutamic acid, and a deletion of one or two amino acids from the carboxyl terminus,
or a functional fragment of the antibody.
[223] The use according to [222], wherein the anti-CDH6 antibody or the functional fragment of the antibody is an antibody in which one or two amino acids are deleted from the carboxyl terminus of a heavy chain thereof, or a functional fragment of the antibody.
[224] The use according to [223], wherein the anti-CDH6 antibody or the functional fragment of the antibody is an antibody in which one amino acid is deleted from the carboxyl terminus of each of the two heavy chains thereof, or a functional fragment of the antibody.
[225] The use according to any one of [222] to [224], wherein the deleted amino acid is lysine.
[226] The use according to any one of [222] to [225], wherein the anti-CDH6 antibody or the functional fragment of the antibody is an antibody in which a proline residue at the carboxyl terminus of a heavy chain thereof is further amidated, or a functional fragment of the antibody.
[227] The use according to any one of [211] to [226], wherein the average number of units of the drug-linker conjugated per antibody in the anti-CDH6 antibody-drug conjugate is in the range of from 1 to 10.
[228] The use according to any one of [211] to [226], wherein the average number of units of the drug-linker conjugated per antibody in the anti-CDH6 antibody-drug conjugate is in the range of from 2 to 8.
[229] The use according to any one of [211] to [226], wherein the average number of units of the drug-linker conjugated per antibody in the anti-CDH6 antibody-drug conjugate is in the range of from 5 to 8.
[230] The use according to any one of [211] to [226], wherein the average number of units of the drug-linker conjugated per antibody in the anti-CDH6 antibody-drug conjugate is in the range of from 7 to 8.
[231] The use according to any one of [211] to [226], wherein the average number of units of the drug-linker conjugated per antibody in the anti-CDH6 antibody-drug conjugate is in the range of from 7.5 to 8.
[232] The use according to any one of [211] to [226], wherein the average number of units of the drug-linker conjugated per antibody in the anti-CDH6 antibody-drug conjugate is approximately 8.
[233] The use according to any one of [211] to [232], wherein the VEGF inhibitor is an anti-VEGF antibody, an anti-VEGF receptor antibody, a fusion protein comprising a VEGF receptor extracellular domain, or a multispecific antibody comprising an anti-VEGF binding domain.

[234] The use according to [233], wherein the VEGF inhibitor is an anti-VEGF antibody.

[235] The use according to [234], wherein the anti-VEGF antibody is bevacizumab.

[236] The use according to [233], wherein the VEGF inhibitor is an anti-VEGF receptor antibody.

[237] The use according to [236], wherein the anti-VEGF receptor antibody is ramucirumab.

[238] The use according to [233], wherein the VEGF inhibitor is a fusion protein comprising a VEGF receptor extracellular domain.

[239] The use according to [238], wherein the fusion protein comprising a VEGF receptor extracellular domain is aflibercept.

[240] The use according to [233], wherein the VEGF inhibitor is a multispecific antibody comprising an anti-VEGF binding domain.

[241] The use according to [240], wherein the multispecific antibody is a bispecific antibody.

[242] The use according to any one of [211] to [241], wherein the anti-CDH6 antibody-drug conjugate and the VEGF inhibitor are separately contained (as active components) in different formulations, and are administered at the same time (at approximately the same time) or at different times.

[243] The use according to any one of [211] to [241], wherein the anti-CDH6 antibody-drug conjugate and the VEGF inhibitor are contained (as active components) in a single formulation for administration.

[244] The use according to any one of [211] to [243], wherein the use is for treating cancer.

[245] The use according to any one of [211] to [243], wherein the use is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, and nasopharyngeal cancer.

[246] The use according to any one of [211] to [243], wherein the use is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

[247] The use according to any one of [211] to [243], wherein the use is for treating at least one selected from the group consisting of renal cell carcinoma, renal clear cell carcinoma, and papillary renal cell carcinoma.

[248] The use according to any one of [211] to [243], wherein the use is for treating at least one selected from the group consisting of ovarian cancer, ovarian serous adenocarcinoma, ovarian clear cell carcinoma, endometrioid ovarian cancer, and ovarian mucinous tumor.

[249] The use according to any one of [211] to [243], wherein the use is for treating ovarian cancer.

[250] The use according to [249], wherein the ovarian cancer is epithelial ovarian cancer, fallopian tube cancer, or primary peritoneal cancer.

[251] The use according to [249] or [250], wherein the ovarian cancer is metastatic.

[252] Use of an anti-CDH6 antibody-drug conjugate for the manufacture of a medicament for treating a disease through being administered in combination with a VEGF inhibitor, wherein the anti-CDH6 antibody-drug conjugate is represented by the formula:

[Formula 8]

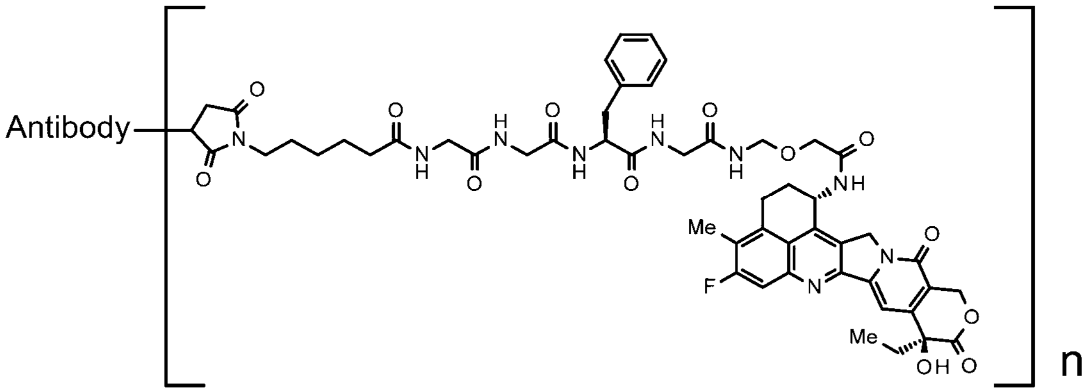

wherein Antibody is an anti-CDH6 antibody, a drug-linker is conjugated to the antibody via a thioether bond, and n represents an average number of units of the drug-linker conjugated per antibody.

[253] The use according to [252], wherein the anti-CDH6 antibody is an antibody comprising a heavy chain consisting of the amino acid sequence shown in SEQ ID NO: 29 and a light chain consisting of the amino acid sequence shown in SEQ ID NO: 38.

[254] The use according to [252] or [253], wherein a lysine residue is deleted from the carboxyl terminus of a heavy chain of the anti-CDH6 antibody.

[255] The use according to any one of [252] to [254], wherein the average number of units of the drug-linker conjugated per antibody in the anti-CDH6 antibody-drug conjugate is in the range of from 1 to 10.

[256] The use according to any one of [252] to [254], wherein the average number of units of the drug-linker conjugated per antibody in the anti-CDH6 antibody-drug conjugate is in the range of from 2 to 8.

[257] The use according to any one of [252] to [254], wherein the average number of units of the drug-linker conjugated per antibody in the anti-CDH6 antibody-drug conjugate is in the range of from 5 to 8.

[258] The use according to any one of [252] to [254], wherein the average number of units of the drug-linker conjugated per antibody in the anti-CDH6 antibody-drug conjugate is in the range of from 7 to 8.

[259] The use according to any one of [252] to [254], wherein the average number of units of the drug-linker conjugated per antibody in the anti-CDH6 antibody-drug conjugate is in the range of from 7.5 to 8.

[260] The use according to any one of [252] to [254], wherein the average number of units of the drug-linker conjugated per antibody in the anti-CDH6 antibody-drug conjugate is approximately 8.

[261] The use according to any one of [252] to [260], wherein the VEGF inhibitor is an anti-VEGF antibody, an anti-VEGF receptor antibody, a fusion protein comprising a VEGF receptor extracellular domain, or a multispecific antibody comprising an anti-VEGF binding domain.

[262] The use according to [261], wherein the VEGF inhibitor is an anti-VEGF antibody.

[263] The use according to [262], wherein the anti-VEGF antibody is bevacizumab.

[264] The use according to [261], wherein the VEGF inhibitor is an anti-VEGF receptor antibody.

[265] The use according to [264], wherein the anti-VEGF receptor antibody is ramucirumab.

[266] The use according to [261], wherein the VEGF inhibitor is a fusion protein comprising a VEGF receptor extracellular domain.

[267] The use according to [266], wherein the fusion protein comprising a VEGF receptor extracellular domain is aflibercept.

[268] The use according to [261], wherein the VEGF inhibitor is a multispecific antibody comprising an anti-VEGF binding domain.

[269] The use according to [268], wherein the multispecific antibody is a bispecific antibody.

[270] The use according to any one of [252] to [269], wherein the anti-CDH6 antibody-drug conjugate and the VEGF inhibitor are separately contained (as active components) in different formulations, and are administered at the same time (at approximately the same time) or at different times.

[271] The use according to any one of [252] to [269], wherein the anti-CDH6 antibody-drug conjugate and the VEGF inhibitor are contained (as active components) in a single formulation for administration.

[272] The use according to any one of [252] to [271], wherein the use is for treating cancer.

[273] The use according to any one of [252] to [271], wherein the use is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid

cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, and nasopharyngeal cancer.

[274] The use according to any one of [252] to [271], wherein the use is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

[275] The use according to any one of [252] to [271], wherein the use is for treating at least one selected from the group consisting of renal cell carcinoma, renal clear cell carcinoma, and papillary renal cell carcinoma.

[276] The use according to any one of [252] to [271], wherein the use is for treating at least one selected from the group consisting of ovarian cancer, ovarian serous adenocarcinoma, ovarian clear cell carcinoma, endometrioid ovarian cancer, and ovarian mucinous tumor.

[277] The use according to any one of [252] to [271], wherein the use is for treating ovarian cancer.

[278] The use according to [277], wherein the ovarian cancer is epithelial ovarian cancer, fallopian tube cancer, or primary peritoneal cancer.

[279] The use according to [277] or [278], wherein the ovarian cancer is metastatic.

[280] The use according to any one of [211] to [279], wherein the anti-CDH6 antibody-drug conjugate is raludotatug deruxtecan (DS-6000a).

[281] A pharmaceutical product comprising an anti-CDH6 antibody-drug conjugate and a VEGF inhibitor for administration in combination, wherein the anti-CDH6 antibody-drug conjugate and the VEGF inhibitor are as defined in any one of [1] to [31], [42] to [59] and [70].

[282] The pharmaceutical product according to [281], wherein the pharmaceutical product is for use in treating a disease as defined in any one of [34] to [41] and [62] to [69].

[283] The pharmaceutical product according to [281] or [282], wherein the anti-CDH6 antibody-drug conjugate and the VEGF inhibitor are separately contained (as active components) in different formulations, and are administered at the same time (at approximately the same time) or at different times.

[284] The pharmaceutical product according to [281] or [282], wherein the anti-CDH6 antibody-drug conjugate and the VEGF inhibitor are contained (as active components) in a single formulation for administration.

[285] A combination medicament comprising an anti-CDH6 antibody-drug conjugate and a VEGF inhibitor, wherein the anti-CDH6 antibody-drug conjugate and the VEGF inhibitor are as defined in any one of [1] to [31], [42] to [59] and [70].

[286] The combination medicament according to [285], wherein the combination medicament is for use in treating a disease as defined in any one of [34] to [41] and [62] to [69].

[287] The combination medicament according to [285] or [286], wherein the anti-CDH6 antibody-drug conjugate and the VEGF inhibitor are separately contained (as active components) in different formulations, and are administered at the same time (at approximately the same time) or at different times.

[288] The combination medicament according to [285] or [286], wherein the anti-CDH6 antibody-drug conjugate and the VEGF inhibitor are contained (as active components) in a single formulation for administration.

[289] A pharmaceutical combination comprising an anti-CDH6 antibody-drug conjugate and a VEGF inhibitor, wherein the anti-CDH6 antibody-drug conjugate and the VEGF inhibitor are as defined in any one of [1] to [31], [42] to [59] and [70].

[290] The pharmaceutical combination according to [289], wherein the pharmaceutical combination is for use in treating a disease as defined in any one of [34] to [41] and [62] to [69].

[291] The pharmaceutical combination according to [289] or [290], wherein the anti-CDH6 antibody-drug conjugate and the VEGF inhibitor are separately contained (as active components) in different formulations, and are administered at the same time (at approximately the same time) or at different times.

[292] The pharmaceutical combination according to [289] or [290], wherein the anti-CDH6 antibody-drug conjugate and the VEGF inhibitor are contained (as active components) in a single formulation for administration.

[293] Use of an anti-CDH6 antibody-drug conjugate combined with a VEGF inhibitor for treatment of a disease, wherein the anti-CDH6 antibody-drug conjugate and the VEGF inhibitor are as defined in any one of [1] to [31], [42] to [59] and [70].

[294] The use according to [293], wherein the disease is as defined in any one of [34] to [41] and [62] to [69].

[295] The use according to [293] or [294], wherein the anti-CDH6 antibody-drug conjugate and the VEGF inhibitor are separately contained (as active components) in different formulations, and are administered at the same time (at approximately the same time) or at different times.

[296] The use according to [293] or [294], wherein the anti-CDH6 antibody-drug conjugate and the VEGF inhibitor are contained (as active components) in a single formulation for administration.
[297] A medicament comprising an anti-CDH6 antibody-drug conjugate and a VEGF inhibitor, wherein the anti-CDH6 antibody-drug conjugate and the VEGF inhibitor are as defined in any one of [1] to [31], [42] to [59] and [70].
[298] The medicament according to [297], wherein the medicament is for use in treating a disease as defined in any one of [34] to [41] and [62] to [69].
[299] The medicament according to [297] or [298], wherein the anti-CDH6 antibody-drug conjugate and the VEGF inhibitor are separately contained (as active components) in different formulations, and are administered at the same time (at approximately the same time) or at different times.
[300] The medicament according to [297] or [298], wherein the anti-CDH6 antibody-drug conjugate and the VEGF inhibitor are contained (as active components) in a single formulation for administration.
[301] A pharmaceutical composition comprising (i) an anti-CDH6 antibody-drug conjugate and (ii) a VEGF inhibitor, wherein the anti-CDH6 antibody-drug conjugate and the VEGF inhibitor are administered in combination, wherein the anti-CDH6 antibody-drug conjugate and the VEGF inhibitor are as defined in any one of [1] to [31], [42] to [59] and [70].
[302] The pharmaceutical composition according to [301], wherein the pharmaceutical composition is for use in treating a disease as defined in any one of [34] to [41] and [62] to [69].
[303] The pharmaceutical composition according to [301] or [302], wherein the anti-CDH6 antibody-drug conjugate and the VEGF inhibitor are separately contained (as active components) in different formulations, and are administered at the same time (at approximately the same time) or at different times.
[304] The pharmaceutical composition according to [301] or [302], wherein the anti-CDH6 antibody-drug conjugate and the VEGF inhibitor are contained (as active components) in a single formulation for administration.
[305] A kit comprising (i) a first composition comprising an anti-CDH6 antibody-drug conjugate and (ii) a second composition comprising a VEGF inhibitor, wherein the anti-CDH6 antibody-drug conjugate and the VEGF inhibitor are administered in combination, wherein the anti-CDH6 antibody-drug conjugate and the VEGF inhibitor are as defined in any one of [1] to [31], [42] to [59] and [70].
[306] The kit according to [305], wherein the kit is for use in treating a disease as defined in any one of [34] to [41] and [62] to [69].
[307] The kit according to [305] or [306], wherein the anti-CDH6 antibody-drug conjugate and the VEGF inhibitor are separately contained (as active components) in different formulations, and are administered at the same time (at approximately the same time) or at different times.
[308] The kit according to [305] or [306], wherein the anti-CDH6 antibody-drug conjugate and the VEGF inhibitor are contained (as active components) in a single formulation for administration.
[309] A method of treatment for cancer, comprising administering an anti-CDH6 antibody-drug conjugate and a VEGF inhibitor in combination to a subject in need of the treatment, wherein the anti-CDH6 antibody-drug conjugate and the VEGF inhibitor are as defined in any one of [1] to [31], [42] to [59] and [70].

[Advantageous Effects of Invention]

The present invention can provide a pharmaceutical composition wherein a specific anti-CDH6 antibody-drug conjugate and a VEGF inhibitor are administered in combination, and/or a method of treatment comprising administering a specific anti-CDH6 antibody-drug conjugate and a VEGF inhibitor in combination to a subject.

[Brief Description of Drawings]

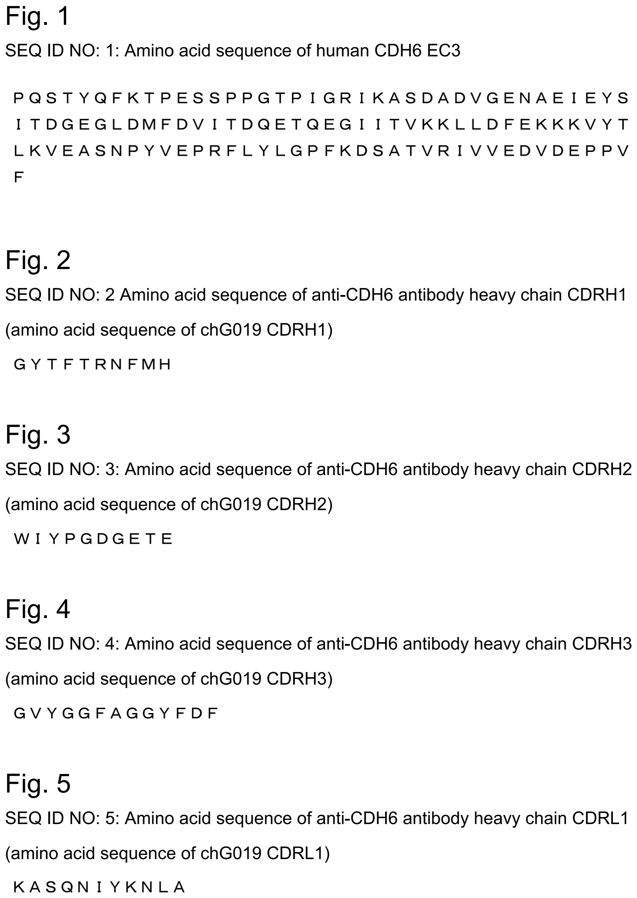

[Figure 1] Figure 1 is a diagram showing the amino acid sequence (SEQ ID NO: 1) of human CDH6 EC3.
[Figure 2] Figure 2 is a diagram showing the amino acid sequence (SEQ ID NO: 2) of an anti-CDH6 antibody heavy chain CDRH1.
[Figure 3] Figure 3 is a diagram showing the amino acid sequence (SEQ ID NO: 3) of an anti-CDH6 antibody heavy chain CDRH2.
[Figure 4] Figure 4 is a diagram showing the amino acid sequence (SEQ ID NO: 4) of an anti-CDH6 antibody heavy chain CDRH3.
[Figure 5] Figure 5 is a diagram showing the amino acid sequence (SEQ ID NO: 5) of an anti-CDH6 antibody light chain CDRL1.

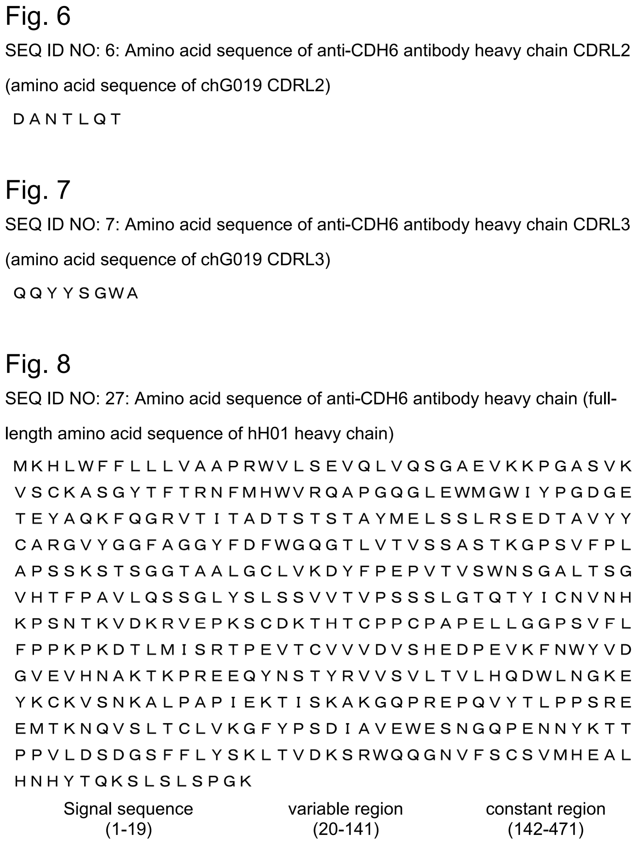

[Figure 6] Figure 6 is a diagram showing the amino acid sequence (SEQ ID NO: 6) of an anti-CDH6 antibody light chain CDRL2.
[Figure 7] Figure 7 is a diagram showing the amino acid sequence (SEQ ID NO: 7) of an anti-CDH6 antibody light chain

CDRL3.

[Figure 8] Figure 8 is a diagram showing the amino acid sequence (SEQ ID NO: 27) of an anti-CDH6 antibody heavy chain.

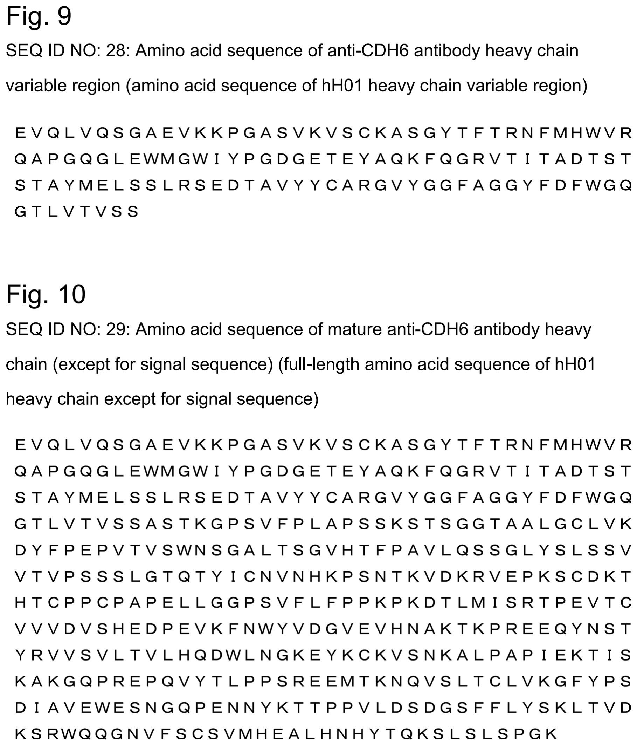

[Figure 9] Figure 9 is a diagram showing the amino acid sequence (SEQ ID NO: 28) of an anti-CDH6 antibody heavy chain variable region.

[Figure 10] Figure 10 is a diagram showing the amino acid sequence (SEQ ID NO: 29) of a mature anti-CDH6 antibody heavy chain.

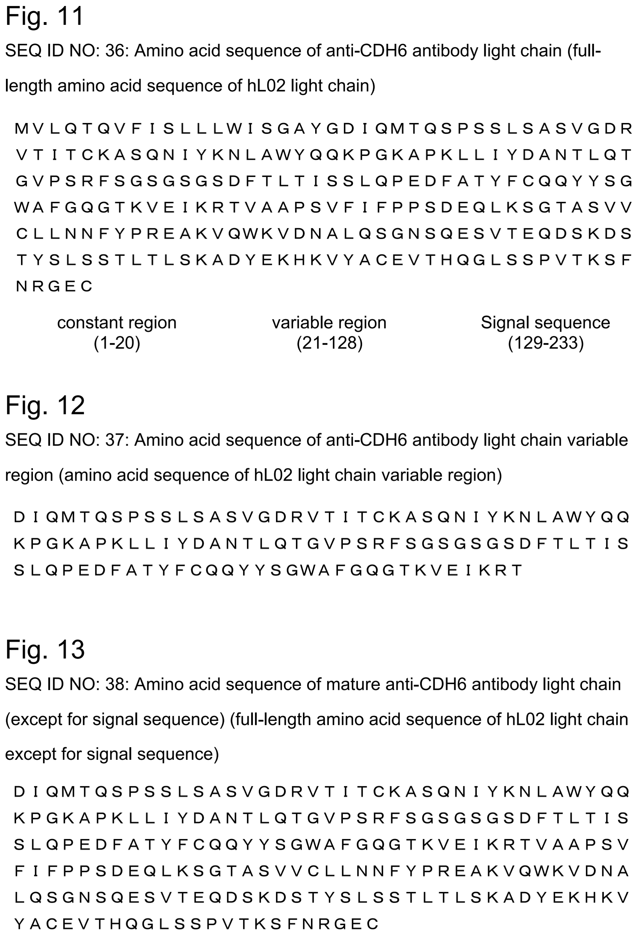

[Figure 11] Figure 11 is a diagram showing the amino acid sequence (SEQ ID NO: 36) of an anti-CDH6 antibody light chain.

[Figure 12] Figure 12 is a diagram showing the amino acid sequence (SEQ ID NO: 37) of an anti-CDH6 antibody light chain variable region.

[Figure 13] Figure 13 is a diagram showing the amino acid sequence (SEQ ID NO: 38) of a mature anti-CDH6 antibody light chain.

[Figure 14] Figure 14 is a diagram showing the tumor growth suppressing effects of respective single administration groups of an anti-CDH6 antibody-drug conjugate (1) and bevacizumab (Bevacizumab) and a combined administration group of the anti-CDH6 antibody-drug conjugate (1) and bevacizumab (Combination) in mice with subcutaneously transplanted OV-90 cells.

[Figure 15] Figure 15 is a diagram showing the percentage change in average tumor volume on Day 21, from an average tumor volume on Day 0 as a baseline, of respective single administration groups of an anti-CDH6 antibody-drug conjugate (1) and bevacizumab (Bevacizumab) and a combined administration group of the anti-CDH6 antibody-drug conjugate (1) and bevacizumab (Combination) in mice with subcutaneously transplanted OV-90 cells.

[Description of Embodiments]

Hereinafter, the preferred embodiments for carrying out the present invention will be described. It is to be noted that the embodiments described below merely illustrate the representative embodiments of the present invention, and the scope of the present invention shall not be narrowly interpreted due to these examples.

1. Definition

Cadherins are glycoproteins present on the surface of cell membranes and function as cell-cell adhesion molecules through the calcium ion-dependent binding of their N-terminal extracellular domains, or as signal molecules responsible for cell-cell interaction. Classic cadherins are in the cadherin superfamily and are single-pass transmembrane proteins composed of five extracellular domains (EC domains), one transmembrane region, and an intracellular domain.

CDH6 (cadherin-6) is a single-pass transmembrane protein composed of 790 amino acids, which is classified into the type II cadherin family, and this protein has N-terminal extracellular and C-terminal intracellular domains. The human CDH6 gene was cloned for the first time in 1995 (Shimoyama Y, et al., Cancer Research, 2206-2211, 55, May 15, 1995), and its sequence can be referred to under, for example, accession Nos. NM_004932 and NP_004923 (NCBI). The amino acid sequence shown in SEQ ID NO: 1 is the amino acid sequence of extracellular domain 3 (in the present description, also referred to as EC domain 3 or EC3) of human CDH6.

In the present description, the term "cancer" is used to have the same meaning as that of the term "tumor".

In the present description, the term "gene" is used to include not only DNA but also its mRNA and cDNA, and cRNA thereof.

In the present description, the term "CDH6" can be used to have the same meaning as that of the CDH6 protein.

In the present description, the term "anti-CDH6 antibody" refers to an antibody which specifically binds to CDH6 (cadherin-6). The anti-CDH6 antibody is preferably an antibody which has the activity of being internalized into CDH6-expressing cells by binding to CDH6.

In the present description, the term "functional fragment of an antibody", also called "antigen-binding fragment of an antibody", is used to mean a partial fragment of the antibody having binding activity against an antigen, and includes Fab, F(ab')2, Fv, scFv, a diabody, a linear antibody and a multispecific antibody formed from antibody fragments, and the like. Fab', which is a monovalent fragment of antibody variable regions obtained by treating F(ab')2 under reducing conditions, is also included in the antigen-binding fragment of an antibody. However, the antigen-binding fragment of an antibody is not limited to these molecules, as long as the antigen-binding fragment has antigen-binding ability. These antigen-binding fragments include not only those obtained by treating a full-length molecule of an antibody protein with an appropriate enzyme, but proteins produced in appropriate host cells using a genetically engineered antibody gene.

In the present description, the term "CDR" is used to mean a complementarity determining region. It is known that the heavy chain and light chain of an antibody molecule each have three CDRs. Such a CDR is also referred to as a hypervariable region, and is located in the variable regions of the heavy chain and light chain of an antibody. These regions

have a particularly highly variable primary structure and are separated into three sites on the primary structure of the polypeptide chain in each of the heavy chain and light chain. In the present description, with regard to the CDR of an antibody, the CDRs of a heavy chain are referred to as CDRH1, CDRH2 and CDRH3, respectively, from the aminoterminal end of the amino acid sequence of the heavy chain, whereas the CDRs of a light chain are referred to as CDRL1, CDRL2 and CDRL3, respectively, from the aminoterminal end of the amino acid sequence of the light chain. These sites are located close to one another on the three-dimensional structure, and determine the specificity of the antibody to an antigen to which the antibody binds.

In the present description, the term "approximately" refers to a value which may vary by up to plus or minus 10%, 8%, 6%, 5%, 4%, 3%, 2%, or 1% from the reference value. Preferably, the term "approximately" refers to the range of plus or minus 10%, 5%, or 1% from the reference value.

In the present description, the term "resistance" refers to a property of having non-response to treatment with an anticancer agent, and can also be expressed as "refractoriness", "non-responsiveness", or "unresponsiveness". The term can also be expressed as "intolerance" because tumor growth cannot be prevented due to the non-response. In the present invention, the term "resistance" includes cases where cancer in a patient has low sensitivity to treatment with an anticancer agent, cases where cancer cells neither disappear nor decrease in size, cases where neither complete response (CR) nor partial response (PR) is obtained, and/or cases where cancer progresses at an early stage (e.g., within, or less than, 6 months for ovarian cancer), after treatment with the anticancer agent.

In the present description, the term "chemotherapy" refers to treatment using one or more chemotherapeutics that are used for treating cancer.

In the present description, the term "chemotherapeutic" refers to an agent for chemotherapy that is used for treating cancer. Examples of the chemotherapeutic can include alkylating agents (e.g., mechlorethamine, cyclophosphamide, ifosfamide, melphalan, chlorambucil, hexamethylmelamine, thiotepa, busulfan, carmustine, lomustine, semustine, streptozocin, and dacarbazine), antimetabolites (e.g., gemcitabine, methotrexate, fluorouracil, doxifluridine, capecitabine, floxuridine, cytarabine, mercaptopurine, thioguanine, and pentostatin), vinca alkaloids (e.g., vinblastine and vincristine), epipodophyllotoxins (e.g., etoposide and teniposide), antibiotics (e.g., dactinomycin, daunorubicin, doxorubicin, bleomycin, plicamycin, and mitomycin), platinum compounds (e.g., cisplatin, carboplatin, and oxaliplatin), taxanes (e.g., paclitaxel and docetaxel), anthracenediones (e.g., mitoxantrone), substituted ureas (e.g., hydroxyurea), methylhydrazines (e.g., procarbazine hydrochloride), vitamin A metabolites (e.g., tretinoin), and pharmaceutically acceptable salts thereof. The chemotherapeutic is not particularly limited as long as the agent is used for chemotherapy.

In the present description, the term "platinum-based chemotherapy" refers to cancer treatment using one or more platinum-based drugs with or without one or more chemotherapeutics other than the platinum-based drugs.

In the present description, the term "platinum-based drug" refers to a platinum compound that is used for treating cancer. Examples of the platinum-based drug include, but are not limited to, cisplatin, carboplatin, and oxaliplatin.

In the present description, the term "cancer recurrence" refers to the return of cancer to the same location as that of a primary tumor or a different location from that of a primary tumor in the body after a period during which no cancer is detectable. The "cancer recurrence" is defined according to the following references.

NCI Dictionaries, "recurrence", NCI Dictionary of Cancer Terms [online]. National Cancer Institute [retrieved on 2023-12-27]. Retrieved from <cancer.gov/publications/dictionaries/cancer-terms/def/recurrence>.

In the present description, the "chemotherapy regimen" refers to the plan of treatment for chemotherapy that defines a drug, a dose, a frequency, and the like. For example, the term "chemotherapy regimen comprising a platinum-based drug" refers to a chemotherapy regimen comprising administering the platinum-based drug, and the term "chemotherapy regimen comprising a platinum-based drug and taxane" refers to a chemotherapy regimen comprising administering the platinum-based drug and taxane.

2. Anti-CDH6 antibody-drug conjugate

The anti-CDH6 antibody-drug conjugate used in the present invention is an antibody-drug conjugate in which a drug-linker represented by the formula:

[Formula 9]

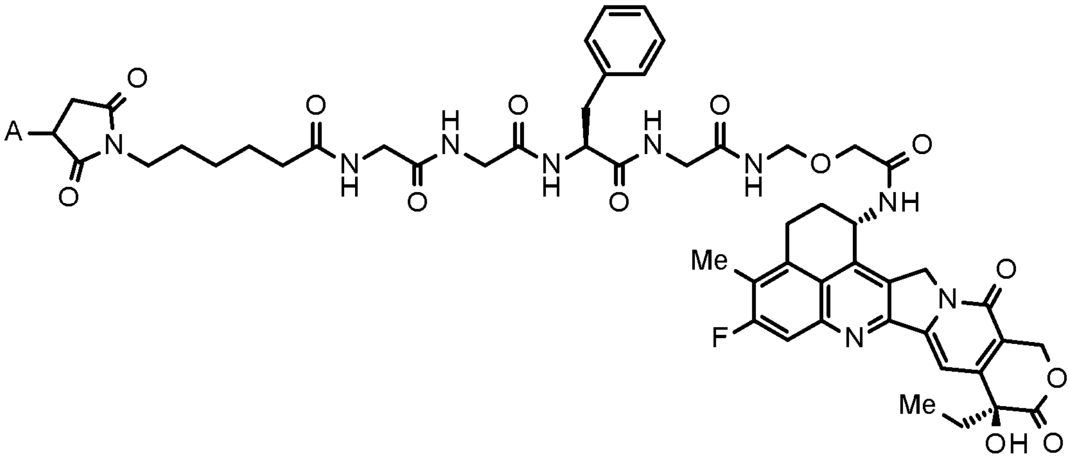

wherein A represents a connecting position to an anti-CDH6 antibody or a functional fragment of the antibody, is conjugated to the anti-CDH6 antibody or the functional fragment of the antibody via a thioether bond.

In the present invention, the partial structure consisting of a linker and a drug in the antibody-drug conjugate is referred to as a "drug-linker". This drug-linker is connected to a thiol group (in other words, the sulfur atom of a cysteine residue) formed at an interchain disulfide bond site (two sites between heavy chains and two sites between a heavy chain and a light chain) in the antibody.

The drug-linker of the present invention comprises a topoisomerase I inhibitor exatecan (IUPAC name: (1S,9S)-1-amino-9-ethyl-5-fluoro-1,2,3,9,12,15-hexahydro-9-hydroxy-4-methyl-10H, 13H-benzo[de]pyrano[3',4':6,7]indolizino[1,2-b]quinolin-10,13-dione, (also expressed as chemical name: (1S,9S)-1-amino-9-ethyl-5-fluoro-2,3-dihydro-9-hydroxy-4-methyl-1H, 12H-benzo[de]pyrano[3',4':6,7]indolizino[1,2-b]quinolin-10,13(9H,15H)-dione)) as a component. Exatecan is represented by the formula:

[Formula 10]

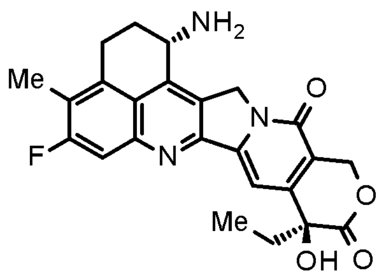

and is a camptothecin derivative having an antitumor effect.

The anti-CDH6 antibody-drug conjugate used in the present invention can also be represented by the following formula:

[Formula 11]

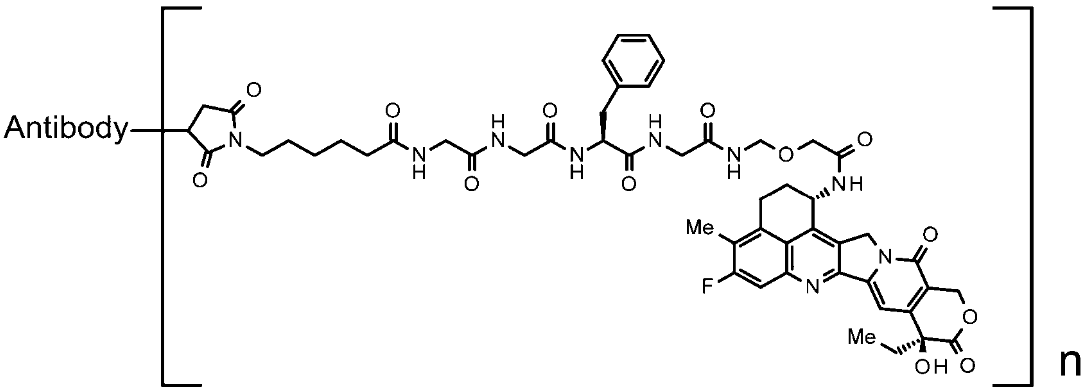

In this context, Antibody is an anti-CDH6 antibody or a functional fragment of the antibody (preferably, an anti-CDH6 antibody), and the drug-linker is conjugated to the antibody via a thioether bond (each of the druglinkers represented by the structure shown in the parentheses in the formula is conjugated to the antibody via a thioether bond). n has the same meaning as that of the so-called average number of conjugated drug molecules (DAR; drug-to-antibody ratio), and represents the average number of units of the drug-linker conjugated per antibody.

In some embodiments, the anti-CDH6 antibody-drug conjugate is raludotatug deruxtecan (also referred to as R-DXd or DS-6000a).

After migrating into cancer cells, the antibody-drug conjugate used in the present invention is cleaved at the linker moiety to release a compound represented by the formula:

[Formula 12]

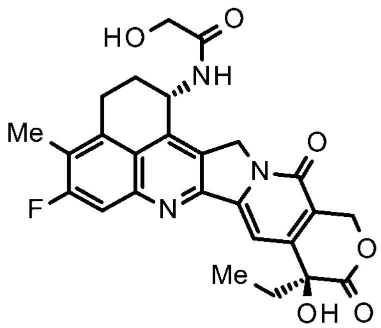

(hereinafter, referred to as a compound (A)).

The aforementioned compound is considered as the original source of the antitumor activity of the anti-CDH6 antibody-drug conjugate used in the present invention, and has been confirmed to have a topoisomerase I inhibitory effect (Ogitani Y. et al., Clinical Cancer Research, 2016, Oct 15; 22 (20): 5097-5108, Epub 2016 Mar 29).

Topoisomerase I is an enzyme that cleaves and rejoins a single strand of DNA thereby transforming the conformation of the DNA to participate in DNA synthesis. Therefore, agents having a topoisomerase I inhibitory effect can inhibit DNA synthesis, thus arrest cell division at S phase (DNA synthesis phase) of the cell cycle and induce cell death by apoptosis, thereby suppressing growth of cancer cells.

The antibody-drug conjugate used in the present invention is also known to have a bystander antitumor effect (Suzuki H, et al., Molecular Cancer Therapeutics, (2024) 23 (3): 257-271).

This bystander antitumor effect is exerted through a process in which the antibody-drug conjugate used in the present invention is internalized into target-expressing cancer cells where the compound is then released so as also to exert an antitumor effect on non-target-expressing cancer cells present therearound.

This bystander antitumor effect is also exerted as an excellent antitumor effect when the antibody-drug conjugate according to the present invention is used in combination with a VEGF inhibitor.

Other examples of the anti-CDH6 antibody-drug conjugate are not particularly limited as long as an antitumor compound is conjugated to an anti-CDH6 antibody via a linker structure moiety. Examples thereof can include those described in CUSP06, BSI-709, and International Publication Nos. WO 2023/102875 and WO 2023/104188.

3. Antibody in anti-CDH6 antibody-drug conjugate

The anti-CDH6 antibody in the anti-CDH6 antibody-drug conjugate used in the present invention may be derived from any species, and is preferably an anti-CDH6 antibody derived from a human, a rat, a mouse or a rabbit. When the anti-CDH6 antibody is derived from a species other than humans, it is preferred to chimerize or humanize the anti-CDH6 antibody by a well-known technique. The anti-CDH6 antibody of the present invention may be a polyclonal antibody or may be a monoclonal antibody, and a monoclonal antibody is preferred.

The anti-CDH6 antibody in the anti-CDH6 antibody-drug conjugate used in the present invention preferably has the property of being able to target cancer cells, and is preferably an antibody that possesses, for example, the property of being able to recognize cancer cells, the property of being able to bind to cancer cells, the property of being internalized into cancer cells by cellular uptake, and/or cytocidal activity against cancer cells.

The binding activity of an antibody against cancer cells can be confirmed by flow cytometry. The uptake of an antibody into cancer cells can be confirmed by (1) an assay of visualizing a cellularly taken-up antibody under a fluorescent microscope using a secondary antibody (fluorescently labeled) binding to the antibody (Cell Death and Differentiation (2008) 15, 751-761), (2) an assay of measuring the amount of cellularly taken-up fluorescence using a secondary antibody (fluorescently labeled) binding to the antibody (Molecular Biology of the Cell Vol. 15, 5268-5282, December 2004) or (3) a Mab-ZAP assay using an immunotoxin binding to the antibody, wherein the toxin is released upon cellular uptake, so as to suppress cell growth (Bio Techniques 28: 162-165, January 2000). A recombinant conjugated protein of a catalytic region of diphtheria toxin and protein G may be used as the immunotoxin.

The antitumor activity of the antibody can be confirmed *in vitro* by measuring inhibitory activity against cell growth. For example, a cancer cell line overexpressing the target protein of the antibody is cultured, and the antibody is added at varying concentrations into the culture system to measure inhibitory activity against focus formation, colony formation and spheroid growth. The antitumor activity can be confirmed *in vivo,* for example, by administering the antibody to a nude mouse into which a tumor cell line highly expressing the target protein has been inoculated, and then measuring a change in the cancer cells.

Since the compound conjugated in the antibody-drug conjugate exerts an antitumor effect, it is preferred but not essential that the antibody itself should have an antitumor effect. For the purpose of specifically and/or selectively exerting the cytotoxicity of the antitumor compound against cancer cells, it is important and preferred that the antibody should have a property of being internalized and transferred into cancer cells.

The anti-CDH6 antibody in the anti-CDH6 antibody-drug conjugate used in the present invention can be obtained by procedures known in the art. For example, the anti-CDH6 antibody can be obtained using a method usually carried out in the art, which involves immunizing an animal with an antigenic polypeptide and then collecting and purifying an antibody produced in vivo. The origin of the antigen is not limited to a human, and thus an animal can also be immunized with an antigen derived from a non-human animal such as a mouse, a rat or the like. In this case, the cross-reactivity of antibodies binding to the obtained heterologous antigen with human antigens can be tested to screen for an antibody applicable to a human disease.

Alternatively, antibody-producing cells that produce an antibody against the antigen can be fused with myeloma cells according to a method known in the art (e.g., Kohler and Milstein, Nature (1975) 256, 495-497; and Kennet, R. ed., Monoclonal Antibodies, 365-367, Plenum Press, N. Y. (1980)) to establish hybridomas, so as to obtain a monoclonal antibody.

The antigen can be obtained by allowing host cells to produce a gene encoding the antigen protein according to genetic manipulation. Specifically, a vector capable of expressing the antigen gene is produced, and the vector is then introduced into host cells, so that the gene is expressed therein, and thereafter, the expressed antigen may be purified. The antibody can also be obtained by a method of immunizing an animal with the antigen-expressing cells based on the above-described genetic manipulation, or a cell line expressing the antigen.

The anti-CDH6 antibody in the anti-CDH6 antibody-drug conjugate used in the present invention is preferably a genetically recombinant antibody that has been artificially modified for the purpose of reducing heterogenetic antigenicity to humans, such as a chimeric antibody or a humanized antibody, or is preferably an antibody having only the gene sequence of a human-derived antibody, i.e., a human antibody. These antibodies can be produced by known methods.

Examples of the chimeric antibody can include antibodies in which a variable region and a constant region are heterologous to each other, such as a chimeric antibody formed by conjugating the variable region of a mouse- or rat-derived antibody to a human-derived constant region (Proc. Natl. Acad. Sci. U.S.A., 81, 6851-6855, (1984)).

Examples of the humanized antibody can include an antibody formed by incorporating only complementarity determining regions (CDRs) of a heterologous antibody into a human-derived antibody (Nature (1986) 321, p. 522-525), an antibody formed by incorporating the amino acid residues from some frameworks of a heterologous antibody, as well as CDR sequences of the heterologous antibody, into a human antibody according to a CDR grafting method (International Publication No. WO90/07861), and an antibody humanized by use of a gene conversion mutagenesis strategy (U.S. Patent No. 5821337).

Examples of the human antibody can include an antibody prepared using a human antibody-producing mouse having a human chromosomal fragment comprising the heavy chain and light chain genes of a human antibody (see Tomizuka, K. et al., Nature Genetics (1997) 16, p. 133-143; Kuroiwa, Y. et al., Nucl. Acids Res. (1998) 26, p. 3447-3448; Yoshida, H. et al., Animal Cell Technology: Basic and Applied Aspects vol. 10, p. 69-73 (Kitagawa, Y., Matsuda, T. and Iijima, S. eds.), Kluwer Academic Publishers, 1999; Tomizuka, K. et al., Proc. Natl. Acad. Sci. USA(2000) 97, p. 722-727; etc.). Another example thereof can include a phage display-derived antibody that has been selected from a human antibody library (see Wormstone, I. M. et al., Investigative Ophthalmology & Visual Science. (2002) 43 (7), p. 2301-2308; Carmen, S. et al., Briefings in Functional Genomics and Proteomics (2002), 1 (2), p. 189-203; Siriwardena, D. et al., Ophthalmology (2002) 109 (3), p. 427-431; etc.).

The anti-CDH6 antibody in the anti-CDH6 antibody-drug conjugate used in the present invention can preferably be an antibody which specifically binds to the amino acid sequence shown in SEQ ID NO: 1 and has an internalization ability that permits cellular uptake. Examples of such an anti-CDH6 antibody can include antibodies described in International Publication No. WO 2018/212136 (H01L02, H02L02, H02L03, H04L02, etc.). The phrase "specifically binds to the amino acid sequence shown in SEQ ID NO: 1" as applied to the antibody is used to mean that the antibody strongly binds to the EC3 domain of CDH6 compared with the other extracellular domains of CDH6.

The internalization activity (internalization ability) of the antibody can be evaluated using, for example, a reagent conjugated with a toxin (saporin) inhibiting protein synthesis (e.g., an anti-rat IgG reagent Rat-ZAP (Advanced Targeting Systems) and an anti-human IgG reagent Hum-ZAP (Advanced Targeting Systems)) (see International Publication No. WO 2018/212136).

The anti-CDH6 antibody in the anti-CDH6 antibody-drug conjugate used in the present invention is preferably an antibody whose survival rate (which is indicated by a ratio relative to a cell survival rate without antibody addition defined as 100%) of CDH6-expressing cells to which the aforementioned antibody and a saporin-labeled anti-rat IgG antibody or a saporin-labeled anti-human IgG antibody have been administered is 80% or less, more preferably 70% or less, and still more preferably 60% or less.

The anti-CDH6 antibody in the anti-CDH6 antibody-drug conjugate used in the present invention is preferably an antibody comprising

a heavy chain comprising CDRH1, CDRH2 and CDRH3 in any one combination selected from the group consisting of the following combinations (1) to (5): (1) CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID NO: 4, (2) CDRH1 consisting of the amino acid sequence shown in SEQ ID NO: 2, CDRH2 consisting of the amino acid sequence shown in SEQ ID NO: 8, and CDRH3 consisting of the amino acid sequence shown in SEQ ID NO: 4, (3) CDRH1 consisting of the amino acid sequence shown in SEQ ID NO: 9, CDRH2 consisting of the amino acid sequence shown in SEQ ID NO: 10, and CDRH3 consisting of the amino acid sequence shown in SEQ ID NO: 11, (4) CDRH1 consisting of the amino acid sequence shown in SEQ ID NO: 15, CDRH2 consisting of the amino acid sequence shown in SEQ ID NO: 16, and CDRH3 consisting of the amino acid sequence shown in SEQ ID NO: 17, and (5) CDRH1 consisting of the amino acid sequence shown in SEQ ID NO: 21, CDRH2 consisting of the amino acid sequence shown in SEQ ID NO: 22, and CDRH3 consisting of the amino acid sequence shown in SEQ ID NO: 23, and

a light chain comprising CDRL1, CDRL2 and CDRL3 in any one combination selected from the group consisting of the following combinations (6) to (9): (6) CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, (7) CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14, (8) CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and (9) CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26.

The anti-CDH6 antibody in the anti-CDH6 antibody-drug conjugate used in the present invention is more preferably an antibody comprising a heavy chain comprising CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID NO: 4, and a light chain comprising CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7.

The anti-CDH6 antibody in the anti-CDH6 antibody-drug conjugate used in the present invention is more preferably an antibody comprising a heavy chain comprising CDRH1 consisting of the amino acid sequence shown in SEQ

ID NO: 2, CDRH2 consisting of the amino acid sequence shown in SEQ ID NO: 8, and CDRH3 consisting of the amino acid sequence shown in SEQ ID NO: 4, and a light chain comprising CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7.

The anti-CDH6 antibody in the anti-CDH6 antibody-drug conjugate used in the present invention is more preferably an antibody comprising a heavy chain comprising CDRH1 consisting of the amino acid sequence shown in SEQ ID NO: 9, CDRH2 consisting of the amino acid sequence shown in SEQ ID NO: 10, and CDRH3 consisting of the amino acid sequence shown in SEQ ID NO: 11, and a light chain comprising CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14.

The anti-CDH6 antibody in the anti-CDH6 antibody-drug conjugate used in the present invention is more preferably an antibody comprising a heavy chain comprising CDRH1 consisting of the amino acid sequence shown in SEQ ID NO: 15, CDRH2 consisting of the amino acid sequence shown in SEQ ID NO: 16, and CDRH3 consisting of the amino acid sequence shown in SEQ ID NO: 17, and a light chain comprising CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20.

The anti-CDH6 antibody in the anti-CDH6 antibody-drug conjugate used in the present invention is more preferably an antibody comprising a heavy chain comprising CDRH1 consisting of the amino acid sequence shown in SEQ ID NO: 21, CDRH2 consisting of the amino acid sequence shown in SEQ ID NO: 22, and CDRH3 consisting of the amino acid sequence shown in SEQ ID NO: 23, and a light chain comprising CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26.

The anti-CDH6 antibody in the anti-CDH6 antibody-drug conjugate used in the present invention can be still more preferably an antibody comprising a given combination of a heavy chain comprising any one heavy chain variable region selected from the group consisting of the following variable regions (1) to (3): (1) the amino acid sequence shown in SEQ ID NO: 28, 31 or 34, (2) an amino acid sequence having an identity of at least 95% or more to the amino acid sequence (1) (preferably an amino acid sequence having a sequence identity of at least 95% or more to the sequence of the framework regions other than at each CDR sequence), and (3) an amino acid sequence comprising a deletion, substitution or addition of one or several amino acids in the amino acid sequence (1), and a light chain comprising any one light chain variable region selected from the group consisting of the following variable regions (4) to (6): (4) the amino acid sequence shown in SEQ ID NO: 37 or 40, (5) an amino acid sequence having an identity of at least 95% or more to the amino acid sequence (4) (preferably an amino acid sequence having a sequence identity of at least 95% or more to the sequence of the framework regions other than at each CDR sequence), and (6) an amino acid sequence comprising a deletion, substitution or addition of one or several amino acids in the amino acid sequence (4).

The anti-CDH6 antibody in the anti-CDH6 antibody-drug conjugate used in the present invention is still more preferably an antibody comprising a heavy chain comprising a heavy chain variable region amino acid sequence shown in SEQ ID NO: 28, a heavy chain comprising a heavy chain variable region amino acid sequence shown in SEQ ID NO: 31, or a heavy chain comprising a heavy chain variable region amino acid sequence shown in SEQ ID NO: 34, and a light chain comprising a light chain variable region amino acid sequence shown in SEQ ID NO: 37 or a light chain having a light chain variable region amino acid sequence shown in SEQ ID NO: 40.

The anti-CDH6 antibody in the anti-CDH6 antibody-drug conjugate used in the present invention is still more preferably an antibody comprising a heavy chain comprising a heavy chain variable region consisting of the amino acid sequence shown in SEQ ID NO: 28 and a light chain comprising a light chain variable region consisting of the amino acid sequence shown in SEQ ID NO: 37.

The anti-CDH6 antibody in the anti-CDH6 antibody-drug conjugate used in the present invention is still more preferably an antibody comprising a heavy chain comprising a heavy chain variable region consisting of the amino acid sequence shown in SEQ ID NO: 31 and a light chain comprising a light chain variable region consisting of the amino acid sequence shown in SEQ ID NO: 37.

The anti-CDH6 antibody in the anti-CDH6 antibody-drug conjugate used in the present invention is still more preferably an antibody comprising a heavy chain comprising a heavy chain variable region consisting of the amino acid sequence shown in SEQ ID NO: 34 and a light chain comprising a light chain variable region consisting of the amino acid sequence shown in SEQ ID NO: 37.

The anti-CDH6 antibody in the anti-CDH6 antibody-drug conjugate used in the present invention is still more preferably an antibody comprising a heavy chain comprising a heavy chain variable region consisting of the amino acid sequence shown in SEQ ID NO: 31 and a light chain comprising a light chain variable region consisting of the amino acid sequence shown in SEQ ID NO: 40.

The anti-CDH6 antibody in the anti-CDH6 antibody-drug conjugate used in the present invention is still more preferably an antibody comprising a heavy chain consisting of the amino acid sequence shown in SEQ ID NO: 29 and a

light chain consisting of the amino acid sequence shown in SEQ ID NO: 38, or an antibody in which a lysine residue is deleted from the carboxyl terminus of a heavy chain of the antibody.

The anti-CDH6 antibody in the anti-CDH6 antibody-drug conjugate used in the present invention is still more preferably an antibody comprising a heavy chain consisting of the amino acid sequence shown in SEQ ID NO: 32 and a light chain consisting of the amino acid sequence shown in SEQ ID NO: 38, or an antibody in which a lysine residue is deleted from the carboxyl terminus of a heavy chain of the antibody.

The anti-CDH6 antibody in the anti-CDH6 antibody-drug conjugate used in the present invention is still more preferably an antibody comprising a heavy chain consisting of the amino acid sequence shown in SEQ ID NO: 35 and a light chain consisting of the amino acid sequence shown in SEQ ID NO: 38, or an antibody in which a lysine residue is deleted from the carboxyl terminus of a heavy chain of the antibody.

The anti-CDH6 antibody in the anti-CDH6 antibody-drug conjugate used in the present invention is still more preferably an antibody comprising a heavy chain consisting of the amino acid sequence shown in SEQ ID NO: 32 and a light chain consisting of the amino acid sequence shown in SEQ ID NO: 41, or an antibody in which a lysine residue is deleted from the carboxyl terminus of a heavy chain of the antibody.

By combining together sequences showing a high identity to the above-described heavy chain amino acid sequences and light chain amino acid sequences, it is possible to select an antibody having a biological activity equivalent to that of each of the above-described antibodies. Such an identity is an identity of generally 80% or more, preferably an identity of 85% or more, more preferably an identity of 90% or more, still more preferably an identity of 95% or more, and most preferably an identity of 99% or more.

The identity between two types of amino acid sequences can be determined by aligning the sequences using the default parameters of Clustal W version 2 (Larkin MA, Blackshields G, Brown NP, Chenna R, McGettigan PA, McWilliam H, Valentin F, Wallace IM, Wilm A, Lopez R, Thompson JD, Gibson TJ and Higgins DG (2007), "Clustal W and Clustal X version 2.0", Bioinformatics. 23 (21): 2947-2948).

The antibody in the anti-CDH6 antibody-drug conjugate used in the present invention also includes a modification of an antibody. The "modification" is used to mean the antibody according to the present invention, which is chemically or biologically modified. Examples of such a chemical modification include chemical modifications such as the binding of a chemical moiety to an amino acid skeleton or the binding of a chemical moiety to an N-linked or O-linked carbohydrate chain. Examples of such a biological modification include antibodies which have undergone a posttranslational modification (e.g., N-linked or O-linked glycosylation, N-terminal or C-terminal processing, deamidation, isomerization of aspartic acid, and oxidation of methionine), and antibodies, to the N-terminus of which a methionine residue is added as a result of having been allowed to be expressed using prokaryote host cells. In addition, such a modification is also meant to include labeled antibodies for enabling detection or isolation of the antibody according to the present invention or an antigen, for example, an enzymatically labeled antibody, a fluorescently labeled antibody, and an affinity-labeled antibody. Such a modification of the antibody according to the present invention is useful for the improvement of the stability and retention in blood of an antibody; a reduction in antigenicity; detection or isolation of an antibody or an antigen; etc.

Moreover, by regulating a sugar chain modification (glycosylation, de-fucosylation, etc.) that binds to the antibody according to the present invention, antibody-dependent cellular cytotoxic activity can be enhanced.

As techniques of regulating the sugar chain modification of an antibody, those described in International Publication Nos. WO 99/54342, WO 00/61739, WO 02/31140, WO 2007/133855, and WO 2013/120066, etc. are known, though the techniques are not limited thereto. The antibody according to the present invention also includes antibodies in respect of which the aforementioned sugar chain modification has been regulated.

It is known that the lysine residue at the carboxyl terminus of the heavy chain of an antibody produced in cultured mammalian cells is deleted (Journal of Chromatography A, 705: 129-134 (1995)), and also, it is known that the two amino acid residues at the heavy chain carboxyl terminus, glycine and lysine, are deleted, and that the proline residue newly positioned at the carboxyl terminus is amidated (Analytical Biochemistry, 360: 75-83 (2007)). However, such deletion and modification of these heavy chain sequences does not have an influence on the antigen-binding activity and effector function (activation of complement, antibody-dependent cellular cytotoxicity, etc.) of an antibody. Accordingly, the antibody according to the present invention also includes an antibody that has undergone the aforementioned modification, and a functional fragment of the antibody, and specific examples of such an antibody include a deletion mutant comprising the deletion of 1 or 2 amino acids at the heavy chain carboxyl terminus, and a deletion mutant formed by amidating the aforementioned deletion mutant (e.g., a heavy chain in which the proline residue at the carboxyl-terminal site is amidated). However, deletion mutants involving a deletion at the carboxyl terminus of the heavy chain of the antibody according to the present invention are not limited to the above-described deletion mutants, as long as they retain antigen-binding activity and effector function. Two heavy chains constituting the antibody according to the present invention may be any one type of heavy chain selected from the group consisting of a full-length antibody and the above-described deletion mutants, or may be a combination of any two types selected from the aforementioned group. The ratio of individual deletion mutants can be influenced by the types of cultured mammalian cells that produce the antibody according to the present invention, and the culture conditions. Examples of the antibody according to the present invention can preferably include antibodies where

one amino acid residue is deleted at the carboxyl terminus of each of the two heavy chains. The amino acid deleted at the carboxyl terminus of the heavy chain in the antibody according to the present invention can preferably be a lysine residue.

Examples of the isotype of the antibody according to the present invention can include IgG (IgG1, IgG2, IgG3, and IgG4). Among others, IgG1, IgG2, and IgG4 are preferable.

In the hH01 heavy chain full-length amino acid sequence shown in SEQ ID NO: 27, the amino acid sequence consisting of the amino acid residues at positions 1 to 19 is the signal sequence, the amino acid sequence consisting of the amino acid residues at positions 20 to 141 is the variable region, and the amino acid sequence consisting of the amino acid residues at positions 142 to 471 is the constant region.

In the hH02 heavy chain full-length amino acid sequence shown in SEQ ID NO: 30, the amino acid sequence consisting of the amino acid residues at positions 1 to 19 is the signal sequence, the amino acid sequence consisting of the amino acid residues at positions 20 to 141 is the variable region, and the amino acid sequence consisting of the amino acid residues at positions 142 to 471 is the constant region.

In the hH04 heavy chain full-length amino acid sequence shown in SEQ ID NO: 33, the amino acid sequence consisting of the amino acid residues at positions 1 to 19 is the signal sequence, the amino acid sequence consisting of the amino acid residues at positions 20 to 141 is the variable region, and the amino acid sequence consisting of the amino acid residues at positions 142 to 471 is the constant region.

In the hL02 light chain full-length amino acid sequence shown in SEQ ID NO: 36, the amino acid sequence consisting of the amino acid residues at positions 1 to 20 is the signal sequence, the amino acid sequence consisting of the amino acid residues at positions 21 to 128 is the variable region, and the amino acid sequence consisting of the amino acid residues at positions 129 to 233 is the constant region.

In the hL03 light chain full-length amino acid sequence shown in SEQ ID NO: 39, the amino acid sequence consisting of the amino acid residues at positions 1 to 20 is the signal sequence, the amino acid sequence consisting of the amino acid residues at positions 21 to 128 is the variable region, and the amino acid sequence consisting of the amino acid residues at positions 129 to 233 is the constant region.

In some embodiments, the antibody in the anti-CDH6 antibody-drug conjugate used in the present invention is raludotatug.

4. Production of anti-CDH6 antibody-drug conjugate

The drug-linker intermediate for use in the production of the anti-CDH6 antibody-drug conjugate according to the present invention is represented by the following formula:

[Formula 13]

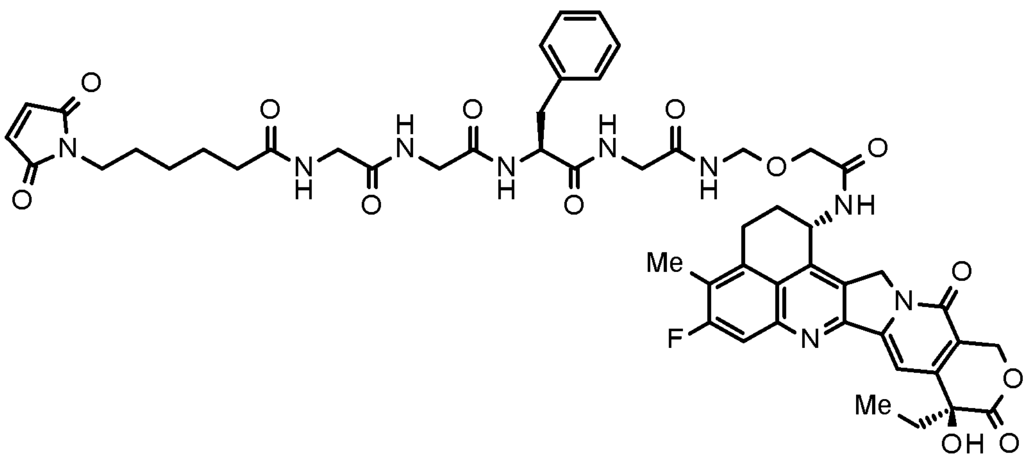

The above-described drug-linker intermediate can be represented by the chemical name N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4' :6,7]indolizino[1,2-b]quinolin-1-yl]amino}-2-oxoethoxy)methyl]glycinamide, and can be produced with reference to the description of International Publication Nos. WO 2014/057687, WO 2015/098099, WO 2015/115091, WO 2015/155998, and WO 2019/044947, etc.

The anti-CDH6 antibody-drug conjugate used in the present invention can be produced by reacting the above-described drug-linker intermediate with an anti-CDH6 antibody having a thiol group (or also referred to as a sulfhydryl group).

The anti-CDH6 antibody having a sulfhydryl group can be obtained by a method well known to a person skilled in the art (Hermanson, G.T, Bioconjugate Techniques, pp. 56-136, pp. 456-493, Academic Press (1996)). For example, by using 0.3 to 3 molar equivalents of a reducing agent such as tris(2-carboxyethyl)phosphine hydrochloride (TCEP) per interchain disulfide within the antibody and reacting with the anti-CDH6 antibody in a buffer solution containing a chelating agent such as ethylenediamine tetraacetic acid (EDTA), an anti-CDH6 antibody having a sulfhydryl group with partially or completely reduced interchain disulfides within the antibody can be obtained.

Further, using 2 to 20 molar equivalents of the drug-linker intermediate per anti-CDH6 antibody having a sulfhydryl group, the anti-CDH6 antibody-drug conjugate in which 2 to 8 drug molecules are conjugated per anti-CDH6 antibody can be produced.

The average number of conjugated drug molecules per antibody molecule in the produced anti-CDH6 antibody-drug conjugate can be calculated by, for example, a calculation method of measuring UV absorbance of the antibody-drug conjugate and a conjugation precursor thereof at two wavelengths of 280 nm and 370 nm (UV method) or a calculation method of treating the antibody-drug conjugate with a reducing agent, and quantifying each resulting fragment by HPLC measurement (HPLC method).

The conjugation between the antibody with the drug-linker intermediate and the calculation of the average number of conjugated drug molecules per antibody molecule in the antibody-drug conjugate can be carried out with reference to the description of International Publication Nos. WO 2014/057687, WO 2015/098099, WO 2015/115091, WO 2015/155998, WO 2018/135501, and WO 2018/212136, etc.

In some embodiments, the average number of units of the drug-linker conjugated per antibody molecule in the anti-CDH6 antibody-drug conjugate according to the present invention is preferably 1 to 10, more preferably 2 to 8, still more preferably 5 to 8, still more preferably 7 to 8, still more preferably 7.5 to 8, and still more preferably approximately 8.

In other embodiments, the number of units of the drug or the drug-linker conjugated per antibody molecule in the anti-CDH6 antibody-drug conjugate according to the present invention is an integer in the range from preferably 2 to 8, more preferably selected from 2, 4, 6, or 8, and still more preferably 8.

The anti-CDH6 antibody-drug conjugate can be produced with reference to the description of International Publication No. WO 2018/212136, etc.

5. VEGF inhibitor

In the present invention, the term "VEGF inhibitor" refers to an agent that inhibits an interaction between VEGF and a VEGF receptor.

The VEGF inhibitor according to the present invention is not particularly limited as long as the agent has the above-described properties. The VEGF inhibitor may be an antibody, a functional fragment of the antibody, a fusion protein, an immunoadhesin, a nucleic acid, an oligonucleotide, an aptamer, a polypeptide, or a low-molecular-weight compound. The VEGF inhibitor according to the present invention is preferably an anti-VEGF antibody, an anti-VEGF receptor antibody, a fusion protein comprising a VEGF receptor extracellular domain, or a multispecific antibody comprising an anti-VEGF binding domain.

In the present invention, the term "anti-VEGF antibody" refers to an antibody that specifically binds to VEGF, or a functional fragment of the antibody.

In the present invention, the term "anti-VEGF receptor antibody" refers to an antibody that specifically binds to a VEGF receptor, or a functional fragment of the antibody.

In the present invention, the term "fusion protein comprising a VEGF receptor extracellular domain" refers to a fusion protein having a VEGF binding domain (also including a mutant of the VEGF binding domain) derived from a VEGF receptor extracellular domain.

In the present invention, the term "multispecific antibody comprising an anti-VEGF binding domain" refers to an antibody having binding specificities for at least two different sites, or a functional fragment of the antibody, wherein at least one of the binding specificities is for VEGF.

In the present invention, the term "bispecific antibody comprising an anti-VEGF binding domain" refers to an antibody having binding specificities for two different sites, or a functional fragment of the antibody, wherein one of the binding specificities is for VEGF.

The VEGF inhibitor according to the present invention is more preferably an anti-VEGF antibody. The anti-VEGF antibody according to the present invention is preferably bevacizumab or sevacizumab, and more preferably bevacizumab.

The VEGF inhibitor according to the present invention is more preferably an anti-VEGF receptor antibody. The anti-VEGF receptor antibody according to the present invention is preferably ramucirumab.

The VEGF inhibitor according to the present invention is more preferably a fusion protein comprising a VEGF receptor extracellular domain. The fusion protein comprising a VEGF receptor extracellular domain according to the present invention is preferably aflibercept.

The VEGF inhibitor according to the present invention is more preferably a multispecific antibody comprising an anti-VEGF binding domain, and still more preferably a bispecific antibody comprising an anti-VEGF binding domain. The bispecific antibody comprising an anti-VEGF binding domain according to the present invention is preferably ivonescimab (Non Patent Literature 3), CTX-009 (also referred to as ABL-001; Non Patent Literature 4), BI836880 (Clin. Exp. Metastasis (2020) 37 (6): 637-648), navicixizumab (Non Patent Literature 3), vanucizumab (Non Patent Literature 4), or PM8002 (Journal of Clinical Oncology 41, no. 16_suppl (June 01, 2023) 2536-2536).

The pharmaceutical composition of the present invention can be administered as a pharmaceutical composition containing a pharmaceutically acceptable carrier, diluent, solubilizer, emulsifier, preservative, aid, and the like. The "pharmaceutically acceptable carrier" and the like can be appropriately selected from a wide range depending on the type of target disease or the dosage form of the agent. The "pharmaceutically acceptable carrier" and the like include, for example, sterilized liquid. Herein, the liquid includes, for example, water and oil (petroleum oil and oil of animal origin, plant origin, or synthetic origin). The oil may be, for example, peanut oil, soybean oil, mineral oil, or sesame oil. Water is a more typical liquid when the pharmaceutical composition above is administered intravenously. Saline solution, an aqueous dextrose solution, and an aqueous glycerol solution can also be used as the liquid, in particular, for an injection solution. A suitable pharmaceutical vehicle can be selected from ones known in the art. Examples of suitable pharmaceutically acceptable carriers are disclosed in "Remington’s Pharmaceutical Sciences" by E. W. Martin. The formulations correspond to the mode of administration.

A method for administering the antitumor agent of the present invention can be appropriately selected. For example, administration by injection can be performed, and local injection, intraperitoneal injection, selective intravenous injection, intravenous injection, subcutaneous injection, organ perfusate injection, or the like can be adopted. The administration can be made by injection or bolus injection, for example. A solution for injection can be formulated using a carrier formed from a salt solution, a glucose solution, or a mixture of salt water and a glucose solution, various buffer solutions, and the like. Alternatively, the solution for injection may be prepared by mixing a formulation in a powder state with the liquid carrier upon use. According to a specific, preferred, embodiment, the administration of the antibody-drug conjugate and the VEGF inhibitor used in the present invention is performed by injection. Parenteral administration is a preferred administration route.

Other administration methods can also be appropriately selected along with the development of a formulation. In the case of oral administration, for example, an oral solution, a powder, a pill, a capsule, and a tablet are applicable. The oral solution can be produced as an oral liquid preparation such as a suspension and a syrup using, for example: water; sugars such as sucrose, sorbitol, and fructose; glycols such as polyethylene glycol; oils such as sesame oil and soybean oil; antiseptics such as alkyl p-hydroxybenzoate; and flavors such as a strawberry flavor and peppermint. The powder, the pill, the capsule, and the tablet can be formulated using, for example: an excipient such as lactose, glucose, sucrose, or mannitol; a disintegrant such as starch or sodium alginate; a lubricant such as magnesium stearate or talc; a binder such as polyvinyl alcohol, hydroxypropylcellulose, or gelatin; a surfactant such as a fatty acid ester; and/or a plasticizer such as glycerin. The tablet and the capsule are preferred unit dosage forms because of the ease of administration. A solid pharmaceutical carrier is used in producing the tablet or the capsule.

6. Medicament

Hereinafter, the pharmaceutical composition and the method of treatment according to the present invention will be described, wherein an anti-CDH6 antibody-drug conjugate and a VEGF inhibitor are administered in combination.

The present invention includes a pharmaceutical composition comprising an anti-CDH6 antibody-drug conjugate to be used in combination with a VEGF inhibitor or administered in combination with a VEGF inhibitor.

In the pharmaceutical composition and the method of treatment of the present invention, the anti-CDH6 antibody-drug conjugate and the VEGF inhibitor may be separately contained as active components in different formulations, and be administered at the same time (a person skilled in the art would naturally understand that "at the same time" may be or may not be at approximately the same time) or at different times, or the anti-CDH6 antibody-drug conjugate and the VEGF inhibitor may be contained as active components in a single formulation for administration.

In the pharmaceutical composition and the method of treatment of the present invention, two or more types of VEGF inhibitors used in the present invention may be combined for administration.

The pharmaceutical composition and the method of treatment of the present invention can be used for treating cancer, can preferably be used for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms’ tumor, neuroblastoma, colorectal cancer, stomach cancer, endometrial cancer, uterine body cancer, and nasopharyngeal cancer, can more preferably be used for treating at least one selected from the group consisting of renal cell carcinoma, renal clear cell carcinoma, papillary renal cell

carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma, and can still more preferably be used for treating at least one cancer selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, and ovarian mucinous tumor.

The pharmaceutical composition and the method of treatment of the present invention can still more preferably be used for treating at least one cancer selected from the group consisting of renal cell carcinoma, renal clear cell carcinoma, and papillary renal cell carcinoma.

The pharmaceutical composition and the method of treatment of the present invention can still more preferably be used for treating at least one cancer selected from the group consisting of ovarian cancer, ovarian serous adenocarcinoma, ovarian clear cell carcinoma, endometrioid ovarian cancer, and ovarian mucinous tumor, can still more preferably be used for treating ovarian cancer, and can still more preferably be used for treating epithelial ovarian cancer, fallopian tube cancer, or primary peritoneal cancer.

The cancer targeted by the pharmaceutical composition and the method of treatment of the present invention can preferably be metastatic cancer.

The pharmaceutical composition and the method of treatment of the present invention can be used for treating cancer with HRD (homologous recombination deficiency), and can preferably be used for treating ovarian cancer with HRD. A cancer with HRD refers to a cancer that manifests damage of the ability to repair DNA double-strand breaks via homologous recombination. An ovarian cancer with HRD refers to an ovarian cancer that manifests damage of the ability to repair DNA double-strand breaks via homologous recombination. The pharmaceutical composition and the method of treatment of the present invention can be used for treating cancer with HRP (also referred to as homologous recombination proficiency or HRD-negative), and can preferably be used for treating ovarian cancer with HRP. A cancer with HRP refers to cancer that retains the ability to perform DNA repair properly via homologous recombination (also referred to as homologous recombination DNA repair or homologous recombination repair). An ovarian cancer with HRP refers to ovarian cancer that retains the ability to perform DNA repair properly via homologous recombination (also referred to as homologous recombination DNA repair or homologous recombination repair).

The pharmaceutical composition and the method of treatment of the present invention can be used for treating cancer having resistance to platinum-based chemotherapy.

The pharmaceutical composition and the method of treatment of the present invention can be used for treating cancer having resistance to chemotherapy comprising a platinum-based drug and taxane.

The pharmaceutical composition and the method of treatment of the present invention can be used for treating cancer that recurs before administration of, or treatment with, the pharmaceutical composition of the present invention.

The cancer recurrence may happen within 6 months (or approximately 6 months) from the completion of a chemotherapy regimen comprising a platinum-based drug. The cancer recurrence may happen less than 6 months (or approximately 6 months) from the completion of a chemotherapy regimen comprising a platinum-based drug. The cancer recurrence may happen within 6 months (or approximately 6 months) from the completion of a chemotherapy regimen comprising a platinum-based drug and taxane. The cancer recurrence may happen less than 6 months (or approximately 6 months) from the completion of a chemotherapy regimen comprising a platinum-based drug and taxane.

The cancer recurrence may happen 6 months (or approximately 6 months) or later from the completion of a chemotherapy regimen comprising a platinum-based drug. The cancer recurrence may happen later than 6 months (or approximately 6 months) from the completion of a chemotherapy regimen comprising a platinum-based drug. The cancer recurrence may happen 6 months (or approximately 6 months) or later from the completion of a chemotherapy regimen comprising a platinum-based drug and taxane. The cancer recurrence may happen later than 6 months (or approximately 6 months) from the completion of a chemotherapy regimen comprising a platinum-based drug and taxane.

The anti-CDH6 antibody-drug conjugate used in the present invention can preferably be used when CDH6 expression is confirmed in cancer.

The presence or absence of a tumor marker of CDH6 can be confirmed, for example, by collecting tumor tissues from a cancer patient, preparing formalin-fixed paraffin-embedded (FFPE) samples, and conducting a test at the gene product (protein) level by immunohistochemistry (IHC), a flow cytometer, Western blot, or the like, or a test at the gene transcription level by *in situ* hybridization (ISH), quantitative PCR (q-PCR), microarray analysis, or the like, or can also be confirmed by collecting cell-free blood circulating tumor DNA (ctDNA) from a cancer patient, and conducting a test using a next-generation sequencer (NGS) or the like.

The pharmaceutical composition and the method of treatment of the present invention can preferably be used for a mammal, and can more preferably be used for a human.

The antitumor effect of the pharmaceutical composition and method of treatment of the present invention can be confirmed by, for example, generating a model in which cancer cells are transplanted to a test animal, and measuring a

reduction in tumor volume or life-prolonging effects due to applying the pharmaceutical composition and method of treatment of the present invention. Furthermore, comparison with the antitumor effect of single administrations of each of the antibody-drug conjugate and the VEGF inhibitor used in the present invention can provide confirmation of the combined effect of the antibody-drug conjugate and the VEGF inhibitor used in the present invention.

In addition, the antitumor effect of the pharmaceutical composition and method of treatment of the present invention can be confirmed, in a clinical study, with the Response Evaluation Criteria in Solid Tumors (RECIST) evaluation method, WHO's evaluation method, Macdonald's evaluation method, measurement of body weight, and other methods; and can be determined by indicators such as Complete response (CR), Partial response (PR), Progressive disease (PD), Objective response rate (ORR), Duration of response (DoR), Progression-free survival (PFS), and Overall survival (OS).

The foregoing methods can provide confirmation of superiority in terms of the antitumor effect of the pharmaceutical composition and method of treatment of the present invention compared to existing pharmaceutical compositions and methods of treatment for cancer therapy.

The pharmaceutical composition and method of treatment of the present invention can retard growth of cancer cells, suppress their proliferation, and further can kill cancer cells. These effects can allow cancer patients to be free from symptoms caused by cancer or can achieve an improvement in the QOL of cancer patients and attain a therapeutic effect by sustaining the lives of the cancer patients. Even if the pharmaceutical composition or the method of treatment does not accomplish killing cancer cells, it can provide higher QOL of cancer patients while achieving longer-term survival, by inhibiting or controlling the growth of cancer cells.

The pharmaceutical composition of the present invention can acquire a therapeutic effect by application as systemic therapy to patients, and additionally, by local application to cancer tissues.

The pharmaceutical composition of the present invention may be administered as a composition comprising one or more pharmaceutically compatible components. The pharmaceutically compatible components can be appropriately selected, for use, from pharmaceutical additives and the like that are usually used in the art, depending on the doses, administration concentrations, etc. of the antibody-drug conjugate and the VEGF inhibitor used in the present invention. For example, the antibody-drug conjugate used in the present invention may be administered as a pharmaceutical composition containing a buffer such as a histidine buffer, an excipient such as sucrose or trehalose, and a surfactant such as polysorbate 80 or 20. The pharmaceutical composition comprising the anti-CDH6 antibody-drug conjugate used in the present invention can preferably be used in an injection, can more preferably be used in an aqueous injection or a freeze-dried injection, and can still more preferably be used in a freeze-dried injection.

In the case of an aqueous injection, the pharmaceutical composition comprising the anti-CDH6 antibody-drug conjugate used in the present invention can preferably be diluted with an appropriate diluent, and then administered by intravenous drip. Examples of the diluent can include glucose solutions and physiological saline, can preferably include glucose solutions, and can more preferably include 5% glucose solutions.

In the case of a freeze-dried injection, the pharmaceutical composition comprising the anti-CDH6 antibody-drug conjugate used in the present invention can preferably be dissolved in injectable water, then diluted in a necessary amount with an appropriate diluent, and then administered by intravenous drip. Examples of the diluent can include glucose solutions and physiological saline, can preferably include glucose solutions, and can more preferably include 5% glucose solutions.

Examples of the administration route that may be used for administering the pharmaceutical composition of the present invention can include intravenous, intradermal, subcutaneous, intramuscular, and intraperitoneal routes, and can preferably include intravenous routes.

The anti-CDH6 antibody-drug conjugate used in the present invention can be administered to a human once at intervals of 1 to 180 days, can preferably be administered once a week, once every 2 weeks, once every 3 weeks, once every 4 weeks, once every 5 weeks, once every 6 weeks, once every 7 weeks, once every 8 weeks, once every 9 weeks or once every 10 weeks, and can more preferably be administered once every 3 weeks or once every 4 weeks, and can be still more preferably administered once every 3 weeks.

The anti-CDH6 antibody-drug conjugate used in the present invention can be administered at a dose of approximately 0.001 to 100 mg/kg per administration, and can be administered at preferably approximately 0.1 to approximately 15 mg/kg, more preferably approximately 0.5 to approximately 12 mg/kg, still more preferably approximately 1.0 to approximately 10 mg/kg, still more preferably approximately 1.6 to approximately 9.6 mg/kg, and still more preferably approximately 4.8 to approximately 8.0 mg/kg per administration.

The anti-CDH6 antibody-drug conjugate used in the present invention can be administered at approximately 0.1, approximately 0.2, approximately 0.3, approximately 0.4, approximately 0.5, approximately 0.6, approximately 0.7, approximately 0.8, approximately 0.9, approximately 1.0, approximately 1.1, approximately 1.2, approximately 1.3, approximately 1.4, approximately 1.5, approximately 1.6, approximately 1.7, approximately 1.8, approximately 1.9, approximately 2.0, approximately 2.1, approximately 2.2, approximately 2.3, approximately 2.4, approximately 2.5, approximately 2.6, approximately 2.7, approximately 2.8, approximately 2.9, approximately 3.0, approximately 3.1, approximately 3.2, approximately 3.3, approximately 3.4, approximately 3.5, approximately 3.6, approximately 3.7,

approximately 3.8, approximately 3.9, approximately 4.0, approximately 4.1, approximately 4.2, approximately 4.3, approximately 4.4, approximately 4.5, approximately 4.6, approximately 4.7, approximately 4.8, approximately 4.9, approximately 5.0, approximately 5.1, approximately 5.2, approximately 5.3, approximately 5.4, approximately 5.5, approximately 5.6, approximately 5.7, approximately 5.8, approximately 5.9, approximately 6.0, approximately 6.1, approximately 6.2, approximately 6.3, approximately 6.4, approximately 6.5, approximately 6.6, approximately 6.7, approximately 6.8, approximately 6.9, approximately 7.0, approximately 7.1, approximately 7.2, approximately 7.3, approximately 7.4, approximately 7.5, approximately 7.6, approximately 7.7, approximately 7.8, approximately 7.9, approximately 8.0, approximately 8.1, approximately 8.2, approximately 8.3, approximately 8.4, approximately 8.5, approximately 8.6, approximately 8.7, approximately 8.8, approximately 8.9, approximately 9.0, approximately 9.1, approximately 9.2, approximately 9.3, approximately 9.4, approximately 9.5, approximately 9.6, approximately 9.7, approximately 9.8, approximately 9.9, approximately 10.0, approximately 10.1, approximately 10.2, approximately 10.3, approximately 10.4, approximately 10.5, approximately 10.6, approximately 10.7, approximately 10.8, approximately 10.9, approximately 11.0, approximately 11.1, approximately 11.2, approximately 11.3, approximately 11.4, approximately 11.5, approximately 11.6, approximately 11.7, approximately 11.8, approximately 11.9, or approximately 12.0 mg/kg or more per administration.

The anti-CDH6 antibody-drug conjugate used in the present invention can preferably be administered at approximately 1.6 mg/kg, approximately 3.2 mg/kg, approximately 4.8 mg/kg, approximately 5.4 mg/kg, approximately 5.6 mg/kg, approximately 6.4 mg/kg, approximately 8.0 mg/kg or approximately 9.6 mg/kg per administration, can more preferably be administered at approximately 3.2 mg/kg, approximately 4.8 mg/kg, approximately 5.4 mg/kg, approximately 5.6 mg/kg, approximately 6.4 mg/kg, approximately 8.0 mg/kg or approximately 9.6 mg/kg, and can be still more preferably administered at approximately 4.8 mg/kg, approximately 5.4 mg/kg, approximately 5.6 mg/kg, approximately 6.4 mg/kg or approximately 8.0 mg/kg.

The anti-CDH6 antibody-drug conjugate used in the present invention can be administered at a dose of 0.001 to 100 mg/kg per administration, and can be administered at preferably 0.1 to 15 mg/kg, more preferably 0.5 to 12 mg/kg, still more preferably 1.0 to 10 mg/kg, still more preferably 1.6 to 9.6 mg/kg, and still more preferably 4.8 to 8.0 mg/kg per administration.

The anti-CDH6 antibody-drug conjugate used in the present invention can be administered at 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, or 12.0 mg/kg or more per administration.

The anti-CDH6 antibody-drug conjugate used in the present invention can preferably be administered at 1.6 mg/kg, 3.2 mg/kg, 4.8 mg/kg, 5.4 mg/kg, 5.6 mg/kg, 6.4 mg/kg, 8.0 mg/kg or 9.6 mg/kg per administration, can more preferably be administered at 3.2 mg/kg, 4.8 mg/kg, 5.4 mg/kg, 5.6 mg/kg, 6.4 mg/kg or 8.0 mg/kg, and can be still more preferably administered at 4.8 mg, 5.4 mg/kg, 5.6 mg/kg, 6.4 mg/kg or 8.0 mg/kg.

The anti-CDH6 antibody-drug conjugate used in the present invention can be administered at a dose of approximately 5 to approximately 3000 mg per administration, can preferably be administered at a dose of approximately 10 to approximately 2000 mg, can more preferably be administered at a dose of approximately 100 to approximately 1500 mg, and can be still more preferably administered at a dose of approximately 200 to approximately 1000 mg.

The anti-CDH6 antibody-drug conjugate used in the present invention can preferably be administered at a dose of approximately 200 mg, approximately 205 mg, approximately 210 mg, approximately 215 mg, approximately 220 mg, approximately 225 mg, approximately 230 mg, approximately 235 mg, approximately 240 mg, approximately 245 mg, approximately 250 mg, approximately 255 mg, approximately 260 mg, approximately 265 mg, approximately 270 mg, approximately 275 mg, approximately 280 mg, approximately 285 mg, approximately 290 mg, approximately 295 mg, approximately 300 mg, approximately 305 mg, approximately 310 mg, approximately 315 mg, approximately 320 mg, approximately 325 mg, approximately 330 mg, approximately 335 mg, approximately 340 mg, approximately 345 mg, approximately 350 mg, approximately 355 mg, approximately 360 mg, approximately 365 mg, approximately 370 mg, approximately 375 mg, approximately 380 mg, approximately 385 mg, approximately 390 mg, approximately 395 mg, approximately 400 mg, approximately 405 mg, approximately 410 mg, approximately 415 mg, approximately 420 mg, approximately 425 mg, approximately 430 mg, approximately 435 mg, approximately 440 mg, approximately 445 mg, approximately 450 mg, approximately 455 mg, approximately 460 mg, approximately 465 mg, approximately 470 mg, approximately 475 mg, approximately 480 mg, approximately 485 mg, approximately 490 mg, approximately 495 mg, approximately 500 mg, approximately 505 mg, approximately 510 mg, approximately 515 mg, approximately 520 mg, approximately 525 mg, approximately 530 mg, approximately 535 mg, approximately 540 mg, approximately 545 mg, approximately 550 mg, approximately 555 mg, approximately 560 mg, approximately 565 mg, approximately 570 mg, approximately 575 mg, approximately 580 mg, approximately 585 mg, approximately 590 mg, approximately 595 mg, approximately 600 mg, approximately 605 mg, approximately 610 mg, approximately 615 mg, approximately 620 mg,

approximately 625 mg, approximately 630 mg, approximately 635 mg, approximately 640 mg, approximately 645 mg, approximately 650 mg, approximately 655 mg, approximately 660 mg, approximately 665 mg, approximately 670 mg, approximately 675 mg, approximately 680 mg, approximately 685 mg, approximately 690 mg, approximately 695 mg, approximately 700 mg, approximately 705 mg, approximately 710 mg, approximately 715 mg, approximately 720 mg, approximately 725 mg, approximately 730 mg, approximately 735 mg, approximately 740 mg, approximately 745 mg, approximately 750 mg, approximately 755 mg, approximately 760 mg, approximately 765 mg, approximately 770 mg, approximately 775 mg, approximately 780 mg, approximately 785 mg, approximately 790 mg, approximately 795 mg, approximately 800 mg, approximately 805 mg, approximately 810 mg, approximately 815 mg, approximately 820 mg, approximately 825 mg, approximately 830 mg, approximately 835 mg, approximately 840 mg, approximately 845 mg, approximately 850 mg, approximately 855 mg, approximately 860 mg, approximately 865 mg, approximately 870 mg, approximately 875 mg, approximately 880 mg, approximately 885 mg, approximately 890 mg, approximately 895 mg, approximately 900 mg, approximately 905 mg, approximately 910 mg, approximately 915 mg, approximately 920 mg, approximately 925 mg, approximately 930 mg, approximately 935 mg, approximately 940 mg, approximately 945 mg, approximately 950 mg, approximately 955 mg, approximately 960 mg, approximately 965 mg, approximately 970 mg, approximately 975 mg, approximately 980 mg, approximately 985 mg, approximately 990 mg, approximately 995 mg or approximately 1000 mg per administration.

The anti-CDH6 antibody-drug conjugate used in the present invention can preferably be administered at a dose of 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 305 mg, 310 mg, 315 mg, 320 mg, 325 mg, 330 mg, 335 mg, 340 mg, 345 mg, 350 mg, 355 mg, 360 mg, 365 mg, 370 mg, 375 mg, 380 mg, 385 mg, 390 mg, 395 mg, 400 mg, 405 mg, 410 mg, 415 mg, 420 mg, 425 mg, 430 mg, 435 mg, 440 mg, 445 mg, 450 mg, 455 mg, 460 mg, 465 mg, 470 mg, 475 mg, 480 mg, 485 mg, 490 mg, 495 mg, 500 mg, 505 mg, 510 mg, 515 mg, 520 mg, 525 mg, 530 mg, 535 mg, 540 mg, 545 mg, 550 mg, 555 mg, 560 mg, 565 mg, 570 mg, 575 mg, 580 mg, 585 mg, 590 mg, 595 mg, 600 mg, 605 mg, 610 mg, 615 mg, 620 mg, 625 mg, 630 mg, 635 mg, 640 mg, 645 mg, 650 mg, 655 mg, 660 mg, 665 mg, 670 mg, 675 mg, 680 mg, 685 mg, 690 mg, 695 mg, 700 mg, 705 mg, 710 mg, 715 mg, 720 mg, 725 mg, 730 mg, 735 mg, 740 mg, 745 mg, 750 mg, 755 mg, 760 mg, 765 mg, 770 mg, 775 mg, 780 mg, 785 mg, 790 mg, 795 mg, 800 mg, 805 mg, 810 mg, 815 mg, 820 mg, 825 mg, 830 mg, 835 mg, 840 mg, 845 mg, 850 mg, 855 mg, 860 mg, 865 mg, 870 mg, 875 mg, 880 mg, 885 mg, 890 mg, 895 mg, 900 mg, 905 mg, 910 mg, 915 mg, 920 mg, 925 mg, 930 mg, 935 mg, 940 mg, 945 mg, 950 mg, 955 mg, 960 mg, 965 mg, 970 mg, 975 mg, 980 mg, 985 mg, 990 mg, 995 mg or 1000 mg per administration.

The dosage regimen of the anti-CDH6 antibody-drug conjugate used in the present invention may be, for example, 0.1 mg/kg (at intervals of 3 weeks; hereinafter, referred to as "q3w"), 0.2 mg/kg (q3w), 0.3 mg/kg (q3w), 0.4 mg/kg (q3w), 0.5 mg/kg (q3w), 0.6 mg/kg (q3w), 0.7 mg/kg (q3w), 0.8 mg/kg (q3w), 0.9 mg/kg (q3w), 1.0 mg/kg (q3w), 1.1 mg/kg (q3w), 1.2 mg/kg (q3w), 1.3 mg/kg (q3w), 1.4 mg/kg (q3w), 1.5 mg/kg (q3w), 1.6 mg/kg (q3w), 1.7 mg/kg (q3w), 1.8 mg/kg (q3w), 1.9 mg/kg (q3w), 2.0 mg/kg (q3w), 2.1 mg/kg (q3w), 2.2 mg/kg (q3w), 2.3 mg/kg (q3w), 2.4 mg/kg (q3w), 2.5 mg/kg (q3w), 2.6 mg/kg (q3w), 2.7 mg/kg (q3w), 2.8 mg/kg (q3w), 2.9 mg/kg (q3w), 3.0 mg/kg (q3w), 3.1 mg/kg (q3w), 3.2 mg/kg (q3w), 3.3 mg/kg (q3w), 3.4 mg/kg (q3w), 3.5 mg/kg (q3w), 3.6 mg/kg (q3w), 3.7 mg/kg (q3w), 3.8 mg/kg (q3w), 3.9 mg/kg (q3w), 4.0 mg/kg (q3w), 4.1 mg/kg (q3w), 4.2 mg/kg (q3w), 4.3 mg/kg (q3w), 4.4 mg/kg (q3w), 4.5 mg/kg (q3w), 4.6 mg/kg (q3w), 4.7 mg/kg (q3w), 4.8 mg/kg (q3w), 4.9 mg/kg (q3w), 5.0 mg/kg (q3w), 5.1 mg/kg (q3w), 5.2 mg/kg (q3w), 5.3 mg/kg (q3w), 5.4 mg/kg (q3w), 5.5 mg/kg (q3w), 5.6 mg/kg (q3w), 5.7 mg/kg (q3w), 5.8 mg/kg (q3w), 5.9 mg/kg (q3w), 6.0 mg/kg (q3w), 6.1 mg/kg (q3w), 6.2 mg/kg (q3w), 6.3 mg/kg (q3w), 6.4 mg/kg (q3w), 6.5 mg/kg (q3w), 6.6 mg/kg (q3w), 6.7 mg/kg (q3w), 6.8 mg/kg (q3w), 6.9 mg/kg (q3w), 7.0 mg/kg (q3w), 7.1 mg/kg (q3w), 7.2 mg/kg (q3w), 7.3 mg/kg (q3w), 7.4 mg/kg (q3w), 7.5 mg/kg (q3w), 7.6 mg/kg (q3w), 7.7 mg/kg (q3w), 7.8 mg/kg (q3w), 7.9 mg/kg (q3w), 8.0 mg/kg (q3w), 8.1 mg/kg (q3w), 8.2 mg/kg (q3w), 8.3 mg/kg (q3w), 8.4 mg/kg (q3w), 8.5 mg/kg (q3w), 8.6 mg/kg (q3w), 8.7 mg/kg (q3w), 8.8 mg/kg (q3w), 8.9 mg/kg (q3w), 9.0 mg/kg (q3w), 9.1 mg/kg (q3w), 9.2 mg/kg (q3w), 9.3 mg/kg (q3w), 9.4 mg/kg (q3w), 9.5 mg/kg (q3w), 9.6 mg/kg (q3w), 9.7 mg/kg (q3w), 9.8 mg/kg (q3w), 9.9 mg/kg (q3w), 10.0 mg/kg (q3w), 10.1 mg/kg (q3w), 10.2 mg/kg (q3w), 10.3 mg/kg (q3w), 10.4 mg/kg (q3w), 10.5 mg/kg (q3w), 10.6 mg/kg (q3w), 10.7 mg/kg (q3w), 10.8 mg/kg (q3w), 10.9 mg/kg (q3w), 11.0 mg/kg (q3w), 11.1 mg/kg (q3w), 11.2 mg/kg (q3w), 11.3 mg/kg (q3w), 11.4 mg/kg (q3w), 11.5 mg/kg (q3w), 11.6 mg/kg (q3w), 11.7 mg/kg (q3w), 11.8 mg/kg (q3w), 11.9 mg/kg (q3w) or 12.0 mg/kg (q3w).

The dosage regimen of the anti-CDH6 antibody-drug conjugate used in the present invention is preferably 1.6 mg/kg (q3w), 3.2 mg/kg (q3w), 4.8 mg/kg (q3w), 5.4 mg/kg (q3w), 5.6 mg/kg (q3w), 6.4 mg/kg (q3w), 8.0 mg/kg (q3w) or 9.6 mg/kg (q3w), more preferably 3.2 mg/kg (q3w), 4.8 mg/kg (q3w), 5.4 mg/kg (q3w), 5.6 mg/kg (q3w), 6.4 mg/kg (q3w) or 8.0 mg/kg (q3w), and still more preferably 4.8 mg/kg (q3w), 5.4 mg/kg (q3w), 5.6 mg/kg (q3w), 6.4 mg/kg (q3w) or 8.0 mg/kg (q3w).

The dosage regimen of the anti-CDH6 antibody-drug conjugate used in the present invention is preferably 200 mg (q3w), 205 mg (q3w), 210 mg (q3w), 215 mg (q3w), 220 mg (q3w), 225 mg (q3w), 230 mg (q3w), 235 mg (q3w), 240 mg (q3w), 245 mg (q3w), 250 mg (q3w), 255 mg (q3w), 260 mg (q3w), 265 mg (q3w), 270 mg (q3w), 275 mg (q3w), 280 mg (q3w), 285 mg (q3w), 290 mg (q3w), 295 mg (q3w), 300 mg (q3w), 305 mg (q3w), 310 mg (q3w), 315 mg (q3w), 320 mg (q3w), 325 mg (q3w), 330 mg (q3w), 335 mg (q3w), 340 mg (q3w), 345 mg (q3w), 350 mg (q3w), 355 mg (q3w), 360 mg

(q3w), 365 mg (q3w), 370 mg (q3w), 375 mg (q3w), 380 mg (q3w), 385 mg (q3w), 390 mg (q3w), 395 mg (q3w), 400 mg (q3w), 405 mg (q3w), 410 mg (q3w), 415 mg (q3w), 420 mg (q3w), 425 mg (q3w), 430 mg (q3w), 435 mg (q3w), 440 mg (q3w), 445 mg (q3w), 450 mg (q3w), 455 mg (q3w), 460 mg (q3w), 465 mg (q3w), 470 mg (q3w), 475 mg (q3w), 480 mg (q3w), 485 mg (q3w), 490 mg (q3w), 495 mg (q3w), 500 mg (q3w), 505 mg (q3w), 510 mg (q3w), 515 mg (q3w), 520 mg (q3w), 525 mg (q3w), 530 mg (q3w), 535 mg (q3w), 540 mg (q3w), 545 mg (q3w), 550 mg (q3w), 555 mg (q3w), 560 mg (q3w), 565 mg (q3w), 570 mg (q3w), 575 mg (q3w), 580 mg (q3w), 585 mg (q3w), 590 mg (q3w), 595 mg (q3w), 600 mg (q3w), 605 mg (q3w), 610 mg (q3w), 615 mg (q3w), 620 mg (q3w), 625 mg (q3w), 630 mg (q3w), 635 mg (q3w), 640 mg (q3w), 645 mg (q3w), 650 mg (q3w), 655 mg (q3w), 660 mg (q3w), 665 mg (q3w), 670 mg (q3w), 675 mg (q3w), 680 mg (q3w), 685 mg (q3w), 690 mg (q3w), 695 mg (q3w), 700 mg (q3w), 705 mg (q3w), 710 mg (q3w), 715 mg (q3w), 720 mg (q3w), 725 mg (q3w), 730 mg (q3w), 735 mg (q3w), 740 mg (q3w), 745 mg (q3w), 750 mg (q3w), 755 mg (q3w), 760 mg (q3w), 765 mg (q3w), 770 mg (q3w), 775 mg (q3w), 780 mg (q3w), 785 mg (q3w), 790 mg (q3w), 795 mg (q3w), 800 mg (q3w), 805 mg (q3w), 810 mg (q3w), 815 mg (q3w), 820 mg (q3w), 825 mg (q3w), 830 mg (q3w), 835 mg (q3w), 840 mg (q3w), 845 mg (q3w), 850 mg (q3w), 855 mg (q3w), 860 mg (q3w), 865 mg (q3w), 870 mg (q3w), 875 mg (q3w), 880 mg (q3w), 885 mg (q3w), 890 mg (q3w), 895 mg (q3w), 900 mg (q3w), 905 mg (q3w), 910 mg (q3w), 915 mg (q3w), 920 mg (q3w), 925 mg (q3w), 930 mg (q3w), 935 mg (q3w), 940 mg (q3w), 945 mg (q3w), 950 mg (q3w), 955 mg (q3w), 960 mg (q3w), 965 mg (q3w), 970 mg (q3w), 975 mg (q3w), 980 mg (q3w), 985 mg (q3w), 990 mg (q3w), 995 mg (q3w) or 1000 mg (q3w).

When the VEGF inhibitor used in the present invention is bevacizumab, examples of the administration method include, but are not limited to, the following dosages and administrations. For example, bevacizumab is injected by intravenous drip at 5 mg/kg (body weight) or 10 mg/kg (body weight) per administration. Administration intervals are set to 2 weeks or more.

As another dosage and administration, bevacizumab is injected by intravenous drip at 7.5 mg/kg (body weight) per administration. Administration intervals are set to 3 weeks or more.

As another dosage and administration, bevacizumab is injected by intravenous drip at 15 mg/kg (body weight) per administration. Administration intervals are set to 3 weeks or more.

As another dosage and administration, bevacizumab is injected by intravenous drip at 10 mg/kg (body weight) per administration. Administration intervals are set to 2 weeks or more.

As another dosage and administration, bevacizumab is injected by intravenous drip at 10 mg/kg (body weight) per administration at intervals of 2 weeks or at 15 mg/kg (body weight) per administration at intervals of 3 weeks.

As another dosage and administration, bevacizumab is injected by intravenous drip at 5 mg/kg or 10 mg/kg per administration at intervals of 2 weeks.

As another dosage and administration, bevacizumab is injected by intravenous drip at 15 mg/kg per administration at intervals of 3 weeks.

As another dosage and administration, bevacizumab is injected by intravenous drip at 10 mg/kg per administration at intervals of 2 weeks.

When the VEGF inhibitor used in the present invention is ramucirumab, examples of the administration method include, but are not limited to, the following dosages and administrations. For example, ramucirumab is injected by intravenous drip at 8 mg/kg (body weight) per administration over approximately 60 minutes every 2 weeks. The length of time of administration at the second administration or later can be shortened by up to 30 minutes.

As another dosage and administration, ramucirumab is injected by intravenous drip at 10 mg/kg (body weight) per administration over approximately 60 minutes every 3 weeks. The length of time of administration at the second administration or later can be shortened by up to 30 minutes.

As another dosage and administration, ramucirumab is injected by intravenous drip at 10 mg/kg (body weight) per administration over approximately 60 minutes every 2 weeks. The length of time of administration at the second administration or later can be shortened by up to 30 minutes.

When the VEGF inhibitor used in the present invention is aflibercept, examples of the administration method include, but are not limited to, the following dosages and administrations. For example, aflibercept is injected by intravenous drip at 4 mg/kg (body weight) per administration over approximately 60 minutes every 2 weeks.

The pharmaceutical composition and the method of treatment of the present invention may further comprise a therapeutic agent for cancer other than the anti-CDH6 antibody-drug conjugate according to the present invention and the VEGF inhibitor. The pharmaceutical composition and the method of treatment of the present invention can also be administered in combination with an additional therapeutic agent for cancer, and can thereby enhance an antitumor effect. The additional therapeutic agent for cancer used for such a purpose may be administered to an individual at the same time (at approximately the same time), separately, or continuously, together with the pharmaceutical composition of the present invention. Otherwise, the additional therapeutic agent and the pharmaceutical composition may each be administered to the subject at different administration intervals. Such a therapeutic agent for cancer is not limited as long as the agent has an antitumor activity. Examples thereof can include at least one selected from the group consisting of irinotecan (CPT-11), cisplatin, carboplatin, oxaliplatin, fluorouracil (5-FU), gemcitabine, capecitabine, doxorubicin, epirubicin, cyclophosphamide, mitomycin C, tegafur/gimeracil/oteracil, panitumumab, regorafenib, trifluridine/tipiracil, gefitinib, erlotinib, afatinib, methotrexate, pemetrexed, tamoxifen, toremifene, fulvestrant, leuprorelin, goserelin, letrozole, anastrozole, progesterone formulations, and lapatinib.

The pharmaceutical composition and the method of treatment of the present invention may be used in combination with radiotherapy. For example, a cancer patient receives radiotherapy before and/or after or at the same time (at approximately the same time) as treatment with the pharmaceutical composition of the present invention.

The pharmaceutical composition and the method of treatment of the present invention may be used as adjunctive chemotherapy combined with surgery. The pharmaceutical composition of the present invention may be administered for the purpose of decreasing a tumor size before surgery (neoadjuvant chemotherapy), or may be administered for the purpose of preventing tumor recurrence after surgery (adjuvant chemotherapy).

The pharmaceutical composition and the method of treatment of the present invention may be used as maintenance therapy. For example, after initial chemotherapy, treatment is continued for the purpose of preventing recurrence.

The present invention includes a method of treatment of a disease, comprising administering the anti-CDH6 antibody-drug conjugate of the present application and a VEGF inhibitor in combination to a subject in need of the treatment. The present invention includes the anti-CDH6 antibody-drug conjugate of the present application for use in combination with a VEGF inhibitor for treating a disease. The present invention includes use of the anti-CDH6 antibody-drug conjugate of the present application combined with a VEGF inhibitor for the manufacture of a medicament for treating a disease. The present invention includes a pharmaceutical product comprising the anti-CDH6 antibody-drug conjugate of the present application and a VEGF inhibitor. The present invention includes a combination medicament comprising the anti-CDH6 antibody-drug conjugate of the present application and a VEGF inhibitor. The present invention includes a pharmaceutical combination comprising the anti-CDH6 antibody-drug conjugate of the present application and a VEGF inhibitor. The present invention includes use of the anti-CDH6 antibody-drug conjugate of the present application combined with a VEGF inhibitor for treating a disease. The present invention includes a medicament comprising the anti-CDH6 antibody-drug conjugate of the present application and a VEGF inhibitor.

[Examples]

Hereinafter, the present invention will be specifically described in the following examples. However, these examples are not intended to limit the scope of the present invention. Furthermore, these examples should not be construed in a limited manner by any means.

Production Example 1: Production of anti-CDH6 antibody-drug conjugate

In accordance with the production method described in International Publication No. WO 2018/212136 with use of a humanized anti-CDH6 antibody (antibody comprising a heavy chain consisting of the amino acid sequence shown in SEQ ID NO: 29 and a light chain consisting of the amino acid sequence shown in SEQ ID NO: 38), an antibody-drug conjugate in which a drug-linker represented by the formula:

[Formula 14]

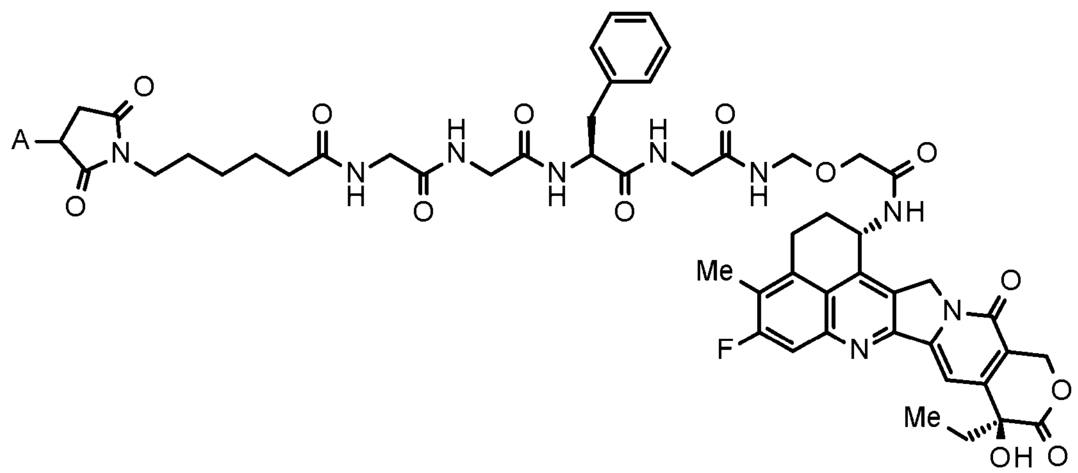

wherein A represents a connecting position to an antibody,

is conjugated to the anti-CDH6 antibody via a thioether bond (hereinafter, referred to as the "anti-CDH6 antibody-drug conjugate (1)") was produced. The DAR of the anti-CDH6 antibody-drug conjugate (1) was 7.9.

Example 1: Antitumor test (1)

Mouse: Six-week-old female BALB/c nude mice (Charles River Laboratories Japan Inc.) were subjected to an experiment.

Measurement and calculation expression: The long diameter and short diameter of each tumor were measured twice or three times a week using electronic digital calipers (CD-15CX, Mitutoyo Corp.), and the volume of the tumor was then calculated. The following expression was used in the calculation.

Tumor volume ($mm^3$) = 1/2 $\times$ Long diameter (mm) $\times$ [Short diameter (mm)]$^2$

The anti-CDH6 antibody-drug conjugate (1) was diluted with ABS buffer (10 mM acetate buffer (pH 5.5), 5% sorbitol), and the dilution was intravenously administered at a dose of 10 mL/kg to the tail of each mouse. Bevacizumab (Avastin(R), 100 mg/4 mL) was diluted with physiological saline, and the dilution was intraperitoneally administered at a dose of 10 mL/kg.

Human ovarian cancer line OV-90 cells purchased from ATCC (American Type Culture Collection) were suspended in Matrigel Matrix, and the cell suspension was subcutaneously inoculated at a dose of 2.5 $\times$ $10^6$ cells to the right flank region of each female nude mouse. 17 days after inoculation, the mice were randomly grouped (Day 0). The anti-CDH6 antibody-drug conjugate (1) was administered at a dose of 3 mg/kg on Day 0. Bevacizumab was administered at a dose of 5 mg/kg on Day 0. Their respective single administration groups and combined administration group, and an ABS buffer administration group as a control group, were established.

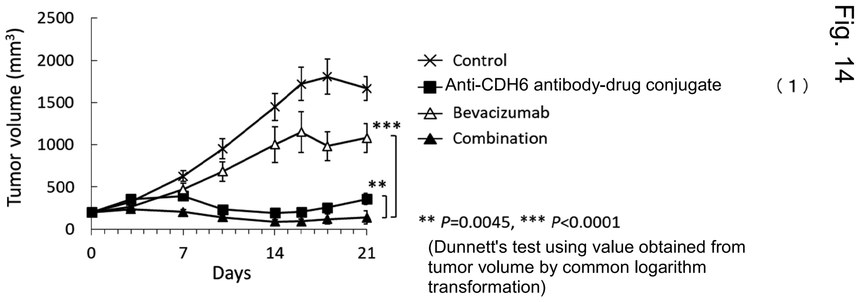

The result of combined use of the anti-CDH6 antibody-drug conjugate (1) and bevacizumab is shown in Figure 14. In Figure 14, the abscissa depicts the number of days after the start of administration, and the ordinate depicts tumor volume.

Tumor growth inhibition (TGI) is calculated according to the following expression.

TGI (%) = [1 - (Average tumor volume of the drug administration group / Average tumor volume of the control group)] $\times$ 100

On Day 21, the tumor growth inhibition (TGI) by the single administration of bevacizumab was 35%. The TGI by the single administration of the anti-CDH6 antibody-drug conjugate (1) was 79%. On the other hand, the combined administration of the anti-CDH6 antibody-drug conjugate (1) and bevacizumab was found to have a significantly better tumor growth suppressing effect than that of the single administration of the anti-CDH6 antibody-drug conjugate (1) and the single administration of bevacizumab ($P = 0.0045$ and $P < 0.0001$, respectively; calculated by Dunnett's test using a value obtained from tumor volume by common logarithm transformation). TGI by the combined administration (92%) was higher than that of the respective single administrations, demonstrating that the combined use enhances the antitumor effect.

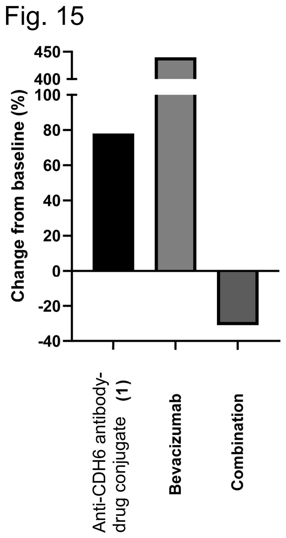

Change from baseline (%) (percentage change in average tumor volume on Day 21 from an average tumor volume on Day 0 as a baseline) was calculated according to the following expression, and is shown in Figure 15.

[Expression 1]

$$\text{Change from baseline (\%)} = \frac{\text{Average tumor volume on Day 21} - \text{Average tumor volume on Day 0}}{\text{Average tumor volume on Day 0}} \times 100$$

In addition, the change from baseline (%) of each group is described in Table 1. As shown in Figure 15 and Table 1, the change from baseline (%) was a negative value in the combined administration group, demonstrating a strong combinatorial effect with regression.

[Table 1]

| Compound | Change from baseline (%) |
|---|---|
| Anti-CDH6 antibody-drug conjugate (1) | 78 |
| Bevacizumab | 440 |
| Anti-CDH6 antibody-drug conjugate (1) + bevacizumab | -31 |

Furthermore, antitumor activity was evaluated as described in Bissery et al., Cancer Res., 51: 4845-4852 (1991). Specifically, T/C (%) was calculated according to the following expression when a median tumor volume in the control group reached 750 to 1500 $mm^3$. T/C of more than 42% was evaluated as "inactive", T/C of 42% or less was evaluated as "active", and T/C of less than 10% was evaluated as "highly active".

[Expression 2]

$$T/C\ (\%) = \frac{\text{Median tumor volume of the drug administration group}}{\text{Median tumor volume of the control group}} \times 100$$

On Day 10, the median tumor volume of the control group was 885 $mm^3$, the median tumor volume in the single administration of the anti-CDH6 antibody-drug conjugate (1) was 231 $mm^3$ (T/C = 26%), the median tumor volume in the single administration of bevacizumab was 630 $mm^3$ (T/C = 71%), and the median tumor volume in the combined administration of the anti-CDH6 antibody-drug conjugate (1) and bevacizumab was 129 $mm^3$ (T/C = 15%).

On Day 14, the median tumor volume of the control group was 1357 $mm^3$, the median tumor volume in the single administration of the anti-CDH6 antibody-drug conjugate (1) was 169 $mm^3$ (T/C = 12%), the median tumor volume in the single administration of bevacizumab was 852 $mm^3$ (T/C = 63%), and the median tumor volume in the combined administration of the anti-CDH6 antibody-drug conjugate (1) and bevacizumab was 78 $mm^3$ (T/C = 6%).

Thus, on Day 14, it was found that the single administration of the anti-CDH6 antibody-drug conjugate (1) was "active" and the single administration of bevacizumab was "inactive" whereas the combined administration group of the anti-CDH6 antibody-drug conjugate (1) and bevacizumab was "highly active" (Table 2).

[Table 2]

| Compound | T/C (%) on Day 14 | Results |
|---|---|---|
| Anti-CDH6 antibody-drug conjugate (1) | 12 | Active |
| Bevacizumab | 63 | Inactive |
| Anti-CDH6 antibody-drug conjugate (1) + bevacizumab | 6 | Highly active |

None of the single administration groups and the combined administration group exhibited any particularly notable finding such as severe weight loss. Hence, the combination of the anti-CDH6 antibody-drug conjugate (1) and bevacizumab increased efficacy without increasing toxicity.

[Industrial Applicability]

From the above-described experimental results, the antibody-drug conjugate according to the present invention has been found to exhibit an excellent antitumor effect by administering the antibody-drug conjugate and a VEGF inhibitor in combination.

[Free Text of Sequence Listing]

SEQ ID NO: 1: Amino acid sequence of human CDH6 EC3
SEQ ID NO: 2: Amino acid sequence of chG019 CDRH1
SEQ ID NO: 3: Amino acid sequence of chG019 CDRH2
SEQ ID NO: 4: Amino acid sequence of chG019 CDRH3

SEQ ID NO: 5: Amino acid sequence of chG019 CDRL1
SEQ ID NO: 6: Amino acid sequence of chG019 CDRL2
SEQ ID NO: 7: Amino acid sequence of chG019 CDRL3
SEQ ID NO: 8: Amino acid sequence of rG019 CDRH2
SEQ ID NO: 9: Amino acid sequence of rG055 CDRH1
SEQ ID NO: 10: Amino acid sequence of rG055 CDRH2
SEQ ID NO: 11: Amino acid sequence of rG055 CDRH3
SEQ ID NO: 12: Amino acid sequence of rG055 CDRL1
SEQ ID NO: 13: Amino acid sequence of rG055 CDRL2
SEQ ID NO: 14: Amino acid sequence of rG055 CDRL3
SEQ ID NO: 15: Amino acid sequence of rG056 CDRH1
SEQ ID NO: 16: Amino acid sequence of rG056 CDRH2
SEQ ID NO: 17: Amino acid sequence of rG056 CDRH3
SEQ ID NO: 18: Amino acid sequence of rG056 CDRL1
SEQ ID NO: 19: Amino acid sequence of rG056 CDRL2
SEQ ID NO: 20: Amino acid sequence of rG056 CDRL3
SEQ ID NO: 21: Amino acid sequence of rG061 CDRH1
SEQ ID NO: 22: Amino acid sequence of rG061 CDRH2
SEQ ID NO: 23: Amino acid sequence of rG061 CDRH3
SEQ ID NO: 24: Amino acid sequence of rG061 CDRL1
SEQ ID NO: 25: Amino acid sequence of rG061 CDRL2
SEQ ID NO: 26: Amino acid sequence of rG061 CDRL3
SEQ ID NO: 27: Full-length amino acid sequence of a hH01 heavy chain
SEQ ID NO: 28: Amino acid sequence of a hH01 heavy chain variable region
SEQ ID NO: 29: Full-length amino acid sequence of the hH01 heavy chain except for a signal sequence
SEQ ID NO: 30: Full-length amino acid sequence of a hH02 heavy chain
SEQ ID NO: 31: Amino acid sequence of a hH02 heavy chain variable region
SEQ ID NO: 32: Full-length amino acid sequence of the hH02 heavy chain except for a signal sequence
SEQ ID NO: 33: Full-length amino acid sequence of a hH04 heavy chain
SEQ ID NO: 34: Amino acid sequence of a hH04 heavy chain variable region
SEQ ID NO: 35: Full-length amino acid sequence of the hH04 heavy chain except for a signal sequence
SEQ ID NO: 36: Full-length amino acid sequence of a hL02 light chain
SEQ ID NO: 37: Amino acid sequence of a hL02 light chain variable region
SEQ ID NO: 38: Full-length amino acid sequence of the hL02 light chain except for a signal sequence
SEQ ID NO: 39: Full-length amino acid sequence of a hL03 light chain
SEQ ID NO: 40: Amino acid sequence of a hL03 light chain variable region
SEQ ID NO: 41: Full-length amino acid sequence of the hL03 light chain except for a signal sequence

## Claims

1. A pharmaceutical composition comprising an anti-CDH6 antibody-drug conjugate, wherein the anti-CDH6 antibody-drug conjugate and a VEGF inhibitor are administered in combination, and
the anti-CDH6 antibody-drug conjugate is an antibody-drug conjugate in which a drug-linker represented by the formula:

[Formula 1]

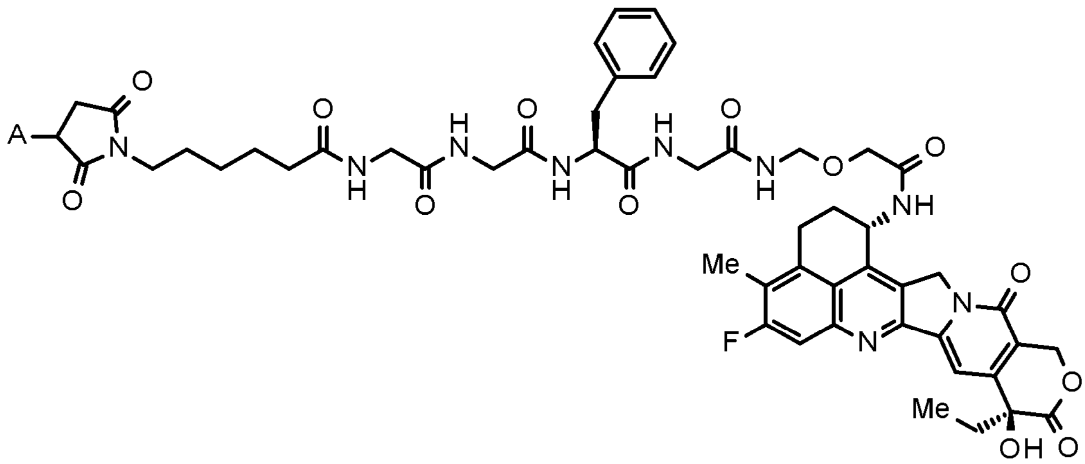

wherein A represents a connecting position to an anti-CDH6 antibody or a functional fragment of the antibody, is conjugated to the anti-CDH6 antibody or the functional fragment of the antibody via a thioether bond.

2. The pharmaceutical composition according to claim 1, wherein the anti-CDH6 antibody or the functional fragment of the antibody is an antibody which specifically binds to the amino acid sequence shown in SEQ ID NO: 1 and has an internalization ability that permits cellular uptake, or a functional fragment of the antibody.

3. The pharmaceutical composition according to claim 1 or 2, wherein the anti-CDH6 antibody or the functional fragment of the antibody is an antibody comprising CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, or a functional fragment of the antibody.

4. The pharmaceutical composition according to any one of claims 1 to 3, wherein the anti-CDH6 antibody or the functional fragment of the antibody is an antibody comprising a heavy chain variable region consisting of the amino acid sequence shown in SEQ ID NO: 28 and a light chain variable region consisting of the amino acid sequence shown in SEQ ID NO: 37, or a functional fragment of the antibody.

5. The pharmaceutical composition according to any one of claims 1 to 4, wherein the anti-CDH6 antibody or the functional fragment of the antibody is an antibody comprising a heavy chain consisting of the amino acid sequence shown in SEQ ID NO: 29 and a light chain consisting of the amino acid sequence shown in SEQ ID NO: 38, or a functional fragment of the antibody.

6. The pharmaceutical composition according to any one of claims 1 to 5, wherein the anti-CDH6 antibody or the functional fragment of the antibody is an antibody in which the heavy chain or the light chain has undergone one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, conversion of N-terminal glutamine or N-terminal glutamic acid to pyroglutamic acid, and a deletion of one or two amino acids from the carboxyl terminus, or a functional fragment of the antibody.

7. The pharmaceutical composition according to claim 6, wherein the anti-CDH6 antibody or the functional fragment of the antibody is an antibody in which one or two amino acids are deleted from the carboxyl terminus of a heavy chain thereof, or a functional fragment of the antibody.

8. The pharmaceutical composition according to claim 7, wherein the anti-CDH6 antibody or the functional fragment of the antibody is an antibody in which one amino acid is deleted from the carboxyl terminus of each of the two heavy chains thereof, or a functional fragment of the antibody.

9. The pharmaceutical composition according to any one of claims 6 to 8, wherein the deleted amino acid is lysine.

10. The pharmaceutical composition according to any one of claims 6 to 9, wherein the anti-CDH6 antibody or the functional fragment of the antibody is an antibody in which a proline residue at the carboxyl terminus of a heavy chain thereof is further amidated, or a functional fragment of the antibody.

11. The pharmaceutical composition according to any one of claims 1 to 10, wherein the average number of units of the drug-linker structure conjugated per antibody in the anti-CDH6 antibody-drug conjugate is in the range of from 1 to 10.

12. The pharmaceutical composition according to any one of claims 1 to 10, wherein the average number of units of the drug-linker structure conjugated per antibody in the anti-CDH6 antibody-drug conjugate is from 7 to 8.

13. The pharmaceutical composition according to any one of claims 1 to 12, wherein the VEGF inhibitor is an anti-VEGF antibody, an anti-VEGF receptor antibody, a fusion protein comprising a VEGF receptor extracellular domain, or a multispecific antibody comprising an anti-VEGF binding domain.

14. The pharmaceutical composition according to claim 13, wherein the VEGF inhibitor is an anti-VEGF antibody.

15. The pharmaceutical composition according to claim 14, wherein the anti-VEGF antibody is bevacizumab.

16. The pharmaceutical composition according to claim 13, wherein the VEGF inhibitor is an anti-VEGF receptor antibody.

17. The pharmaceutical composition according to claim 16, wherein the anti-VEGF receptor antibody is ramucirumab.

18. The pharmaceutical composition according to claim 13, wherein the VEGF inhibitor is a fusion protein comprising a VEGF receptor extracellular domain.

19. The pharmaceutical composition according to claim 18, wherein the fusion protein comprising a VEGF receptor extracellular domain is aflibercept.

20. The pharmaceutical composition according to claim 13, wherein the VEGF inhibitor is a multispecific antibody comprising an anti-VEGF binding domain.

21. The pharmaceutical composition according to claim 20, wherein the multispecific antibody is a bispecific antibody.

22. The pharmaceutical composition according to any one of claims 1 to 21, wherein the anti-CDH6 antibody-drug conjugate and the VEGF inhibitor are separately contained in different formulations, and are administered at the same time or at different times.

23. The pharmaceutical composition according to any one of claims 1 to 21, wherein the anti-CDH6 antibody-drug conjugate and the VEGF inhibitor are contained in a single formulation for administration.

24. The pharmaceutical composition according to any one of claims 1 to 23, wherein the pharmaceutical composition is for use in treating cancer.

25. The pharmaceutical composition according to any one of claims 1 to 23, wherein the pharmaceutical composition is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, and nasopharyngeal cancer.

26. The pharmaceutical composition according to any one of claims 1 to 23, wherein the pharmaceutical composition is for use in treating at least one selected from the group consisting of renal cell carcinoma, renal clear cell carcinoma, and papillary renal cell carcinoma.

**27.** The pharmaceutical composition according to any one of claims 1 to 23, wherein the pharmaceutical composition is for use in treating at least one selected from the group consisting of ovarian cancer, ovarian serous adenocarcinoma, ovarian clear cell carcinoma, endometrioid ovarian cancer, and ovarian mucinous tumor.

**28.** The pharmaceutical composition according to any one of claims 1 to 23, wherein the pharmaceutical composition is for use in treating ovarian cancer.

**29.** The pharmaceutical composition according to claim 28, wherein the ovarian cancer is epithelial ovarian cancer, fallopian tube cancer, or primary peritoneal cancer.

**30.** The pharmaceutical composition according to claim 28 or 29, wherein the ovarian cancer is metastatic.

**31.** A pharmaceutical composition comprising an anti-CDH6 antibody-drug conjugate, wherein the anti-CDH6 antibody-drug conjugate and a VEGF inhibitor are administered in combination, and
the anti-CDH6 antibody-drug conjugate is an antibody-drug conjugate represented by the formula:

[Formula 2]

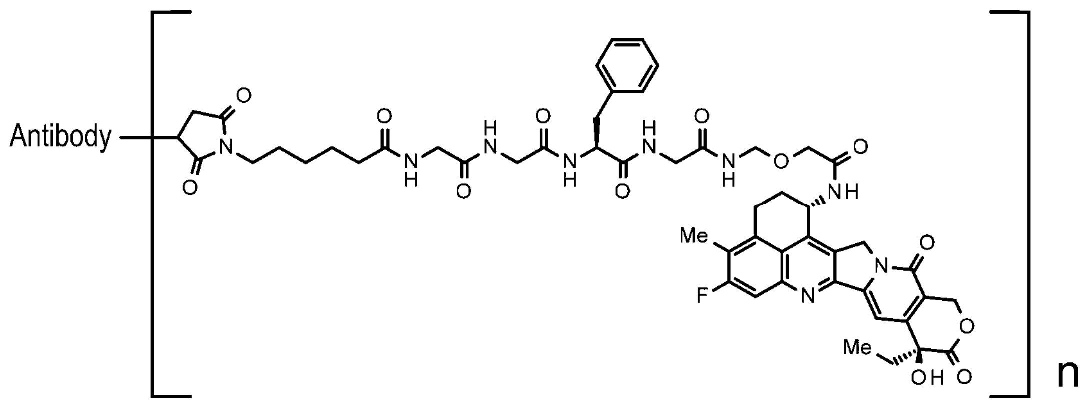

wherein Antibody is an anti-CDH6 antibody, a drug-linker is conjugated to the antibody via a thioether bond, and n represents an average number of units of the drug-linker conjugated per antibody.

**32.** The pharmaceutical composition according to claim 31, wherein the anti-CDH6 antibody is an antibody comprising a heavy chain consisting of the amino acid sequence shown in SEQ ID NO: 29 and a light chain consisting of the amino acid sequence shown in SEQ ID NO: 38.

**33.** The pharmaceutical composition according to claim 31 or 32, wherein a lysine residue is deleted from the carboxyl terminus of a heavy chain of the anti-CDH6 antibody.

**34.** The pharmaceutical composition according to any one of claims 31 to 33, wherein the average number of units of the drug-linker conjugated per antibody in the anti-CDH6 antibody-drug conjugate is in the range of from 7 to 8.

**35.** The pharmaceutical composition according to any one of claims 31 to 34, wherein the VEGF inhibitor is an anti-VEGF antibody, an anti-VEGF receptor antibody, a fusion protein comprising a VEGF receptor extracellular domain, or a multispecific antibody comprising an anti-VEGF binding domain.

**36.** The pharmaceutical composition according to claim 35, wherein the VEGF inhibitor is an anti-VEGF antibody.

**37.** The pharmaceutical composition according to claim 36, wherein the anti-VEGF antibody is bevacizumab.

**38.** The pharmaceutical composition according to claim 35, wherein the VEGF inhibitor is an anti-VEGF receptor antibody.

**39.** The pharmaceutical composition according to claim 38, wherein the anti-VEGF receptor antibody is ramucirumab.

**40.** The pharmaceutical composition according to claim 35, wherein the VEGF inhibitor is a fusion protein comprising a VEGF receptor extracellular domain.

**41.** The pharmaceutical composition according to claim 40, wherein the fusion protein comprising a VEGF receptor extracellular domain is aflibercept.

**42.** The pharmaceutical composition according to claim 35, wherein the VEGF inhibitor is a multispecific antibody comprising an anti-VEGF binding domain.

**43.** The pharmaceutical composition according to claim 42, wherein the multispecific antibody is a bispecific antibody.

**44.** The pharmaceutical composition according to any one of claims 31 to 43, wherein the anti-CDH6 antibody-drug conjugate and the VEGF inhibitor are separately contained in different formulations, and are administered at the same time or at different times.

**45.** The pharmaceutical composition according to any one of claims 31 to 43, wherein the anti-CDH6 antibody-drug conjugate and the VEGF inhibitor are contained in a single formulation for administration.

**46.** The pharmaceutical composition according to any one of claims 31 to 45, wherein the pharmaceutical composition is for use in treating cancer.

**47.** The pharmaceutical composition according to any one of claims 31 to 45, wherein the pharmaceutical composition is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

**48.** The pharmaceutical composition according to any one of claims 31 to 45, wherein the pharmaceutical composition is for use in treating at least one selected from the group consisting of renal cell carcinoma, renal clear cell carcinoma, and papillary renal cell carcinoma.

**49.** The pharmaceutical composition according to any one of claims 31 to 45, wherein the pharmaceutical composition is for use in treating at least one selected from the group consisting of ovarian cancer, ovarian serous adenocarcinoma, ovarian clear cell carcinoma, endometrioid ovarian cancer, and ovarian mucinous tumor.

**50.** The pharmaceutical composition according to any one of claims 31 to 45, wherein the pharmaceutical composition is for use in treating ovarian cancer.

**51.** The pharmaceutical composition according to claim 50, wherein the ovarian cancer is epithelial ovarian cancer, fallopian tube cancer, or primary peritoneal cancer.

**52.** The pharmaceutical composition according to claim 50 or 51, wherein the ovarian cancer is metastatic.

**53.** The pharmaceutical composition according to any one of claims 1 to 52, wherein the anti-CDH6 antibody-drug conjugate is raludotatug deruxtecan (DS-6000a).

**54.** A method of treatment, comprising administering an anti-CDH6 antibody-drug conjugate and a VEGF inhibitor in combination to a subject in need of the treatment, wherein the antibody-drug conjugate is an antibody-drug conjugate in which a drug-linker represented by the formula:

[Formula 3]

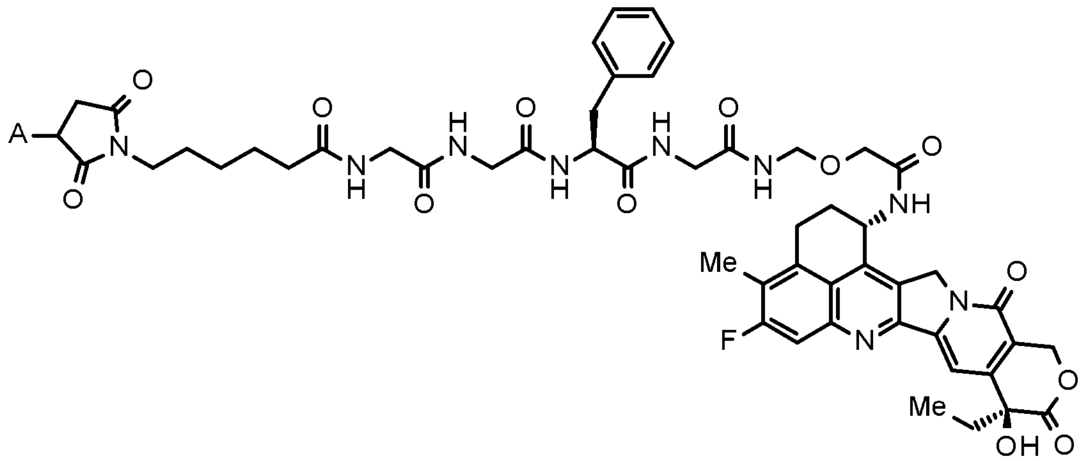

wherein A represents a connecting position to an anti-CDH6 antibody or a functional fragment of the antibody, is conjugated to the anti-CDH6 antibody or the functional fragment of the antibody via a thioether bond.

**55.** The method of treatment according to claim 54, wherein the anti-CDH6 antibody or the functional fragment of the antibody is an antibody which specifically binds to the amino acid sequence shown in SEQ ID NO: 1 and has an internalization ability that permits cellular uptake, or a functional fragment of the antibody.

**56.** The method of treatment according to claim 54 or 55, wherein the anti-CDH6 antibody or the functional fragment of the antibody is an antibody comprising CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, or a functional fragment of the antibody.

**57.** The method of treatment according to any one of claims 54 to 56, wherein the anti-CDH6 antibody or the functional fragment of the antibody is an antibody comprising a heavy chain variable region consisting of the amino acid sequence shown in SEQ ID NO: 28 and a light chain variable region consisting of the amino acid sequence shown in SEQ ID NO: 37, or a functional fragment of the antibody.

**58.** The method of treatment according to any one of claims 54 to 57, wherein the anti-CDH6 antibody or the functional fragment of the antibody is an antibody comprising a heavy chain consisting of the amino acid sequence shown in SEQ ID NO: 29 and a light chain consisting of the amino acid sequence shown in SEQ ID NO: 38, or a functional fragment of the antibody.

**59.** The method of treatment according to any one of claims 54 to 58, wherein the anti-CDH6 antibody or the functional fragment of the antibody is an antibody in which the heavy chain or the light chain has undergone one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, conversion of N-terminal glutamine or N-terminal glutamic acid to pyroglutamic acid, and a deletion of one or two amino acids from the carboxyl terminus, or a functional fragment of the antibody.

**60.** The method of treatment according to claim 59, wherein the anti-CDH6 antibody or the functional fragment of the antibody is an antibody in which one or two amino acids are deleted from the carboxyl terminus of a heavy chain thereof, or a functional fragment of the antibody.

**61.** The method of treatment according to claim 60, wherein the anti-CDH6 antibody or the functional fragment of the antibody is an antibody in which one amino acid is deleted from the carboxyl terminus of each of the two heavy chains thereof, or a functional fragment of the antibody.

**62.** The method of treatment according to any one of claims 59 to 61, wherein the deleted amino acid is lysine.

**63.** The method of treatment according to any one of claims 59 to 62, wherein the anti-CDH6 antibody or the functional fragment of the antibody is an antibody in which a proline residue at the carboxyl terminus of a heavy chain thereof is further amidated, or a functional fragment of the antibody.

**64.** The method of treatment according to any one of claims 54 to 63, wherein the average number of units of the drug-linker structure conjugated per antibody in the anti-CDH6 antibody-drug conjugate is in the range of from 1 to 10.

**65.** The method of treatment according to any one of claims 54 to 63, wherein the average number of units of the drug-linker structure conjugated per antibody in the anti-CDH6 antibody-drug conjugate is from 7 to 8.

**66.** The method of treatment according to any one of claims 54 to 65, wherein the VEGF inhibitor is an anti-VEGF antibody, an anti-VEGF receptor antibody, a fusion protein comprising a VEGF receptor extracellular domain, or a multispecific antibody comprising an anti-VEGF binding domain.

**67.** The method of treatment according to claim 66, wherein the VEGF inhibitor is an anti-VEGF antibody.

**68.** The method of treatment according to claim 67, wherein the anti-VEGF antibody is bevacizumab.

**69.** The method of treatment according to claim 66, wherein the VEGF inhibitor is an anti-VEGF receptor antibody.

**70.** The method of treatment according to claim 69, wherein the anti-VEGF receptor antibody is ramucirumab.

**71.** The method of treatment according to claim 66, wherein the VEGF inhibitor is a fusion protein comprising a VEGF receptor extracellular domain.

**72.** The method of treatment according to claim 71, wherein the fusion protein comprising a VEGF receptor extracellular domain is aflibercept.

**73.** The method of treatment according to claim 66, wherein the VEGF inhibitor is a multispecific antibody comprising an anti-VEGF binding domain.

**74.** The method of treatment according to claim 73, wherein the multispecific antibody is a bispecific antibody.

**75.** The method of treatment according to any one of claims 54 to 74, wherein the anti-CDH6 antibody-drug conjugate and the VEGF inhibitor are separately contained in different formulations, and are administered at the same time or at different times.

**76.** The method of treatment according to any one of claims 54 to 74, wherein the anti-CDH6 antibody-drug conjugate and the VEGF inhibitor are contained in a single formulation for administration.

**77.** The method of treatment according to any one of claims 54 to 76, wherein the method of treatment is for treating cancer.

**78.** The method of treatment according to any one of claims 54 to 76, wherein the method of treatment is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

**79.** The method of treatment according to any one of claims 54 to 76, wherein the method of treatment is for treating at least one selected from the group consisting of renal cell carcinoma, renal clear cell carcinoma, and papillary renal cell carcinoma.

**80.** The method of treatment according to any one of claims 54 to 76, wherein the method of treatment is for treating at least one selected from the group consisting of ovarian cancer, ovarian serous adenocarcinoma, ovarian clear cell carcinoma, endometrioid ovarian cancer, and ovarian mucinous tumor.

**81.** The method of treatment according to any one of claims 54 to 76, wherein the method of treatment is for treating ovarian cancer.

**82.** The method of treatment according to claim 81, wherein the ovarian cancer is epithelial ovarian cancer, fallopian tube cancer, or primary peritoneal cancer.

**83.** The method of treatment according to claim 81 or 82, wherein the ovarian cancer is metastatic.

**84.** A method of treatment, comprising administering an anti-CDH6 antibody-drug conjugate and a VEGF inhibitor in combination to a subject in need of the treatment, wherein the anti-CDH6 antibody-drug conjugate is an antibody-drug conjugate represented by the formula:

[Formula 4]

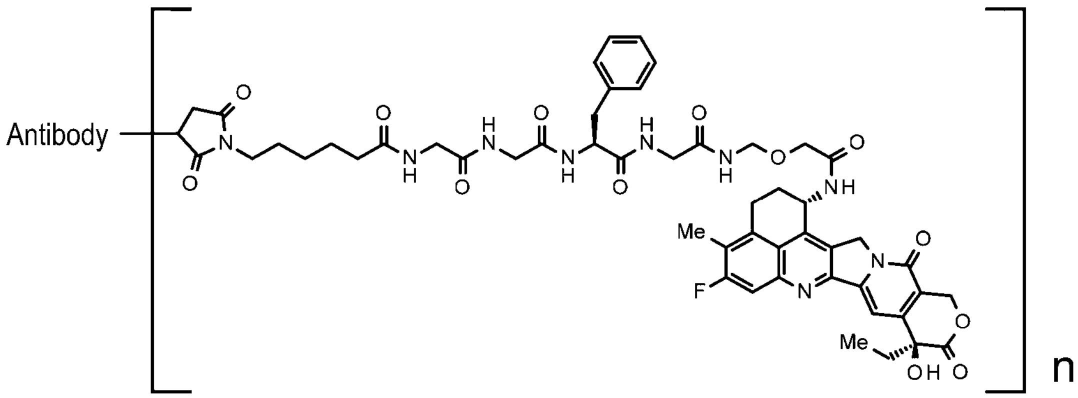

wherein Antibody is an anti-CDH6 antibody, a drug-linker is conjugated to the antibody via a thioether bond, and n represents an average number of units of the drug-linker conjugated per antibody.

**85.** The method of treatment according to claim 84, wherein the anti-CDH6 antibody is an antibody comprising a heavy chain consisting of the amino acid sequence shown in SEQ ID NO: 29 and a light chain consisting of the amino acid sequence shown in SEQ ID NO: 38.

**86.** The method of treatment according to claim 84 or 85, wherein a lysine residue is deleted from the carboxyl terminus of a heavy chain of the anti-CDH6 antibody.

**87.** The method of treatment according to any one of claims 84 to 86, wherein the average number of units of the drug-linker conjugated per antibody in the anti-CDH6 antibody-drug conjugate is in the range of from 7 to 8.

**88.** The method of treatment according to any one of claims 84 to 87, wherein the VEGF inhibitor is an anti-VEGF antibody, an anti-VEGF receptor antibody, a fusion protein comprising a VEGF receptor extracellular domain, or a multispecific antibody comprising an anti-VEGF binding domain.

**89.** The method of treatment according to claim 88, wherein the VEGF inhibitor is an anti-VEGF antibody.

**90.** The method of treatment according to claim 89, wherein the anti-VEGF antibody is bevacizumab.

**91.** The method of treatment according to claim 88, wherein the VEGF inhibitor is an anti-VEGF receptor antibody.

**92.** The method of treatment according to claim 91, wherein the anti-VEGF receptor antibody is ramucirumab.

**93.** The method of treatment according to claim 88, wherein the VEGF inhibitor is a fusion protein comprising a VEGF receptor extracellular domain.

**94.** The method of treatment according to claim 93, wherein the fusion protein comprising a VEGF receptor extracellular domain is aflibercept.

**95.** The method of treatment according to claim 88, wherein the VEGF inhibitor is a multispecific antibody comprising an anti-VEGF binding domain.

**96.** The method of treatment according to claim 95, wherein the multispecific antibody is a bispecific antibody.

**97.** The method of treatment according to any one of claims 84 to 96, wherein the antibody-drug conjugate and the VEGF inhibitor are separately contained in different formulations, and are administered at the same time or at different times.

**98.** The method of treatment according to any one of claims 84 to 96, wherein the anti-CDH6 antibody-drug conjugate and the VEGF inhibitor are contained in a single formulation for administration.

**99.** The method of treatment according to any one of claims 84 to 98, wherein the method of treatment is for treating cancer.

**100.**
The method of treatment according to any one of claims 84 to 98, wherein the method of treatment is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

**101.**
The method of treatment according to any one of claims 84 to 98, wherein the method of treatment is for treating at least one selected from the group consisting of renal cell carcinoma, renal clear cell carcinoma, and papillary renal cell carcinoma.

**102.**
The method of treatment according to any one of claims 84 to 98, wherein the method of treatment is for treating at least one selected from the group consisting of ovarian cancer, ovarian serous adenocarcinoma, ovarian clear cell carcinoma, endometrioid ovarian cancer, and ovarian mucinous tumor.

**103.**
The method of treatment according to any one of claims 84 to 98, wherein the method of treatment is for treating ovarian cancer.

**104.**
The method of treatment according to claim 103, wherein the ovarian cancer is epithelial ovarian cancer, fallopian tube cancer or primary peritoneal cancer.

**105.**
The method of treatment according to claim 103 or 104, wherein the ovarian cancer is metastatic.

**106.**
The method of treatment according to any one of claims 54 to 105, wherein the anti-CDH6 antibody-drug conjugate is raludotatug deruxtecan (DS-6000a).

# Fig. 1

SEQ ID NO: 1: Amino acid sequence of human CDH6 EC3

P Q S T Y Q F K T P E S S P P G T P I G R I K A S D A D V G E N A E I E Y S
I T D G E G L D M F D V I T D Q E T Q E G I I T V K K L L D F E K K K V Y T
L K V E A S N P Y V E P R F L Y L G P F K D S A T V R I V V E D V D E P P V
F

# Fig. 2

SEQ ID NO: 2 Amino acid sequence of anti-CDH6 antibody heavy chain CDRH1

(amino acid sequence of chG019 CDRH1)

G Y T F T R N F M H

# Fig. 3

SEQ ID NO: 3: Amino acid sequence of anti-CDH6 antibody heavy chain CDRH2

(amino acid sequence of chG019 CDRH2)

W I Y P G D G E T E

# Fig. 4

SEQ ID NO: 4: Amino acid sequence of anti-CDH6 antibody heavy chain CDRH3

(amino acid sequence of chG019 CDRH3)

G V Y G G F A G G Y F D F

# Fig. 5

SEQ ID NO: 5: Amino acid sequence of anti-CDH6 antibody heavy chain CDRL1

(amino acid sequence of chG019 CDRL1)

K A S Q N I Y K N L A

# Fig. 6

SEQ ID NO: 6: Amino acid sequence of anti-CDH6 antibody heavy chain CDRL2

(amino acid sequence of chG019 CDRL2)

D A N T L Q T

# Fig. 7

SEQ ID NO: 7: Amino acid sequence of anti-CDH6 antibody heavy chain CDRL3

(amino acid sequence of chG019 CDRL3)

Q Q Y Y S G W A

# Fig. 8

SEQ ID NO: 27: Amino acid sequence of anti-CDH6 antibody heavy chain (full-length amino acid sequence of hH01 heavy chain)

M K H L W F F L L L V A A P R W V L S E V Q L V Q S G A E V K K P G A S V K
V S C K A S G Y T F T R N F M H W V R Q A P G Q G L E W M G W I Y P G D G E
T E Y A Q K F Q G R V T I T A D T S T S T A Y M E L S S L R S E D T A V Y Y
C A R G V Y G G F A G G Y F D F W G Q G T L V T V S S A S T K G P S V F P L
A P S S K S T S G G T A A L G C L V K D Y F P E P V T V S W N S G A L T S G
V H T F P A V L Q S S G L Y S L S S V V T V P S S S L G T Q T Y I C N V N H
K P S N T K V D K R V E P K S C D K T H T C P P C P A P E L L G G P S V F L
F P P K P K D T L M I S R T P E V T C V V V D V S H E D P E V K F N W Y V D
G V E V H N A K T K P R E E Q Y N S T Y R V V S V L T V L H Q D W L N G K E
Y K C K V S N K A L P A P I E K T I S K A K G Q P R E P Q V Y T L P P S R E
E M T K N Q V S L T C L V K G F Y P S D I A V E W E S N G Q P E N N Y K T T
P P V L D S D G S F F L Y S K L T V D K S R W Q Q G N V F S C S V M H E A L
H N H Y T Q K S L S L S P G K

| Signal sequence (1-19) | variable region (20-141) | constant region (142-471) |
|---|---|---|

# Fig. 9

SEQ ID NO: 28: Amino acid sequence of anti-CDH6 antibody heavy chain variable region (amino acid sequence of hH01 heavy chain variable region)

E V Q L V Q S G A E V K K P G A S V K V S C K A S G Y T F T R N F M H W V R
Q A P G Q G L E W M G W I Y P G D G E T E Y A Q K F Q G R V T I T A D T S T
S T A Y M E L S S L R S E D T A V Y Y C A R G V Y G G F A G G Y F D F W G Q
G T L V T V S S

# Fig. 10

SEQ ID NO: 29: Amino acid sequence of mature anti-CDH6 antibody heavy chain (except for signal sequence) (full-length amino acid sequence of hH01 heavy chain except for signal sequence)

E V Q L V Q S G A E V K K P G A S V K V S C K A S G Y T F T R N F M H W V R
Q A P G Q G L E W M G W I Y P G D G E T E Y A Q K F Q G R V T I T A D T S T
S T A Y M E L S S L R S E D T A V Y Y C A R G V Y G G F A G G Y F D F W G Q
G T L V T V S S A S T K G P S V F P L A P S S K S T S G G T A A L G C L V K
D Y F P E P V T V S W N S G A L T S G V H T F P A V L Q S S G L Y S L S S V
V T V P S S S L G T Q T Y I C N V N H K P S N T K V D K R V E P K S C D K T
H T C P P C P A P E L L G G P S V F L F P P K P K D T L M I S R T P E V T C
V V V D V S H E D P E V K F N W Y V D G V E V H N A K T K P R E E Q Y N S T
Y R V V S V L T V L H Q D W L N G K E Y K C K V S N K A L P A P I E K T I S
K A K G Q P R E P Q V Y T L P P S R E E M T K N Q V S L T C L V K G F Y P S
D I A V E W E S N G Q P E N N Y K T T P P V L D S D G S F F L Y S K L T V D
K S R W Q Q G N V F S C S V M H E A L H N H Y T Q K S L S L S P G K

# Fig. 11

SEQ ID NO: 36: Amino acid sequence of anti-CDH6 antibody light chain (full-length amino acid sequence of hL02 light chain)

M V L Q T Q V F I S L L L W I S G A Y G D I Q M T Q S P S S L S A S V G D R
V T I T C K A S Q N I Y K N L A W Y Q Q K P G K A P K L L I Y D A N T L Q T
G V P S R F S G S G S G S D F T L T I S S L Q P E D F A T Y F C Q Q Y Y S G
W A F G Q G T K V E I K R T V A A P S V F I F P P S D E Q L K S G T A S V V
C L L N N F Y P R E A K V Q W K V D N A L Q S G N S Q E S V T E Q D S K D S
T Y S L S S T L T L S K A D Y E K H K V Y A C E V T H Q G L S S P V T K S F
N R G E C

| constant region (1-20) | variable region (21-128) | Signal sequence (129-233) |
|---|---|---|

# Fig. 12

SEQ ID NO: 37: Amino acid sequence of anti-CDH6 antibody light chain variable region (amino acid sequence of hL02 light chain variable region)

D I Q M T Q S P S S L S A S V G D R V T I T C K A S Q N I Y K N L A W Y Q Q
K P G K A P K L L I Y D A N T L Q T G V P S R F S G S G S G S D F T L T I S
S L Q P E D F A T Y F C Q Q Y Y S G W A F G Q G T K V E I K R T

# Fig. 13

SEQ ID NO: 38: Amino acid sequence of mature anti-CDH6 antibody light chain (except for signal sequence) (full-length amino acid sequence of hL02 light chain except for signal sequence)

D I Q M T Q S P S S L S A S V G D R V T I T C K A S Q N I Y K N L A W Y Q Q
K P G K A P K L L I Y D A N T L Q T G V P S R F S G S G S G S D F T L T I S
S L Q P E D F A T Y F C Q Q Y Y S G W A F G Q G T K V E I K R T V A A P S V
F I F P P S D E Q L K S G T A S V V C L L N N F Y P R E A K V Q W K V D N A
L Q S G N S Q E S V T E Q D S K D S T Y S L S S T L T L S K A D Y E K H K V
Y A C E V T H Q G L S S P V T K S F N R G E C

Fig. 14

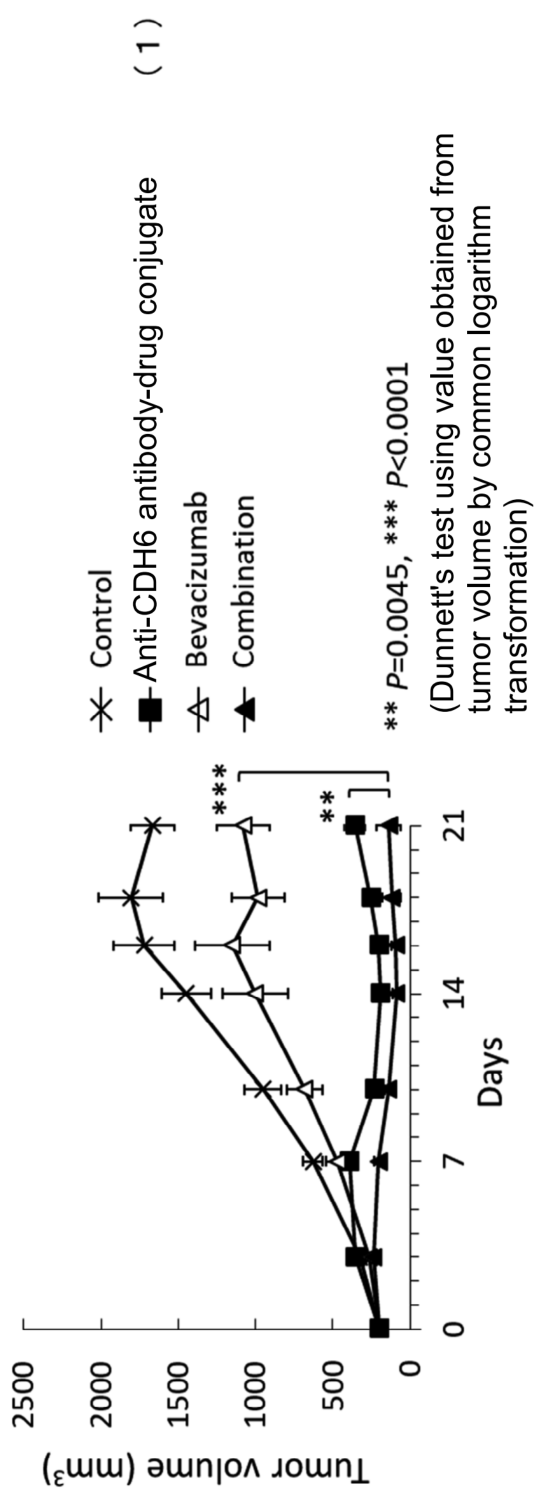
(1)
Tumor volume (mm³)
2500
2000
1500
1000
500
0
0
7
14
21
Days
***
**
Control
Anti-CDH6 antibody-drug conjugate
Bevacizumab
Combination
** P=0.0045, *** P<0.0001
(Dunnett's test using value obtained from tumor volume by common logarithm transformation)

Fig. 15

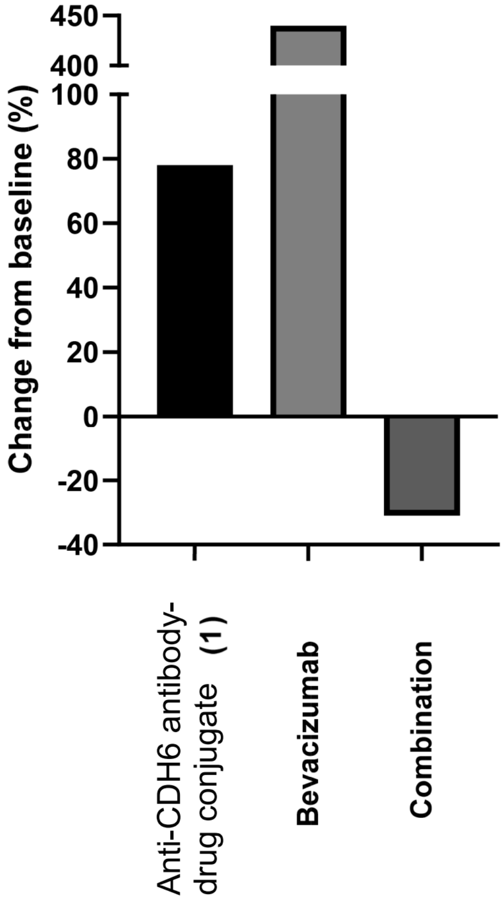

Change from baseline (%)
450
400
100
80
60
40
20
0
-20
-40
Anti-CDH6 antibody-drug conjugate (1)
Bevacizumab
Combination

## INTERNATIONAL SEARCH REPORT

International application No. **PCT/JP2024/009716**

### A. CLASSIFICATION OF SUBJECT MATTER

***A61K 47/68***(2017.01)i; ***A61K 31/4745***(2006.01)i; ***A61K 39/395***(2006.01)i; ***A61K 45/00***(2006.01)i; ***A61P 13/12***(2006.01)i; ***A61P 15/00***(2006.01)i; ***A61P 35/00***(2006.01)i; ***A61P 43/00***(2006.01)i; ***C07K 7/02***(2006.01)i; ***C07K 16/30***(2006.01)i; ***C12N 15/13***(2006.01)i

FI: A61K47/68; A61P13/12; A61P35/00; A61P43/00 121; A61K45/00; A61K39/395 N; A61K39/395 L; A61K39/395 T; C07K7/02; C07K16/30; C12N15/13; A61P15/00 ZNA; A61K31/4745

According to International Patent Classification (IPC) or to both national classification and IPC

### B. FIELDS SEARCHED

Minimum documentation searched (classification system followed by classification symbols)

A61K47/68; A61K31/4745; A61K39/395; A61K45/00; A61P13/12; A61P15/00; A61P35/00; A61P43/00; C07K7/02; C07K16/30; C12N15/13

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2020/130125 A1 (DAIICHI SANKYO COMPANY, LIMITED) 25 June 2020 (2020-06-25)<br>claims, paragraph [0356], examples 10, 11 | 1-106 |
| Y | WO 2020/122034 A1 (DAIICHI SANKYO COMPANY, LIMITED) 18 June 2020 (2020-06-18)<br>claims, paragraph [0237], example 19 | 1-106 |
| Y | WO 2020/031936 A1 (DAIICHI SANKYO COMPANY, LIMITED) 13 February 2020 (2020-02-13)<br>claims, paragraph [0181], examples 9, 10 | 1-106 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 May 2024** | **21 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
**PCT/JP2024/009716**

**C. DOCUMENTS CONSIDERED TO BE RELEVANT**

| Category* | Citation of document, with indication, where appropriate, of the relevant passages | Relevant to claim No. |
|---|---|---|
| Y | WO 2018/212136 A1 (DAIICHI SANKYO COMPANY, LIMITED) 22 November 2018 (2018-11-22) claims, examples | 1-106 |
| Y | WO 2021/182573 A1 (TORAY INDUSTRIES, INC.) 16 September 2021 (2021-09-16) claims, example 2 | 1-106 |

Form PCT/ISA/210 (second sheet) (July 2022)

**INTERNATIONAL SEARCH REPORT**

International application No.
**PCT/JP2024/009716**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/009716**

| Patent document cited in search report | Publication date (day/month/year) | Patent family member(s) | Publication date (day/month/year) |
|---|---|---|---|
| WO 2020/130125 A1 | 25 June 2020 | US 2022/0040324 A1<br>claims, paragraph [0281], examples 10, 11<br>EP 3903828 A1<br>CN 113195000 A<br>KR 10-2021-0107069 A | |
| WO 2020/122034 A1 | 18 June 2020 | US 2022/0023436 A1<br>claims, paragraph [0573], example 19<br>EP 3909580 A1<br>CN 113271942 A<br>KR 10-2021-0102341 A | |
| WO 2020/031936 A1 | 13 February 2020 | US 2021/0290775 A1<br>claims, paragraph [0414], examples 9, 10<br>EP 3834843 A1<br>CN 112512587 A<br>KR 10-2021-0042120 A | |
| WO 2018/212136 A1 | 22 November 2018 | US 2020/0171163 A1<br>claims, examples<br>EP 3626825 A1<br>CN 110651045 A<br>KR 10-2020-0006061 A | |
| WO 2021/182573 A1 | 16 September 2021 | US 2023/0129035 A1<br>claims, example 2<br>EP 4119156 A1<br>KR 10-2022-0152318 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018212136 A
- WO 2023102875 A
- WO 2023104188 A
- WO 9007861 A
- US 5821337 A
- WO 9954342 A
- WO 0061739 A
- WO 0231140 A
- WO 2007133855 A
- WO 2013120066 A
- WO 2014057687 A
- WO 2015098099 A
- WO 2015115091 A
- WO 2015155998 A
- WO 2019044947 A
- WO 2018135501 A


### Non-patent literature cited in the description


- **GARCIA et al.** *Cancer Treat. Rev*, 2020, vol. 86, 102017
- **GEINDREAU et al.** *Int. J. Mol. Sci.*, 2021, vol. 22 (9), 4871
- **ESFANDIARI et al.** *Nat. Rev. Drug Discov.*, 2022, vol. 21 (6), 411-412
- **XU et al.** *Cancer Lett.*, 2022, vol. 538, 215699
- **SHIMOYAMA Y et al.** *Cancer Research*, 15 May 1995, vol. 55, 2206-2211
- **OGITANI Y. et al.** *Clinical Cancer Research*, 29 March 2016, vol. 22 (20), 5097-5108
- **SUZUKI H et al.** *Molecular Cancer Therapeutics*, 2024, vol. 23 (3), 257-271
- *Cell Death and Differentiation*, 2008, vol. 15, 751-761
- *Molecular Biology of the Cell*, December 2004, vol. 15, 5268-5282
- *Bio Techniques*, January 2000, vol. 28, 162-165
- **KOHLER** ; **MILSTEIN**. *Nature*, 1975, vol. 256, 495-497
- Monoclonal Antibodies. Plenum Press, 1980, 365-367
- *Proc. Natl. Acad. Sci. U.S.A.*, 1984, vol. 81, 6851-6855
- *Nature*, 1986, vol. 321, 522-525
- **TOMIZUKA, K. et al.** *Nature Genetics*, 1997, vol. 16, 133-143
- **KUROIWA, Y. et al.** *Nucl. Acids Res.*, 1998, vol. 26, 3447-3448
- **YOSHIDA, H. et al.** Animal Cell Technology: Basic and Applied Aspects. Kluwer Academic Publishers, 1999, vol. 10, 69-73
- **TOMIZUKA, K. et al.** *Proc. Natl. Acad. Sci. USA*, 2000, vol. 97, 722-727
- **WORMSTONE, I. M. et al.** *Investigative Ophthalmology & Visual Science*, 2002, vol. 43 (7), 2301-2308
- **CARMEN, S. et al.** *Briefings in Functional Genomics and Proteomics*, 2002, vol. 1 (2), 189-203
- **SIRIWARDENA, D. et al.** *Ophthalmology*, 2002, vol. 109 (3), 427-431
- **LARKIN MA** ; **BLACKSHIELDS G** ; **BROWN NP** ; **CHENNA R** ; **MCGETTIGAN PA** ; **MCWILLIAM H** ; **VALENTIN F** ; **WALLACE IM** ; **WILM A** ; **LOPEZ R**. Clustal W and Clustal X version 2.0. *Bioinformatics*, 2007, vol. 23 (21), 2947-2948
- *Journal of Chromatography A*, 1995, vol. 705, 129-134
- *Analytical Biochemistry*, 2007, vol. 360, 75-83
- **HERMANSON, G.T**. Bioconjugate Techniques. Academic Press, 1996, 456-493
- *Clin. Exp. Metastasis*, 2020, vol. 37 (6), 637-648
- *Journal of Clinical Oncology*, 01 June 2023, vol. 41 (16), 2536-2536
- **BISSERY et al.** *Cancer Res.*, 1991, vol. 51, 4845-4852